# EUROPEAN PATENT APPLICATION

(11) **EP 2 298 932 A1**
(43) Date of publication of application: **23.03.2011**
(21) Application number: 10175696.3
(22) Date of filing: 28.09.2006
(51) Int. Cl.: C12Q 1/68

(54) **Methods and nucleic acids for the analysis of gene expression, in particular methylation of KAAG1, associated with tissue classification**

(30) Priority: 29.09.2005 EP 05021331; 23.12.2005 EP 05090346; 15.06.2006 EP 06090110
(62) Divisional of application: 06792318.5
(71) Applicant: Epigenomics AG, 10178 Berlin (DE)
(72) Inventor: Berlin, Kurt, 14532 Stahnsdorf (DE); Beck, Stephan, Whittlesford Cambridge CB2 4NZ (GB); Burger, Matthias, 10967 Berlin (DE); Cortese, Rene, Toronto, ON M4Y 1N2 (CA); Eckhardt, Florian, 10405 Berlin (DE); Haefliger, Carolina, 4052 Basel (CH); Lewin, Jörn, 10115 Berlin (DE); Model, Fabian, 12683 Berlin (DE); Olek, Alexander, 12207 Berlin (DE)

(57) **Abstract**

The following application provides methods and nucleic acids the classification of a biological sample. This is achieved by the analysis of the expression status and/or methylation status of the gene KAAG1 (RU2AS) which is methylated in liver tissue.

## Description

### FIELD OF THE INVENTION

The present invention relates to human DNA sequences that exhibit tissue specific expression patterns. Particular embodiments of the invention provide methods for classifying a biological sample.

### SEQUENCE LISTING

### BACKGROUND

*Bisulfite modification of DNA is an art-recognized tool used to assess CpG methylation status. 5-*methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g*., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil, which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

*Methylation Assay Procedures.* Various methylation assay procedures are known in the art, and can be used in conjunction with the present invention. These assays allow for determination of the methylation state of one or a plurality of CpG dinucleotides (*e.g*., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.*, the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

Preferably, assays such as "MethyLight^{™}" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), are used alone or in combination with other of these methods.

*MethyLight^{™}.* The MethyLight^{™} assay is a high-throughput quantitative methylation assay that utilizes fluorescence-based real-time PCR (TaqMan^{™}) technology that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight^{™} assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; *e.g.,* with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical MethyLight^{™}-based kit) for MethyLight^{™} analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g*., microdisected pathology sections), and it avoids utilization of restriction enzymes for determining the methylation status at CpG sites.

Typical reagents (*e.g*., as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers; Ms-SNuPE primers for specific gene; reaction buffer (for the Ms-SNuPE reaction); and labeled nucleotides. Additionally, bisulfite conversion reagents may include: DNA denaturation buffer; sulfonation buffer; DNA recovery reagents or kit (*e.g*., precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated DNA specific PCR primers for specific gene (or bisulfite treated DNA sequence or CpG island), optimized PCR buffers and deoxynucleotides, and specific probes.

*Prior art of several markers.* If not stated otherwise, the following information was received from the Entrez database (http://www.ncbi.nlm.nih.gov/entrez/query.fcgi?db=gene) and the OMIM database (http://www.ncbi.nlm.nih.gov/entrez/query.fcgi?db=OMIM).

*Glycoprotein Ib (platelet) beta polypeptide.* (GP1BB) Glycoprotein Ib (platelet) beta polypeptide (GP1 BB) is subunit of the platelet glycoprotein Ib (GPIb). GPIb is a heterodimeric transmembrane protein consisting of a disulfide-linked 140 kD alpha chain and 22 kD beta chain. It is part of the GPIb-V-IX system that constitutes the receptor for von Willebrand factor (VWF), and mediates platelet adhesion in the arterial circulation. GPIb alpha chain provides the VWF binding site, and GPIb beta contributes to surface expression of the receptor and participates in transmembrane signaling through phosphorylation of its intracellular domain. Mutations in the GPIb beta subunit have been associated with Bernard-Soulier syndrome, velocardiofacial syndrome and giant platelet disorder. The 206 amino acid precursor of GPIb beta is synthesized from a 1.0 kb mRNA expressed in plateletes and megakaryocytes. A 411 amino acid protein arising from a longer, unspliced transcript in endothelial cells has been described; however, the authenticity of this product has been questioned. Yet another less abundant GPIb beta mRNA species of 3.5 kb, expressed in nonhematopoietic tissues such as endothelium, brain and heart, was shown to result from inefficient usage of a non-consensus polyA_signal within a separate gene (PNUTL1) located upstream of this gene. In the absence of polyadenylation from its own imperfect site, the PNUTL1 gene uses the consensus polyA_signal of this gene (Entrez database).

*Glutathione peroxidase* 5. Glutathione peroxidase 5 (GPX5) also known as glutathione reductase is part of the hydrogen peroxide scavenging system found within the epididymis in the mammalian male reproductive tract. GPX5 expression is epididymis-specific and the transcript is unique from other GPXs because it contains a deletion resulting in an mRNA that does not contain a selenocysteine (UGA) codon (an unusual amino acid present in other GPXs). This deletion also renders the mRNA incapable of encoding an active GPX isoenzyme. For this reason, GPX5 is selenium-independent and has very little activity towards hydrogen peroxide or organic hydroperoxides. GPX5, which is bound to the acrosome of sperm, may act to protect sperm from premature acrosome reaction in the epididymis (Entrez database). The cDNA of human GPX5 is cloned. Thereby it was shown that the majority of transcripts contain a 118 nt frame-shifting deletion, arising, most likely, from inappropriate excision of exon 3 during processing. Antisera raised against recombinant human GPX5 cross-reacted with rat and macaque (Macaca fascicularis) epididymal proteins of the size expected for full-length, active GPX5. However, no similar reactivity could be demonstrated in any of the human samples tested (Hall et al, 1998). The tissue-restricted polyoma enhancer activator protein (PEA3) of the ETS oncogene family of DNA-binding proteins is a putative modulator of the epididymis-specific glutathione peroxidase 5 gene GPX5 (Drevet et al, 1998). At least part of the androgenic control of the GPX5 expression is exerted at the transcriptional level via an androgen response element localized in the distal promoter region of the GPX5 gene (Lareyre et al, 1997).

*Cytidine monophosphate-N-acetylneuraminic acid hydroxylase.* Cytidine monophosphate-N-acetylneuraminic acid hydroxylase (CMAH) is also known as CMP-N-acetylneuraminate monooxygenase, CMP-NeuAc hydroxylase, CMP-Neu5Ac hydroxylase, CMP-sialic acid hydroxylase (CSAH), CMP-N-acetylneuraminic acid hydroxylase). Sialic acids are terminal components of the carbohydrate chains of glycoconjugates involved in ligand-receptor, cell-cell, and cell-pathogen interactions. The two most common forms of sialic acid found in mammalian cells are N-acetylneuraminic acid (Neu5Ac) and its hydroxylated derivative, N-glycolylneuraminic acid (Neu5Gc). Studies of sialic acid distribution show that Neu5Gc is not detectable in normal human tissues although it was an abundant sialic acid in other mammals. Neu5Gc is, in actuality, immunogenic in humans. The absense of Neu5Gc in humans is due to a deletion within the human gene CMAH encoding cytidine monophosphate-N-acetylneuraminic acid hydroxylase, an enzyme responsible for Neu5Gc biosynthesis. Sequences encoding the mouse, pig, and chimpanzee hydroxylase enzymes were obtained by cDNA cloning and found to be highly homologous. However, the homologous human cDNA differs from these cDNAs by a 92-bp deletion in the 5' region. This deletion, corresponding to exon 6 of the mouse hydroxylase gene, causes a frameshift mutation and premature termination of the polypeptide chain in human. It seems unlikely that the truncated human hydroxylase mRNA encodes for an active enzyme explaining why Neu5Gc is undetectable in normal human tissues. Human genomic DNA also shows evidence of this deletion which does not occur in the genomes of African great apes. Nonetheless, the CMAH gene maps to 6p21.32 in humans and great apes indicating that mutation of the CMAH gene occurred following human divergence from chimpanzees and bonobos (Entrez database). Studies indicate that the CMAH gene is inactivated shortly before the time when brain expansion began in humankind's ancestry, approximately 2.1-2.2 mya. In this regard, it is of interest that although Neu5Gc is the major sialic acid in most organs of the chimpanzee, its expression is selectively down-regulated in the brain (Chou et al, 2002).

*Bone morphogenetic protein 7 precursor.* Bone morphogenetic protein 7 precursor (BMP-7) is also known as Osteogenic protein 1 (OP-1) or Eptotermin alfa. The bone morphogenetic proteins (BMPs) are a family of secreted signaling molecules that can induce ectopic bone growth. Many BMPs are part of the transforming growth factor-beta (TGFB) superfamily. BMPs were originally identified by an ability of demineralized bone extract to induce endochondral osteogenesis in vivo in an extraskeletal site. Based on its expression early in embryogenesis, the BMP encoded by this gene has a proposed role in early development. In addition, the fact that this BMP is closely related to BMP5 and BMP7 has lead to speculation of possible bone inductive activity (Entrez database).

Caspase *recruitment domain protein 10*. Caspase recruitment domain protein 10 (CARD10) is also known as CARD-containing MAGUK protein 3 (CARMA 3). The caspase recruitment domain (CARD) is a protein module that consists of 6 or 7 antiparallel alpha helices. It participates in apoptosis signaling through highly specific protein-protein homophilic interactions. CARDs induce nuclear factor kappa-B (NFKB; MIM 164011) activity through the IKK (e.g., IKBKB; MIM 603258) complex. CARD9 (MIM 607212), CARD10, CARD11 (MIM 607210), and CARD14 (MIM 607211) interact with BCL10 (MIM 603517) and are involved in NFKB signaling complexes. Except for CARD9, these CARD proteins are members of the membrane-associated guanylate kinase (MAGUK) family (OMIM database). CARMA3 physically associate with Ikappa kinase gamma/NFkappaB essential modulator (IkappaKgamma-NEMO) in lymphoid and non-lymphoid cells. Expression of the NEMO-binding region of CARMA3 exerts a dominant negative effect on BCL10-mediated activation of NfkappaB (Stilo et al, 2004). CARD10 is a novel BCL10 interactor that belongs to the membrane-associated guanylate kinase family, a class of proteins that function to organize signaling complexes at plasma membranes. When expressed in cells, CARD10 binds to BCL10 and signals the activation of NF-kappaB through its N-terminal effector CARD domain. It is proposed that CARD10 functions as a molecular scaffold for the assembly of a BCL10 signaling complex that activates NF-kappaB (Wang et al, 2001).

*Platelet-derived growth factor B chain precursor.* Platelet-derived growth factor B chain precursor (PDGF B-chain, PDGFB, SIS, SSV, PDGF2, c-sis) is a member of the platelet-derived growth factor family. The four members of this family are mitogenic factors for cells of mesenchymal origin and are characterized by a motif of eight cysteines. This gene product can exist either as a homodimer (PDGF-BB) or as a heterodimer with the platelet-derived growth factor alpha polypeptide (PDGF-AB), where the dimers are connected by disulfide bonds. Mutations in this gene are associated with meningioma. Reciprocal translocations between chromosomes 22 and 7, at sites where this gene and that for COL1A1 are located, are associated with a particular type of skin tumor called dermatofibrosarcoma protuberans resulting from unregulated expression of growth factor. Two splice variants have been identified for this gene (Entrez database). Medulloblastomas contained the highest amounts of PDGF B-chain, some four to eight times more than that in control brain tissue. Tumors that contained a high level of PDGF B-chain showed high proliferative activity (Nakamura et al, 1993). Trichostatin A (TSA) activates reporter gene constructs driven by the human platelet-derived growth factor B (PDGF-B) gene promoter. This activation showed an inverse correlation with the cell type-specific transcriptional activities of the promoter (Ulleras et al, 2001). Tissue specificity was not clear in the 5' upstream region alone, and regulation by gene methylation or by elements other than in the 5' region seemed to be necessary (Takimoto et al, 1993). Platelet-derived growth factor B-chain was also abnormally methylated in 4 of 13 (31%) multinodular goiters (MNG), 17 of 24 (71%) follicular adenomas (FA), and 9 of 13 (69%) papillary carcinomas (PC) (Matsuo et al, 1993). Gene expression of PDGF-A and PDGF-B mRNA were increased 22- and 6-fold, respectively, in biopsies from patients with diabetic nephropathy compared with control tissue (Langham et al, 2003). Adult SPC-PDGFB transgenic mice exhibited lung pathology characterized by enlarged airspaces, inflammation, and fibrosis (Hoyle et al, 1999). The transition between hyperplasia (complete hydatidiform mole) and neoplasia (choriocarcinoma) in these cells correlates with at least a 10- to 20-fold activation of the PDGF-B gene (Holmgren et al, 1993).

*Transmembrane protease serine 6*. Transmembrane protease serine 6 (TMPRSS6) is a type II transmembrane serine proteinase that is found attached to the cell surface. The encoded protein may be involved in matrix remodeling processes in the liver (Entrez database).

*Myosin-18B.* Myosin-18B, also known as Myosin XVIIIb alias BK125H2.1, may regulate muscle-specific genes when in the nucleus and may influence intracellular trafficking when in the cytoplasm. The encoded protein functions as a homodimer and may interact with F actin. Mutations in this gene are associated with lung cancer (Entrez database). Genetic and epigenetic alterations of the MYO18B gene was analyzed in colorectal cancers. Alleic imbalance at the MYO18B locus was detected in 16 of 43 (40%) informative cases. Mutations of the MYO18B gene were detected in 2 of 11 (18%) cell lines and 1 of 47 (2%) surgical specimens. Nine of 11 (82%) cell lines showed reduced MYO18B expression, which was restored in all 9 by treatment with 5-aza-2'-deoxycytidine and/or trichostatin A (TSA). Although hypermethylation of the promoter CpG island for MYO18B was not detected, a significant correlation was observed between the level of MYO18B expression and the level of acetylation of histones H3 and H4 in 6 cell lines with and without TSA treatment (Nakano et al, 2005). Missense MYO18B mutations were detected in 1 of 4 (25%) ovarian cancer cell lines and in 1 of 17 (5.9%) primary ovarian cancers. MYO18B expression was reduced in all 4 ovarian cancer cell lines and in 12 of 17 (71 %) of primary ovarian cancers. MYO18B expression was restored by treatment with 5-aza-2'-deoxycytidine and/or trichostatin A in 3 of 4 cell lines with reduced MYO18B expression, and hypermethylation of the promoter CpG island for MYO18B was observed in 2 of these 3 cell lines. Its hypermethylation was also observed in 2 of 15 (13%) primary ovarian cancers (Yanaihara et al, 2004). MYO18B, located at chromosome 22q12.1 and found that it is frequently deleted, mutated, and hypermethylated in lung cancers (Nishioka et al, 2002).

*Forkhead box C1.* Forkhead box C1 (FOXC1, FKHL7, IRID1, FREAC3, ARA, IGDA, IHG1) belongs to the forkhead family of transcription factors which is characterized by a distinct DNA-binding forkhead domain. The specific function of this gene has not yet been determined; however, it has been shown to play a role in the regulation of embryonic and ocular development. Mutations in this gene cause various glaucoma phenotypes including primary congenital glaucoma, autosomal dominant iridogoniodysgenesis anomaly, and Axenfeld-Rieger anomaly (Entrez database).

*Disheveled associated activator of morphogenesis 2.* Disheveled associated activator of morphogenesis 2 (DAAM2, KIAA0381, MGC90515, dJ90A20A.1, RP1-278E11.1) regulates the morphogenetic movements of vertebrate gastrulation in a Wnt-dependent manner through direct interactions with Dsh/Dvi and RhoA (Nakaya et al, 2004). The observed expression patterns in developing central nervous tissues suggested that vertebrate Daam genes were involved in pivotal steps in neuronal cell differentiation and movement (Kida et al, 2004).

*Nesprin-1.* Nesprin-1 (Nuclear envelope spectrin repeat protein 1) is also known as synaptic nuclear envelope protein 1 (Syne-1), Myocyte nuclear envelope protein 1 (Myne-1) or Enaptin (SYNE1 alias SYNE-1 B, KIAA0796, 8B, nesprin-1, enaptin, MYNE1, CPG2). Transgenic mice overexpressing the conserved C-terminal Klarsicht/ANC-1/Syne homology domain of Syne-1 were generated. The transgene acted in a dominant interfering fashion, displacing endogenous Syne-1 from the nuclear envelope. Muscle nuclei failed to aggregate at the NMJ in transgenic mice, demonstrating that localization and positioning of synaptic nuclei require Syne proteins (Grady et al, 2005). Integral membrane protein nesprin-1alpha serves as a receptor for mAKAP on the nuclear envelope in cardiac myocytes (Pare et al, 2005). Syne-1 and KIF3B function together in cytokinesis by facilitating the accumulation of membrane vesicles at the spindle midbody (Fan and Beck, 2004 ). Syne-1 gene is expressed in a variety of forms that are multifunctional and are capable of functioning at both the Golgi and the nuclear envelope, perhaps linking the two organelles during muscle differentiation (Gough et al, 2003). Nesprin-1 is developmentally regulated in both smooth and skeletal muscle and is re-localized from the nuclear envelope to the nucleus and cytoplasm during C2C12 myoblast differentiation (Zhang et al, 2001).

*VARS.* VARS is also known as valyl-tRNA synthetase; ENSG00000096171; G7A or VARS2. Aminoacyl-tRNA synthetases catalyze the aminoacylation of tRNA by their cognate amino acid. Because of their central role in linking amino acids with nucleotide triplets contained in tRNAs, aminoacyl-tRNA synthetases are thought to be among the first proteins that appeared in evolution. The protein encoded by this gene belongs to class-I aminoacyl-tRNA synthetase family and is located in the class III region of the major histocompatibility complex (Entrez database).

*Major histocompatibility complex, class II, DP beta* 1. Major histocompatibility complex, class II, DP beta 1 is also known as OTTHUMG00000031076; HLA-DPB1; HLA-DPB1; DPB1; HLA-DP1B; MHC DPB1. HLA-DPB belongs to the HLA class II beta chain paralogues. This class II molecule is a heterodimer consisting of an alpha (DPA) and a beta chain (DPB), both anchored in the membrane. It plays a central role in the immune system by presenting peptides derived from extracellular proteins. Class II molecules are expressed in antigen presenting cells (APC: B lymphocytes, dendritic cells, macrophages). The beta chain is approximately 26-28 kDa and its gene contains 6 exons. Exon one encodes the leader peptide, exons 2 and 3 encode the two extracellular domains, exon 4 encodes the transmembrane domain and exon 5 encodes the cytoplasmic tail. Within the DP molecule both the alpha chain and the beta chain contain the polymorphisms specifying the peptide binding specificities, resulting in up to 4 different molecules (Entrez database).

*PRAME.* PRAME is also known as melanoma antigen preferentially expressed in tumors; preferentially expressed antigen of melanoma or OPA-interacting protein 4 (OIP4 alias ENSG00000185686; MAPE). This gene encodes an antigen that is predominantly expressed in human melanomas and that is recognized by cytolytic T lymphocytes. It is not expressed in normal tissues, except testis. This expression pattern is similar to that of other CT antigens, such as MAGE, BAGE and GAGE. However, unlike these other CT antigens, this gene is also expressed in acute leukemias. Five alternatively spliced transcript variants encoding the same protein have been observed for this gene (Entrez database).

*FBLN1.* FBLN1 is also known as fibulin 1 or ENSG00000077942. Fibulin 1 is a secreted glycoprotein that becomes incorporated into a fibrillar extracellular matrix. Calcium-binding is apparently required to mediate its binding to laminin and nidogen. It mediates platelet adhesion via binding fibrinogen. Four splice variants which differ in the 3' end have been identified. Each variant encodes a different isoform, but no functional distinctions have been identified among the four variants (Entrez database).

*GPR24.* GPR24 is also known as G protein-coupled receptor 24; ENSG00000128285; SLC1; MCHR1; MGC32129. The protein encoded by this gene, a member of the G protein-coupled receptor family 1, is an integral plasma membrane protein which binds melanin-concentrating hormone. The encoded protein can inhibit cAMP accumulation and stimulate intracellular calcium flux, and is probably involved in the neuronal regulation of food consumption. Although structurally similar to somatostatin receptors, this protein does not seem to bind somatostatin (Entrez database).

*TCN2.* TCN2 is also known as transcobalamin II; macrocytic anemia; ENSG00000185339 (alias TC2; D22S676; D22S750). This gene encodes a member of the vitamin B12-binding protein family. This family of proteins, alternatively referred to as R binders, is expressed in various tissues and secretions. This plasma protein binds cobalamin and mediates the transport of cobalamin into cells. This protein and other mammalian cobalamin-binding proteins, such as transcobalamin I and gastric intrisic factor, may have evolved by duplication of a common ancestral gene (Entrez database).

*APOBEC3B*. APOBEC3B is also known as apolipoprotein B mRNA editing enzyme, catalytic polypeptide-like 3B; ENSG00000179750 (alias ARP4; ARCD3; PHRBNL; APOBEC1L; FLJ21201; DJ742C19.2). This gene is a member of the cytidine deaminase gene family. It is one of seven related genes or pseudogenes found in a cluster, thought to result from gene duplication, on chromosome 22. Members of the cluster encode proteins that are structurally and functionally related to the C to U RNA-editing cytidine deaminase APOBEC1. It is thought that the proteins may be RNA editing enzymes and have roles in growth or cell cycle control (Entrez database).

*CRYBA4.* CRYBA4 is also known as crystallin, beta A4 or ENSG00000196431. Crystallins are separated into two classes: taxon-specific, or enzyme, and ubiquitous. The latter class constitutes the major proteins of vertebrate eye lens and maintains the transparency and refractive index of the lens. Since lens central fiber cells lose their nuclei during development, these crystallins are made and then retained throughout life, making them extremely stable proteins. Mammalian lens crystallins are divided into alpha, beta, and gamma families; beta and gamma crystallins are also considered as a superfamily. Alpha and beta families are further divided into acidic and basic groups. Seven protein regions exist in crystallins: four homologous motifs, a connecting peptide, and N- and C-terminal extensions. Beta-crystallins, the most heterogeneous, differ by the presence of the C-terminal extension (present in the basic group, none in the acidic group). Beta-crystallins form aggregates of different sizes and are able to self-associate to form dimers or to form heterodimers with other beta-crystallins. This gene, a beta acidic group member, is part of a gene cluster with beta-B1, beta-B2, and beta-B3 (Entrez database).

*RPL3.* RPL3 is also known as ribosomal protein L3 or ENSG00000100316 alias (TARBP-B; MGC104284). Ribosomes, the complexes that catalyze protein synthesis, consist of a small 40S subunit and a large 60S subunit. Together these subunits are composed of 4 RNA species and approximately 80 structurally distinct proteins. This gene encodes a ribosomal protein that is a component of the 60S subunit. The protein belongs to the L3P family of ribosomal proteins and it is located in the cytoplasm. The protein can bind to the HIV-1 TAR mRNA, and it has been suggested that the protein contributes to tatmediated transactivation. This gene is co-transcribed with several small nucleolar RNA genes, which are located in several of this gene's introns. Alternate transcriptional splice variants, encoding different isoforms, have been characterized. As is typical for genes encoding ribosomal proteins, there are multiple processed pseudogenes of this gene dispersed through the genome (Entrez database).

*PTPNS1.* PTPNS1 is also known as protein tyrosine phosphatase, non-receptor type substrate 1 or ENSG00000198053 (alias BIT; MFR; P84; SIRP; MYD-1; SHPS1; SHPS-1; SIRPalpha; SIRPalpha2; SIRP-ALPHA-1 The protein encoded by this gene is a member of the signal-regulatory-protein (SIRP) family, and also belongs to the immunoglobulin superfamily. SIRP family members are receptor-type transmembrane glycoproteins known to be involved in the negative regulation of receptor tyrosine kinase-coupled signaling processes. This protein can be phosphorylated by tyrosine kinases. The phosphotyrosine residues of this PTP have been shown to recruit SH2 domain containing tyrosine phosphatases (PTP), and serve as substrates of PTPs. This protein was found to participate in signal transduction mediated by various growth factor receptors. CD47 has been demonstrated to be a ligand for this receptor protein. This gene and its product share very high similarity with several other members of the SIRP family. These related genes are located in close proximity to each other on chromosome 20p13 (Entrez database).

*EYA2.* EYA2 is also known as eyes absent homolog 2 (Drosophila) or ENSG00000064655 (alias EAB1; MGC10614). This gene encodes a member of the eyes absent (EYA) family of proteins. The encoded protein may be post-translationally modified and may play a role in eye development. A similar protein in mice can act as a transcriptional activator. Five transcript variants encoding three distinct isoforms have been identified for this gene (Entrez database).

*Solute carrier family* 35 *member E4.* Solute carrier family 35 member E4 is also known as SLC35E4.

*RP4-695020_B.9*. RP4-695020_B.9 is also known as dJ695020B.C22.9.

*Cat eye syndrome critical region protein 1 precursor.* Cat eye syndrome critical region protein 1 precursor (CECR1 alias IDGFL) is member of a subfamily of the adenosine deaminase protein family. The encoded protein may act as a growth factor and have adenosine deaminase activity. It may be responsible for some of the phenotypic features associated with cat eye syndrome. Two transcript variants encoding distinct isoforms have been identified for this gene (Entrez database).

*Transcription factor 19.* Transcription factor 19 is also known as Transcription factor SC1 (TCF19_HUMAN or SC1 ).

### SUMMARY OF THE INVENTION

The present invention provides novel and efficacious methods and nucleic acids for the classification of biological samples.

The subject matter of the invention has specific utility in the fields of medicine and/or molecular biology. In a particular aspect the utility of the present invention is to provide molecular markers and methods for the analysis thereof that may be considered an alternative to traditional histological or pathological analysis. Said molecular biological markers accordingly offer an alternative to current means such as staining and microscopic analysis.

In a particular aspect, the present invention is of particular use in determining the presence or absence of specific organ, tissue or cell types in a biological sample. Wherein said sample is heterogeneous in nature, the method according to the present invention may be used for the identification of a population or subpopulation of specific organs, tissue or cell types.

In a particular aspect, the present invention has further utility in the detection and/or classification of a cell proliferative disorder, for example but not limited to cancer.

In a particular aspect, the method of the present invention has a further alternative utility in the analysis of cellular differentiation, for example in the field of tissue engineering.

In a particular aspect, the invention solves this longstanding need in the art by providing genes, genomic sequences and/or regulatory regions thereof according to Table 1 (or to one or more of those), the expression thereof being indicative of the class of biological sample.

In a particularly preferred aspect, the methylation status of CpG positions of genes, genomic sequences and/or regulatory regions thereof according to Table 1 is used in the classification of a biological sample.

According to the invention, the provided markers, in particular the genes, genomic sequences, regulatory regions, and corresponding mRNAs, cDNAs, proteins or peptides have a particular utility in the following aspects. Thereby a single marker is used either alone or in combination with other marker or markers herein provided or not.

The herein provided markers have utility (i) for the characterization of the marker corresponding tissue or cell, (ii) for the identification of marker corresponding tissue or cell, (iii) for the isolation of marker corresponding tissue or cell, (iv) for the purification of the corresponding tissue or cell, or (v) combinations thereof. Therefore known methods, so far unreported methods, or combinations thereof are useable. Said application is useful in the field of research, diagnostics as well as therapeutics.

In addition, the herein provided markers have utility for the prospective profiling, retrospective profiling, or both of donors and/or recipients in organ transplantation procedures. The correct characterization, identification, or both of the donor and/or the recipient is mandatory during organ transplantation procedures to assure the success of the intervention. The use of the markers of the invention enables the profiling of both, donor and recipient, form which prospective or retrospective observations or conclusions about the feasibility of the procedure are drawn.

In addition, the herein provided markers have utility for histological, chemical and/or immunohistochemical analysis. Accordingly, they have utility in the fields of research as well as diagnostics, in particular for histological or pathological analysis.

In addition, the herein provided markers have utility for phylogenetic profiling of species or tissues. The ontogenetic origin or the developmental lineage is then determined by comparison of the determined profiles.

In addition, the herein provided markers have utility for quality control of a genetically modified organism, tissue, group of cells or cell.

In addition, the herein provided markers have utility for controlling side effects in in vivo gene therapy procedures wherein genetically modified organism, tissue, group of cells or cell is used.

In addition, the herein provided markers have utility for the characterization, identification, or labelling of corresponding tissue or combinations thereof. This is of particular utility in the field of tissue bank storage and proliferation. Furthermore it has utility in a prospective as well as in a retrospective manner. The provided markers allow the individualization of samples by a precise molecular method. This is mandatory in storing biological material from patients or healthy individuals. In addition, this also advantageous for isolation or purification of tissues cells.

In addition, the herein provided markers have utility for controlling cell differentiation in stem-cell research and/or therapeutics. Cells undergo many genetic and/or epigenetic changes throughout differentiation. These changes influence the physiology of the cell and their control is mandatory in any procedure involving stem-cell in research and/or therapeutics. The provided markers allow to control this changes by giving a reference of the adult (completely differentiated) and embryonic (partially differentiated) status of the cells.

CD4+ and CD8+ lymphocytes:
The herein provided markers of Table 8A and Table 9A have util ity for the quantification of lymphocytes, in particular in peripheral blood. The said markers enable the identification of CD4+ and CD8+ lymphocytes among other cells in blood samples. A low number of leucocytes in blood (leucopenia) may indicate bone marrow failure (for example, due to infection, tumor, fibrosis); presence of cytotoxic substance; collagen-vascular diseases (such as lupus erythematosus); disease of the liver or spleen; or radiation. A high number of leucocytes in blood (leucocytosis) may indicate infectious diseases; inflammatory disease (such as rheumatoid arthritis or allergy); leukemia; severe emotional or physical stress; tissue damage (for example, bums); or anemia.
In addition, the herein provided markers of Table 8A and Table 9A have utility for the study of CD4 and/or CD8 T-lymphocyte infiltration in other tissues healthy or diseased. Infiltration of lymphocytes in healthy or diseased tissues is an indication of several diseases such immunological malignances or even in tumor development. The said markers represent a target for the development of molecular probes that coupled to any detection method (e.g. Fluorescent dye) allow the identification of these cells in histological preparations.
In addition, the herein provided markers of Table 8A and Table 9A have utility for identification, isolation and/or purification of CD4 T-lymphocytes and/or CD8 T-lymphocytes, in particular from surrounding tissue infiltrated by the T-lymphocytes; from blood; and/or from other body fluids.
In addition, the herein provided markers of Table 8A and Table 9A have utility for the identification of an individual. Thereby at least two samples are used. One samples is obtained from an individual and another sample is a forensic sample, in particular traces of body cells, tissues or fluids, for example but not limited to, traces of blood and/or body fluids. This is of particular utility in the field of forensic medicine or of legal medicine. As constituent of blood or body fluids, CD4 T-lymphocytes and CD8 T-lymphocytes are part of the mentioned traces. The said markers have the advantage of being stable over time because they are DNA based. In addition said markers have the advantage that they enable a highly detailed and accurate characterization of samples. Through this an unambiguous identification of an individual is enabled.
In addition, the herein provided markers of Table 8A and Table 9A have utility for diagnosing the presence or absence of a disease. Thereby the number of CD4 T-lymphocytes, CD8 T-lymphocytes or both is quantified in normalized samples of healthy individuals. The determined number of CD4 T-lymphocytes, CD8 T-lymphocytes or both are then considered as indicative for healthy condition or a diseased condition with respect to an individual. Preferably, large amount of normalized samples are considered to generate reference values of CD4 T-lymphocytes, CD8 T-lymphocytes or both for a healthy condition and/or for one or more diseased conditions. The diseased condition can be any kind of diseased condition. Preferably, the diseased condition is a disease which causes a immune reaction. For example but not limited to the diseased condition is a cancer disease, a cell proliferation disease, or HIV. Preferably the total number of cells present in a sample is determined. The number of CD4 T-lymphocytes, CD8 T-lymphocytes or both are then normalized to the total number of cells.

### Embryonic

The herein provided markers of Table 8B, Table 8C, Table 9B and Table 9C have utility for the study, identification and/or quantification of fetal cells or fetal DNA circulating in maternal blood and /or amniotic fluid. During pregnancy cells and DNA from the fetus are continuously brought to the maternal blood stream as well as the amniotic fluid. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101).

In addition, the herein provided markers of Table 8B, Table 8C, Table 9B and Table 9C have utility for the study, identification and/or quantification of fetal cells or fetal DNA from amniocentesis and/or chorionic villus sampling. This is of particular utility in the field of prenatal diagnosis. Prenatal diagnosis procedures involve the study of fetal cells obtained by amniocentesis and chorionic villus biopsies.

### Skin

The herein provided markers of Tables 8D, G, I and Tables 9D, F, H have utility for identifying individuals from traces of skin and/ or adjacent tissues (such as hair, nail pieces, etc). This is of particular utility in forensic medicine and/or legal medicine. Skin or skin adjacent tissue is usually used as study material in forensic and legal medicine. The markers provided in Table 8G and 9F have a particular utility because of the following reason. Keratinocytes constitute the external layer of the skin and therefore are the first cell type to be de-attached and a high number of these cells is expected in skin traces. Variations of one marker alone or in combination with other markers herein provided or not enable the accurate assessment of identity.

In addition, the herein provided markers of Tables 8D, G, I and Tables 9D, F, H have utility for characterizing the skin, hair, nail, or adjacent tissue of an individual.

In addition, the herein provided markers of Tables 8D, G, I and Tables 9D, F, H have utility for determining the composition of the skin, hair, nail, or adjacent tissue of an individual. Said composition being dependent from the content of at least one of the three major constituting cell types of the skin (fibroblasts, keratinocytes and melanocytes).

In addition, the herein provided markers of Tables 8D, G, I and Tables 9D, F, H have utility in the field of drugs. They have particular utility for the development of drugs as well as for the treatment with drugs. The skin, hair, nail or adjacent tissue of an individual can be characterized by means of the provided markers of Tables 8D, G, I and Tables 9D, F, H. This information can then be used to develop new drugs or to access already existing drugs with regard to skin, hair, nail etc. of an individual or to subgroups of individuals. These subgroups are for example but not limited to be characterized by a disease and/or a defined type of skin or hair, etc. The efficiency of said drugs i.e. the presence or absence of the desired effect is also characterized or monitored by means of the provided markers of Tables 8D, G, I and Tables 9D, F, H.

In addition, the herein provided markers of Tables 8D, G, I and Tables 9D, F, H have utility as prognostic and/or diagnostic markers for wound healing, in particular in the field of surgery procedures wherein the skin is affected.

### Liver

The herein provided markers of Tables 8H and Tables 9G have utility for deducing the presence of absence of an event which affects the liver. For example but not limited to it, said event is at least one select from the group comprising liver cirrhosis; liver cancer; hepatitis A; hepatitis B; hepatitis C; healthy condition, recently or longer chemical, physical or biological exposure; recently or longer exposure to a drug, or alcohol; or treatment procedures. In the case the event is adverse, said event affecting the liver leads to the death of liver cells. In the case the event is benign, said event leads to a reduction of liver cell death. The genomic DNA of dead liver cells can then be found in the body fluids in particular in the blood of a affected individual.

In addition, the herein provided markers of Tables 8H and Tables 9G have utility for deducing the sensitivity of an individual to alcohol. Alcohol consumption may change the DNA methylation status as reviewed by Poschl et al, 2004 (Poschl G, Stickel F, Wang XD, Seitz HK. Alcohol and cancer: genetic and nutritional aspects. Proc Nutr Soc. 2004 Feb;63(1 ):65-71.).

### Heart Muscle

The herein provided markers of Tables 8E, Table 8F and Tables 9E have utility for deducing the presence of absence of an event or condition affecting the heart. For example but not limited to it, said event or condition is at least one select from the group comprising heart failure; heart attack; athletic capacity; healthy condition; recently or longer chemical, physical or biological exposure; recently or longer exposure to a drug; or treatment procedure. In the case the event is adverse, said event or condition affecting the heart leads to death of heart cells. In the case the event is benign, said event leads to a reduction of heart cell death. The genomic DNA of dead heart cells can then be found in the body fluids in particular in the blood of an affected individual.

### Placenta

The herein provided markers of Table 8J and Table 91 have utility for the study, monitoring, identification and/or quantification of placental cells or placental DNA circulating in maternal blood and /or amniotic fluid. In this respect, the said markers have also utility for the isolation or purification of placental cell or placental genomic DNA. Placenta constitute an extra-embryonic fetal tissue and as such, it shares many genetic characteristics with the fetal tissue. Therefore, cells from the placenta as well as DNA from placental cells can surrogate fetal cells and fetal DNA for diagnostic means. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101). During pregnancy placenta cells are de-attached and brought to the maternal blood stream as well as the amniotic fluid.

In addition, the herein provided markers of Table 8J and Table 91 have utility for the monitoring of embryonic development or the monitoring of placental development, in particular of extra-embryonic tissue or of interaction of extra-embryonic tissue with maternal placental tissue.

In addition, the herein provided markers of Table 8J and Table 91 have utility for the study, monitoring, identification and/or quantification of placental cells in regenerative medicine, in particular in the field of tissue engineering. Corresponding methods for the study, monitoring, identification and/or quantification of placental cells are applied in particular before and after storage, before and after cell differentiation, before and after cell proliferation, before and after cell culture expansion, and before and after tissue expansion as well as before and after transplantation.

### Sperm

The herein provided markers of Table 8L have utility for diagnosing a male infertility related disease. A major cause of male infertility is either a low amount of sperm cells (spermatozoa) in the ejaculate (oligospermia) or a complete lack of sperm cells (spermatozoa) in the ejaculate (azoospermia). Thus, methods for the quantification of sperm cells are widely useable in diagnosing male infertility.

In addition, the herein provided markers of Table 8L have utility as a tool to access the viability of the sperm cells.

In addition, the herein provided markers of Table 8L have utility for increasing the fertility of a male individual. As said above male fertility is often limited by the amount of sperm cells in the ejaculate. Thus, male fertility can be enhanced by enriching, isolating or purifying sperm cells.

In addition, the herein provided markers of Table 8L have utility for assisted fertilization procedures. Assisted fertilization procedures are for example but not limited to intracytoplasmic sperm injection (ICSI) or in vitro fertilization (IVF). All assisted fertilization procedures require the management of sperm cells prior to the procedure. Such management comprises at least the characterization, identification, quantification, enrichment, isolation, purification of sperm cells or combinations thereof.

In addition, the herein provided markers of Table 8L have utility in the fields of forensic and/or legal medicine. By use of the said markers it is possible to determine the presence or absence of sperm in a sample. Furthermore, it is possible to identify an individual by use of said markers.

### Skeletal Muscle

The herein provided markers of Table 8F, 8K and Table 9J have utility for characterizing the efficiency of skeletal muscle cells. This utility is of particular value in the field of sports medicine.

In addition, the herein provided markers of Table 8F, 8K and Table 9J have utility for identifying fully differentiated muscle cells in cell culture. This is of particular utility in the field of tissue engineering. Muscle cells are generate in cell culture by cultivation and differentiation of muscle cell progenitor cells. Fully differentiated skeletal muscle cells can be identified by means of the provided markers of Table 8F, 8K and Table 9J.

In addition, the herein provided markers of Table 8F, 8K and Table 9J have utility for diagnosing muscle cell associated diseases, in particular disease which are characterized by a death of muscle cells like muscular distrophy. The DNA of dead muscle cells is found in body fluids such as blood or urine. This DNA can be identified by means of the herein provided markers of Table 8F, 8K and Table 9J.

### CD8 T-lymphocytes

The herein provided markers of Table 8A specific only for CD8 T-lymphocytes have utility for quantifying CD8 T-lymphocytes, in particular for monitoring the immune system of individuals infected with HIV. The periodically determining of the number of CD8 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection.

### CD4+ lymphocytes

The herein provided markers of Table 8A specific only for CD4 T-lymphocytes have utility for quantifying CD4 T-lymphocytes, in particular for monitoring the immune system of individuals infected with HIV. The periodically determining of the number of CD4 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection.

It is particularly preferred that said biological sample is classified according to at least one parameter selected from the group consisting of the cell, organ or tissue type of said biological sample or features thereof such as disease state.

To enable this analysis the invention provides a method for the analysis of biological samples for genomic methylation associated with the classification of biological samples. Said method is characterized in that at least one nucleic acid, or a fragment thereof, from the group consisting of SEQ ID NO: 413 TO SEQ ID NO: 824 is/are contacted with a reagent or series of reagents capable of distinguishing between methylated and non methylated CpG dinucleotides within the genomic sequence, or sequences of interest.

It is particularly preferred that the method and nucleic acids according to the invention are utilised for at least one of histological analysis, pathological analysis, detection and/or characterization of cell proliferative disorders and monitoring of cellular or tissue differentiation.

The DNA source may be any suitable source. Preferably, the source of the DNA sample is selected from the group consisting of cells or cell lines, histological slides, biopsies, surgical samples, tissue samples, body fluids, sputum, stool, nipple aspirate, cerebrospinal fluid, ejaculate, urine, serum, plasma, whole blood, saliva, fluids from the pleural or peritoneal cavity, cerebrospinal fluid or a smear from a epithelial surface and combinations thereof.

Furthermore, said sample may be fresh or archived and can be treated by any means standard in the art, for example but not limited to fresh-frozen, paraffin-embedded or formalin-fixed sample.

Specifically, the present invention provides a method for classifying a biological sample, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of a gene or genomic sequence selected from Table 1 said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably said target sequences are selected from Table 3, which provides particularly preferred regions of the sequences of Table 1. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the equivalent converted sequence selected from Table 1, and contiguous regions thereof corresponding to the target sequence. It is further preferred that said converted sequences are selected from Table 3, which provides particularly preferred regions of the genes according to Table 1 (converted sequences thereof provided in Table 2).

Additional embodiments provide a method for the classification of a biological sample, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a converted sequence selected from Table 1 and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence of a gene or genomic sequence selected form Table 1, or an average, or a value reflecting an average methylation state of a plurality of CpG dinucleotide sequences thereof.

Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a converted sequence selected from Table 2 and complements thereof; hybridizing at least one nucleic acid molecule, bound to a solid phase, comprising a contiguous sequence at least 9 nucleotides in length that is complementary to, or hybridizes under moderately stringent or stringent conditions to a converted sequence selected from Table 2, and complements thereof; hybridizing at least one nucleic acid molecule comprising a contiguous sequence at least 9 nucleotides in length that is complementary to, or hybridizes under moderately stringent or stringent conditions to a sequence selected from a converted sequence selected from Table 2 (SEQ ID NO: 1650 TO SEQ ID NO: 4120), and complements thereof, and extending at least one such hybridized nucleic acid molecule by at least one nucleotide base; and sequencing of the amplificate.

Further embodiments provide a method for classifying a biological sample , comprising: obtaining a biological sample having subject genomic DNA; extracting the genomic DNA; contacting the genomic DNA, or a fragment thereof, comprising one or more sequences selected from the group consisting of SEQ ID NO: 413 TO SEQ ID NO: 824 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NO: 413 TO SEQ ID NO: 824, or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO: 413 TO SEQ ID NO: 824.

### BRIEF DESCRIPTION OF THE DRAWINGS

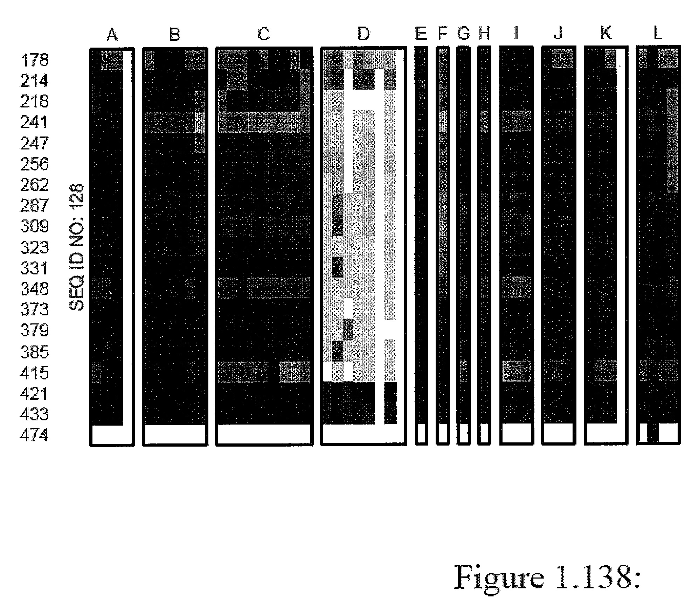

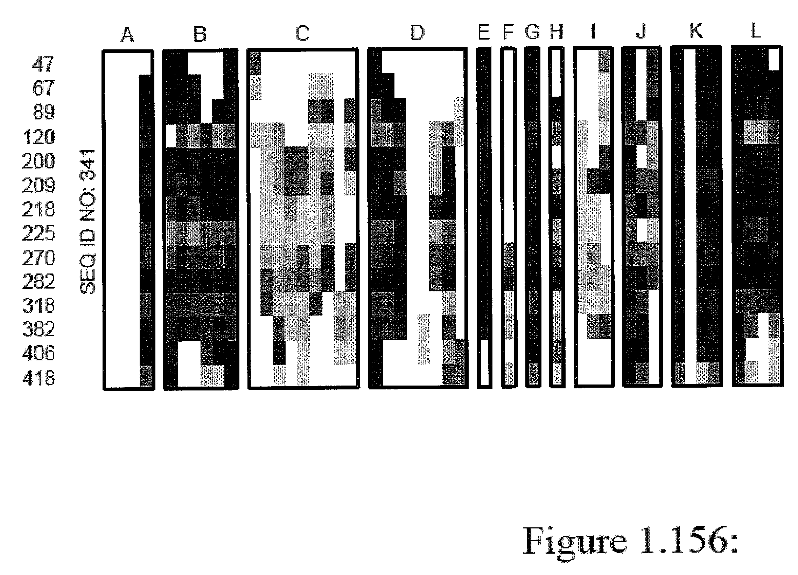

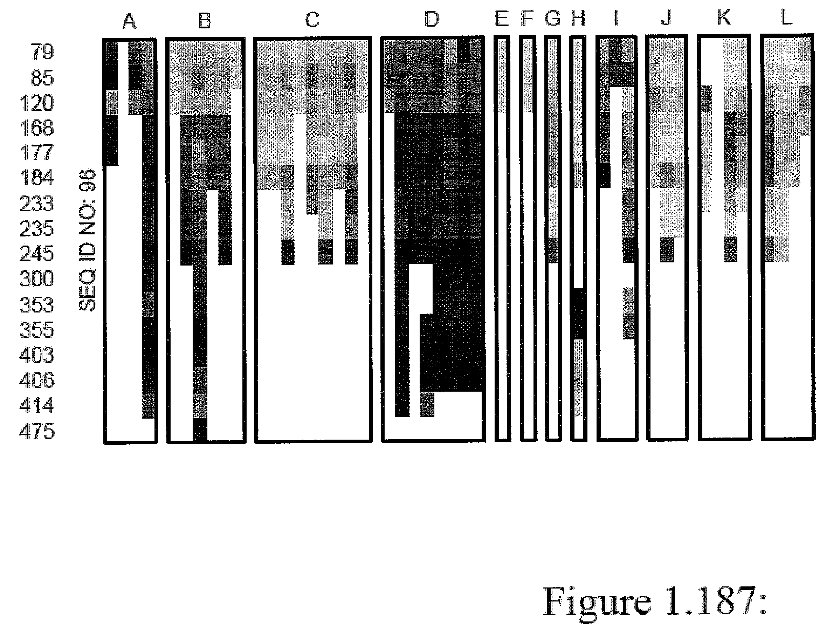

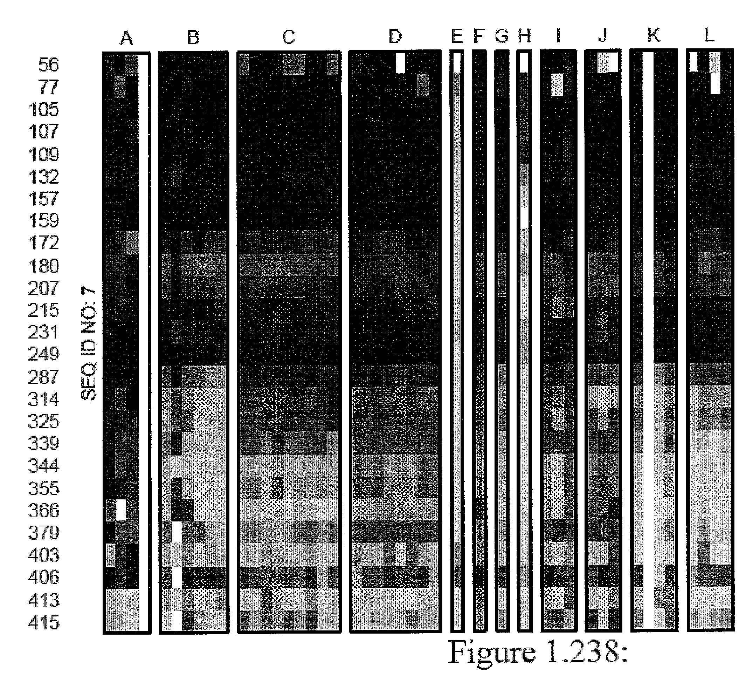

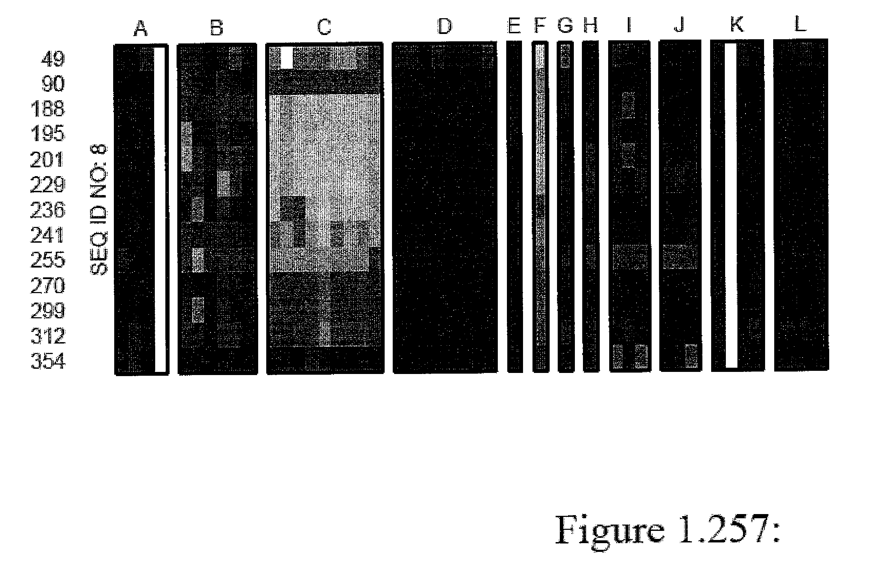

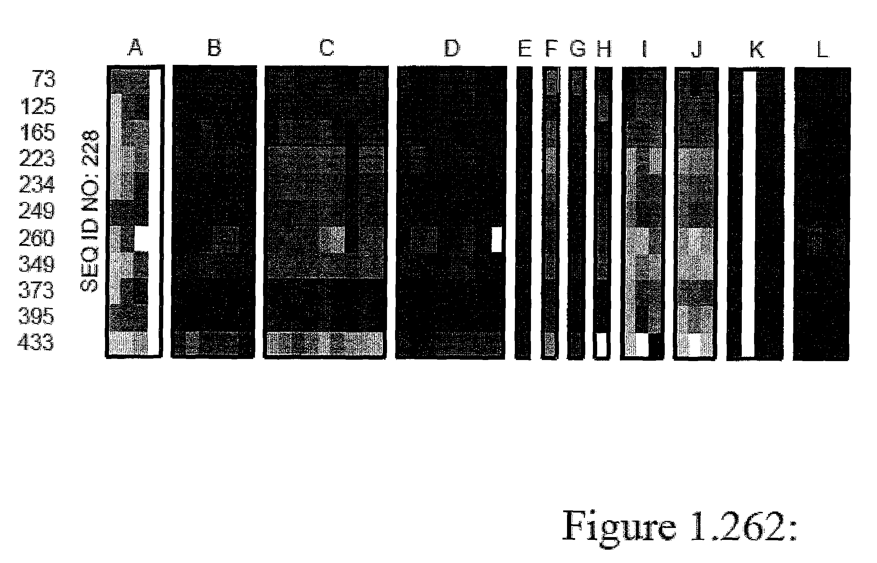

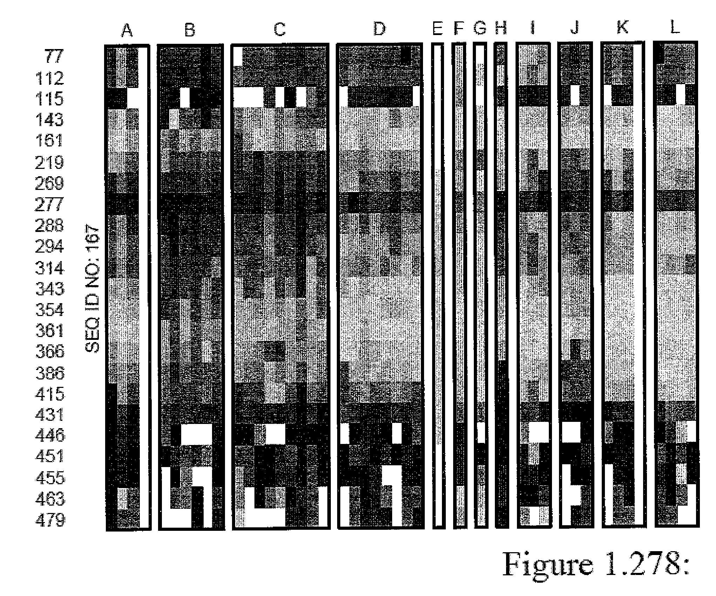

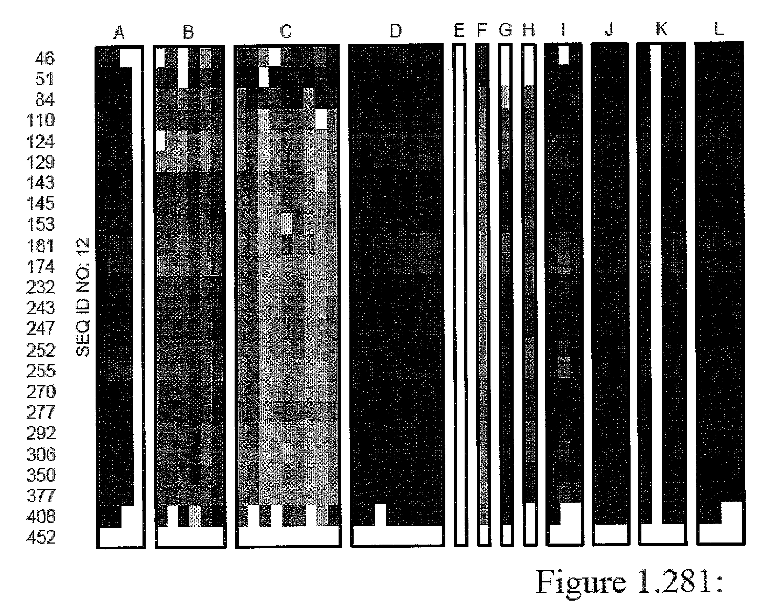

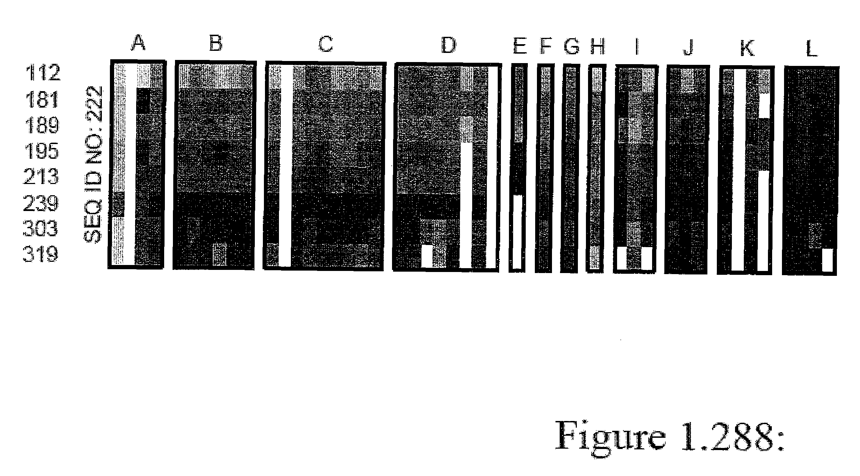

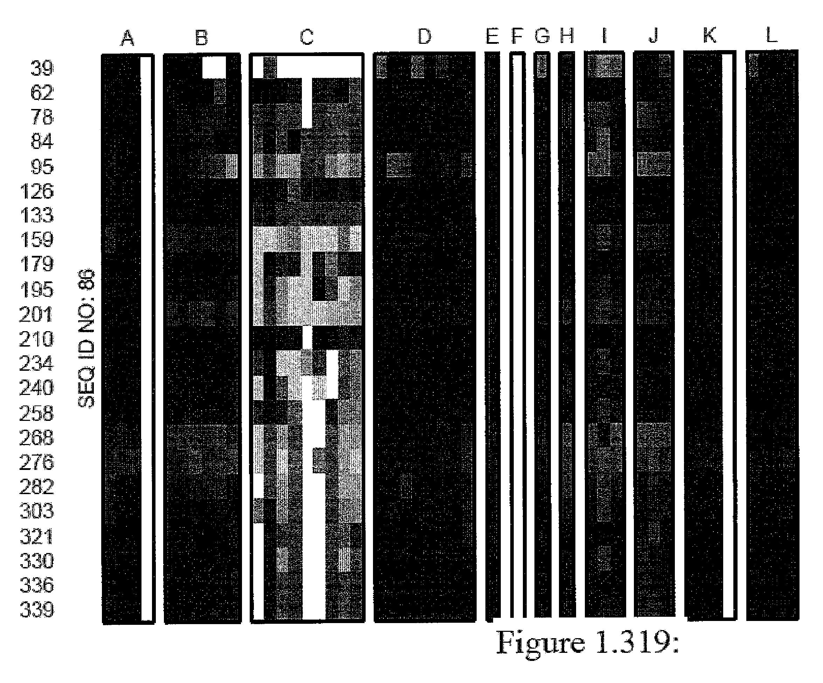

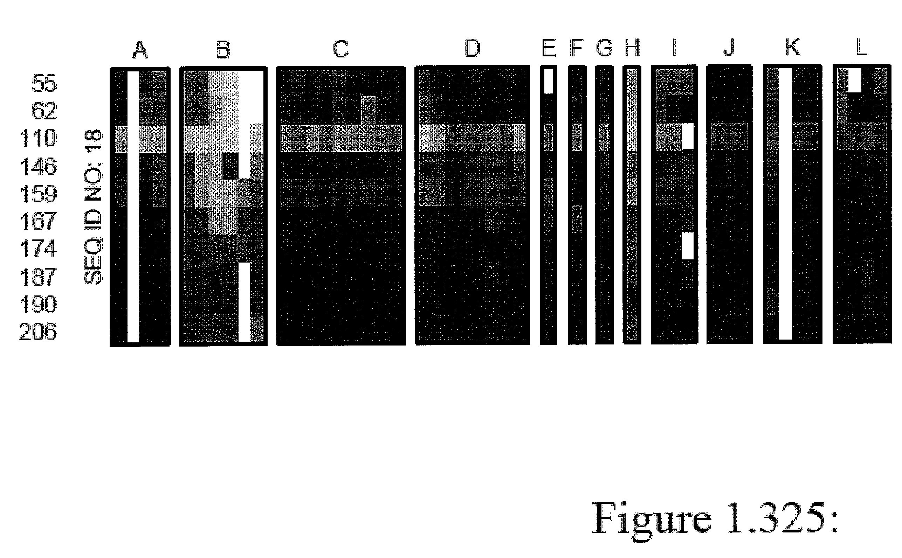

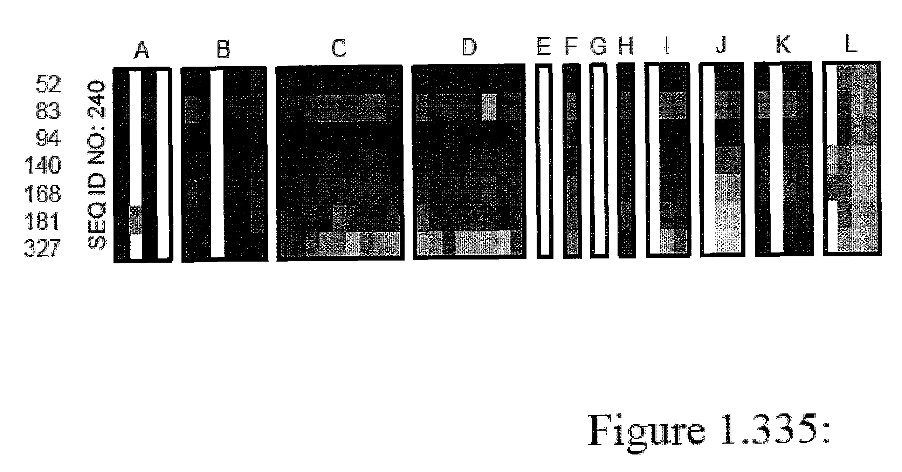

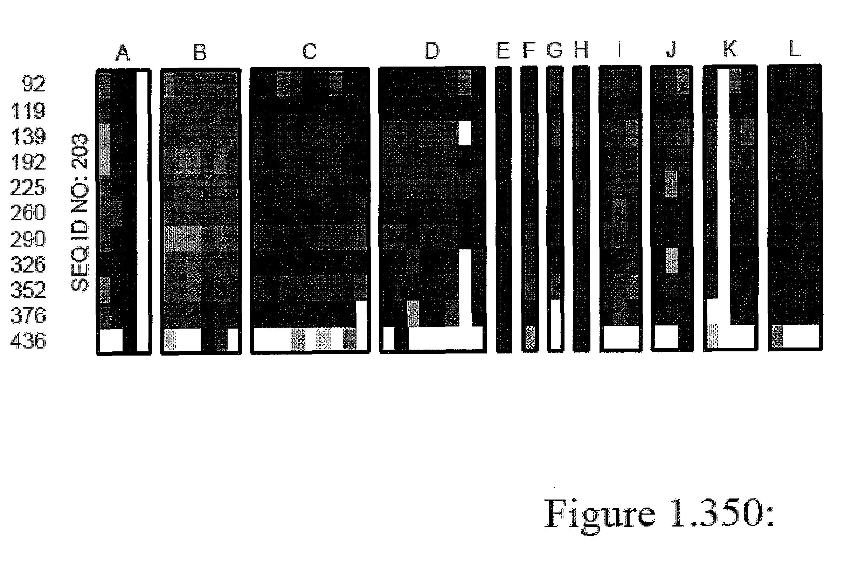

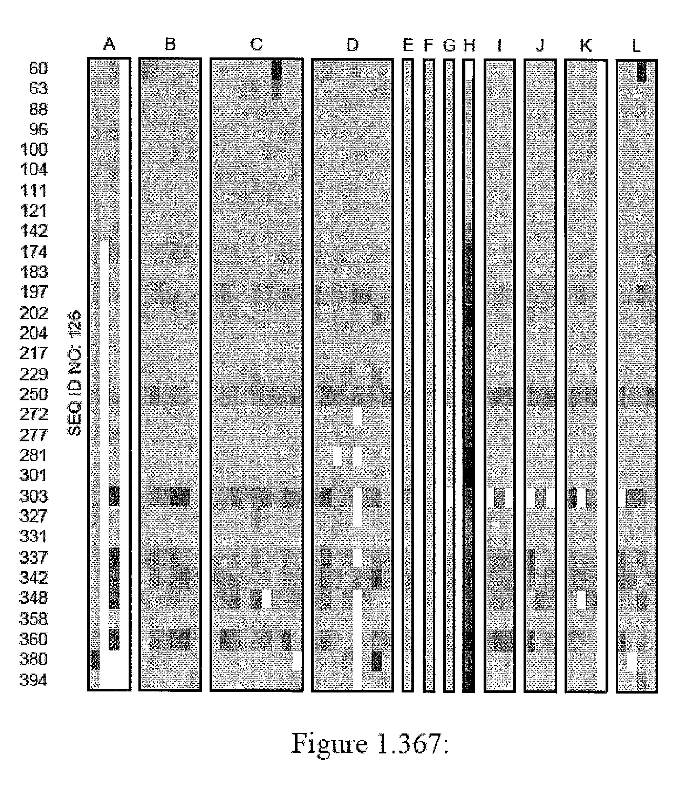

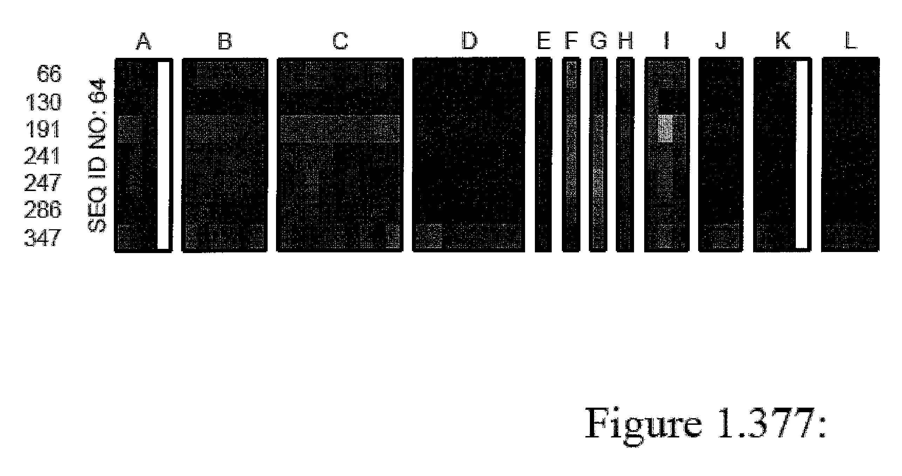

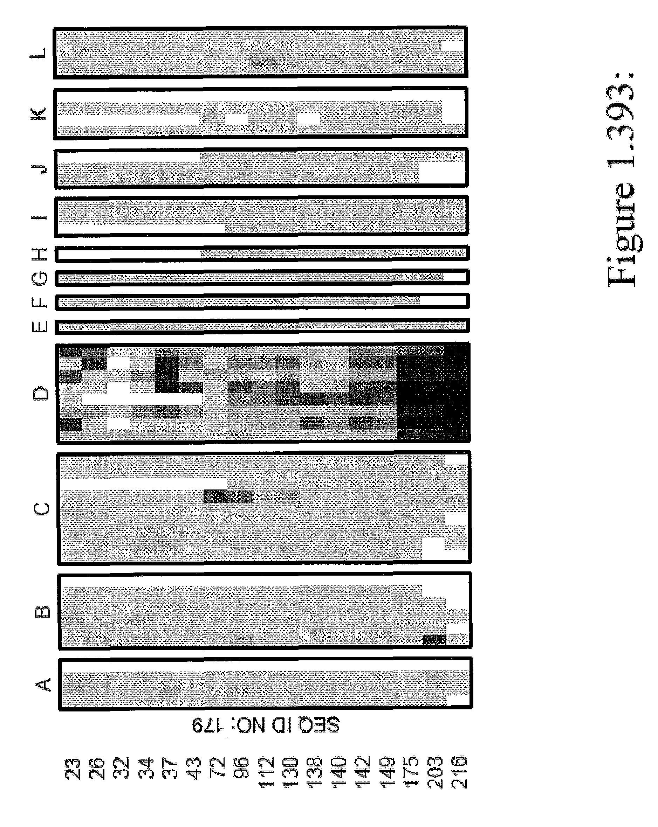

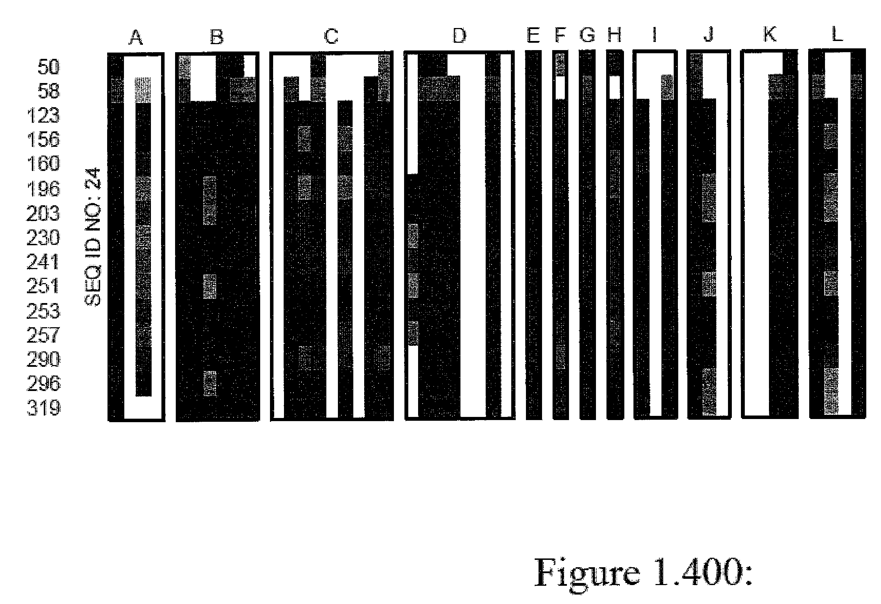

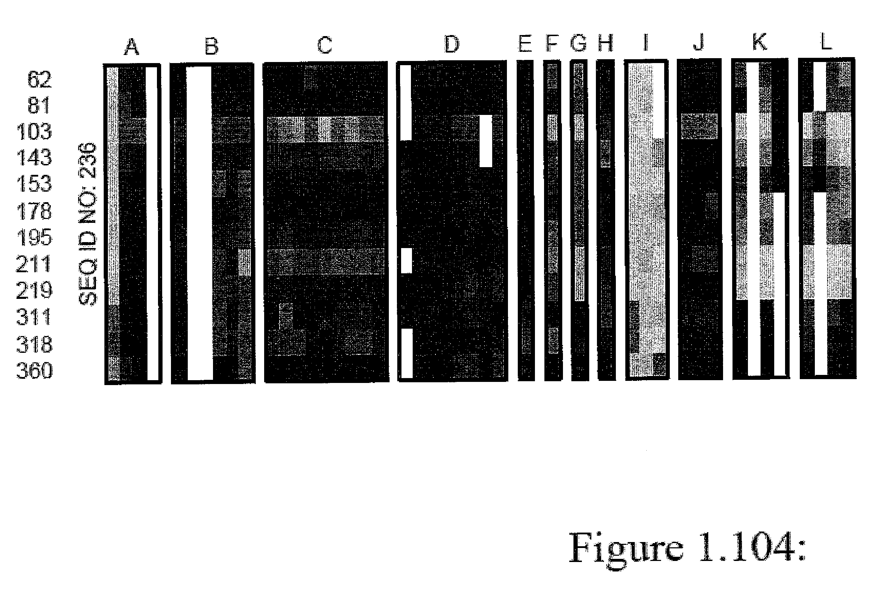

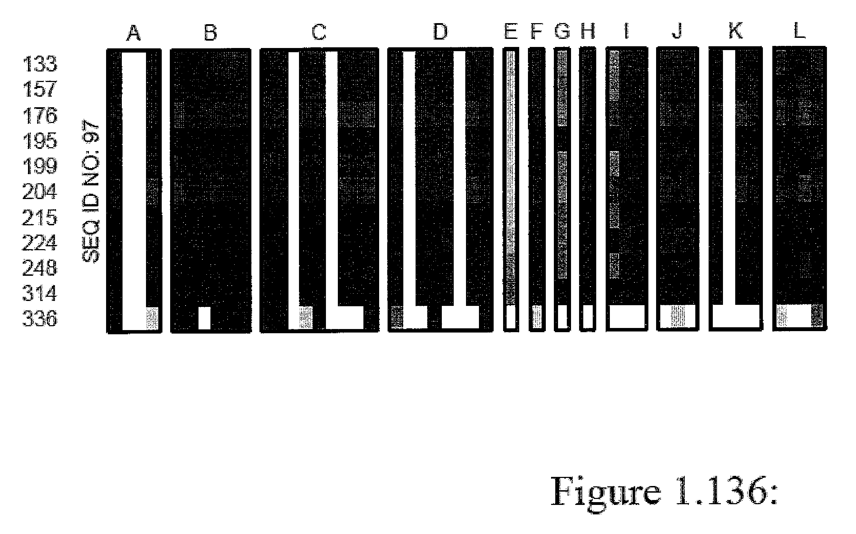

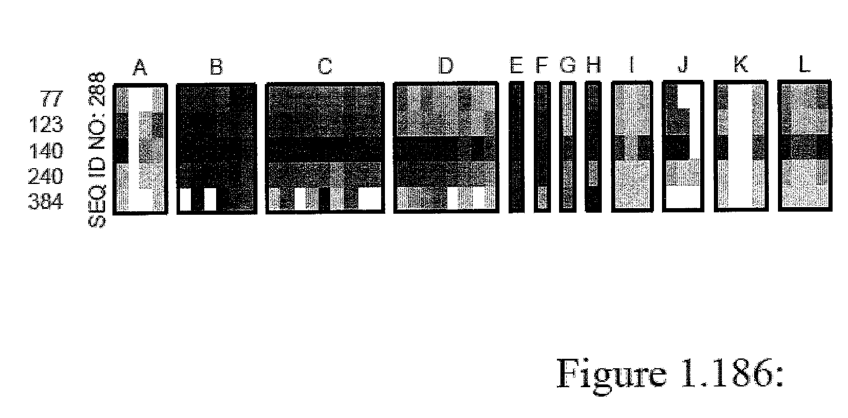

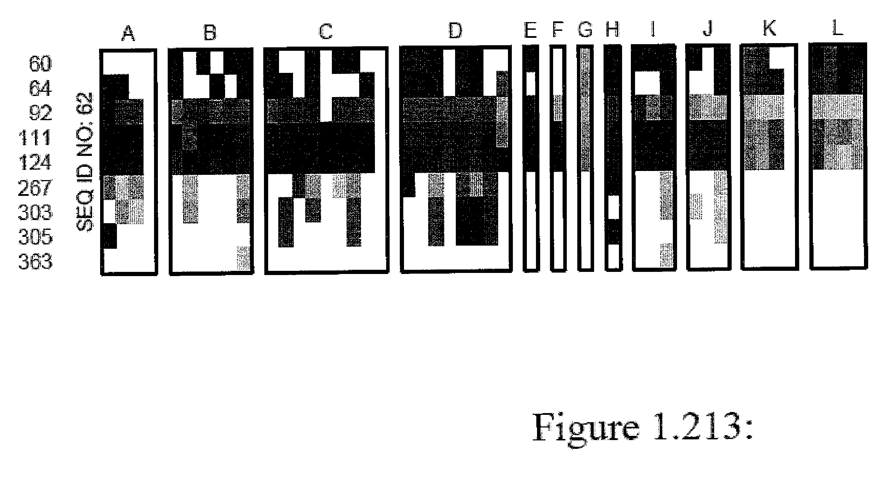

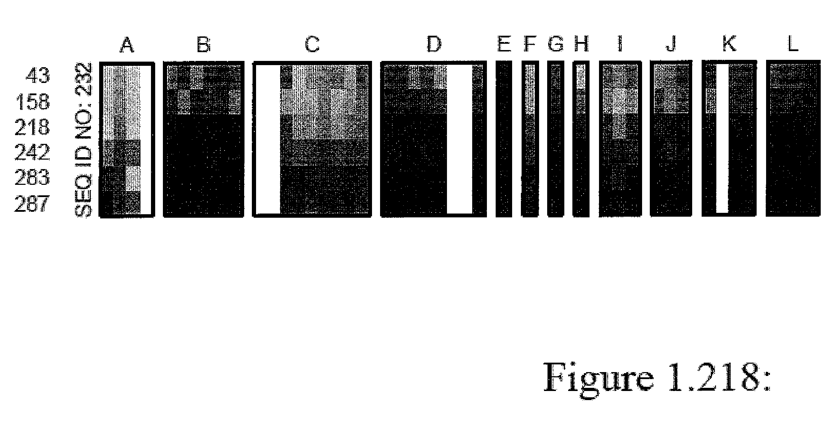

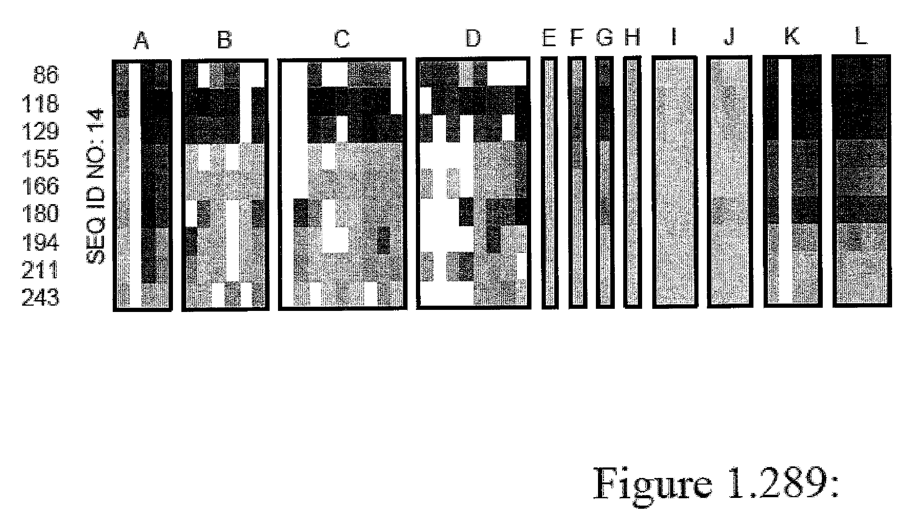

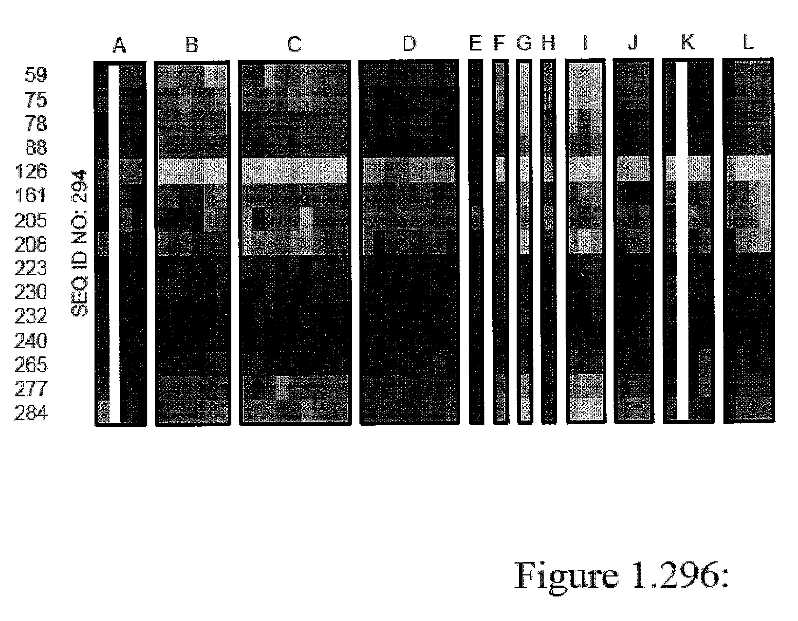

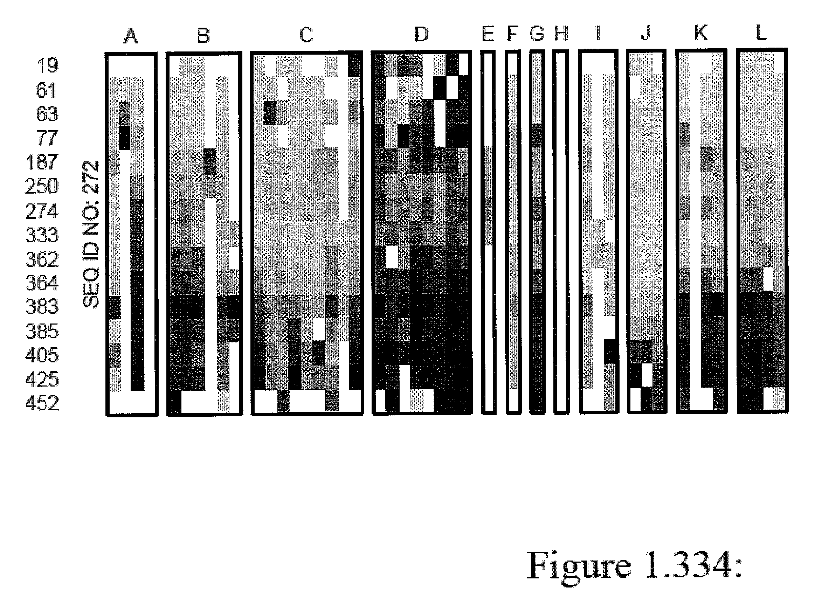

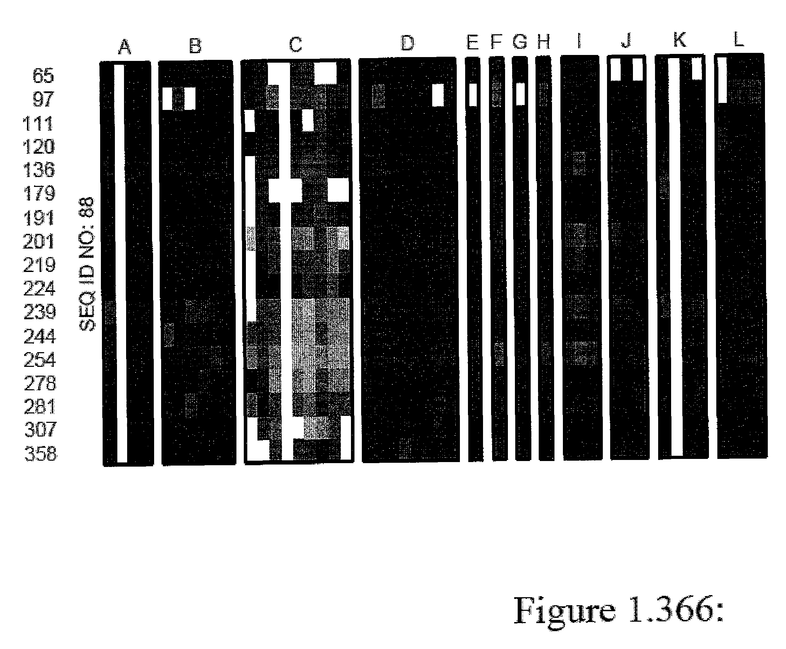

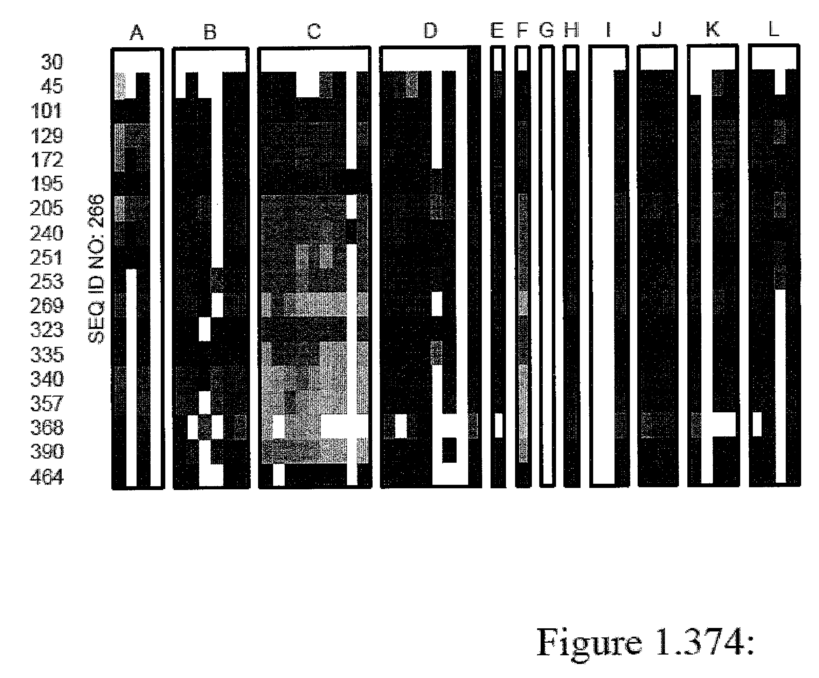

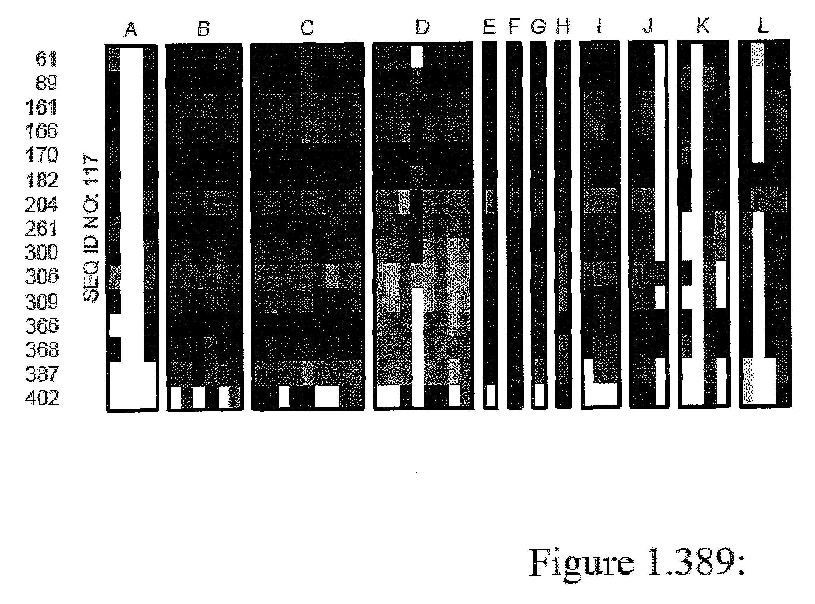

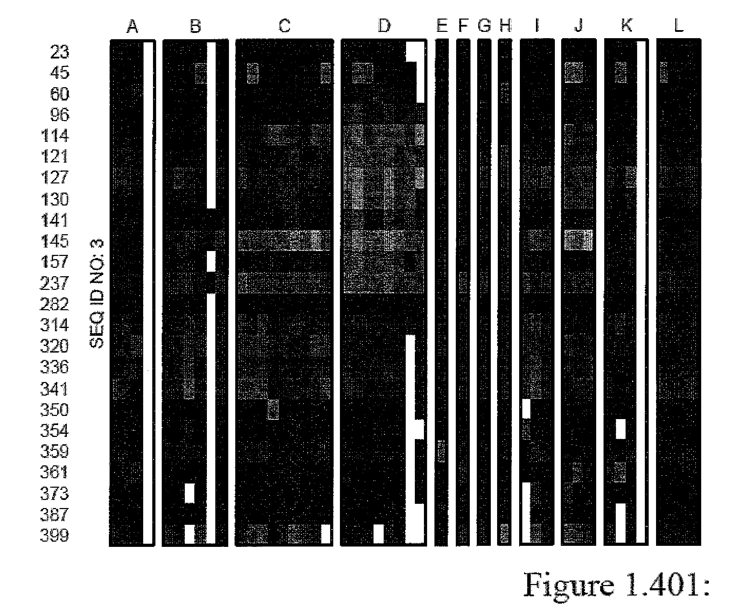

Figure 1.1 to 1.403 provide the results of bisulphite sequencing as carried out in Example 2. Each individual matrix represents the sequencing data for an individual amplificate. Each of the discrete blocks of the matrix represent a single sample type and are labeled 'A' through 'L', said letters representing in each case the following tissue/cell types: A: Melanocytes; B: Heart Muscle: C: Skeletal muscle; D: Liver; E: Sperm; F: Embryonic skeletal muscle; G: Embryonic liver; H: Placental; I: Fibroblast; J: Keratinocytes; K: CD8; L: CD4. The SEQ ID NO: of the genomic region of each amplificate is shown to the left of the matrices. This may be cross referenced in Table 4 to determine the amplificate and primer sequences. Each row of a matrix represents a single CpG site within the amplificate (according to the corresponding SEQ ID NO: from Table 4) and is numbered accordingly, each column represents a single pooled DNA sample. The degree of methylation is represented by the shade of each position within the column from black representing 100% methylation to light gray representing 0% methylation. White positions represented a measurement for which no data was available.

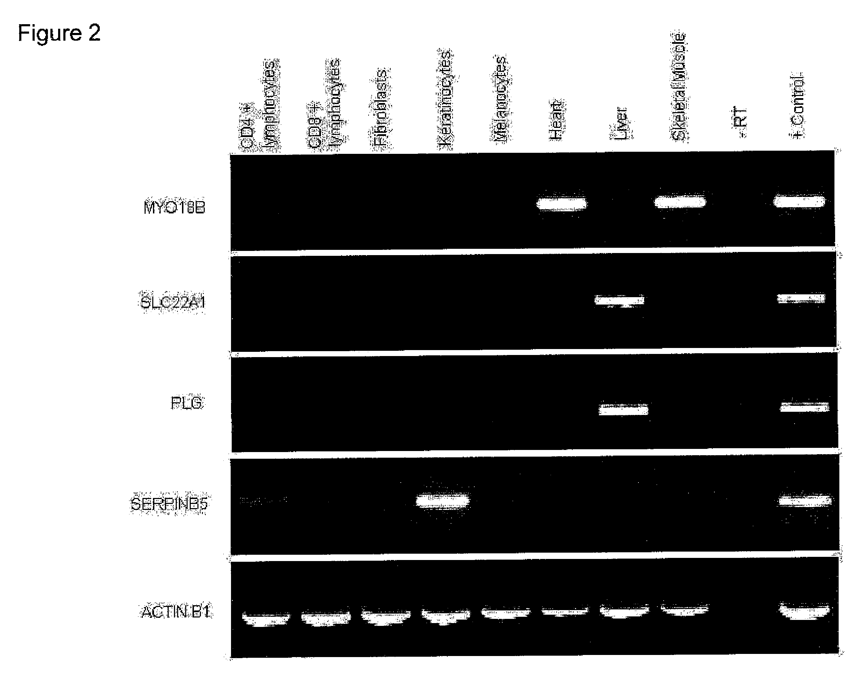

Figure 2: Example of RT-PCR results. Studied samples are arranged in columns and genes in rows. High DNA methylation correlates with gene silencing in MYO18 SLC22A1 and PLG. SERPINB5 expression has been previously reported as silenced by DNA methylation (Futscher et al, 2002). ACTIN B1 is a housekeeping gene and its expression in all analyzed samples confirms the feasibility of the assay. + Control is the amplification of cDNA produced from a pool of total RNAs from several tissues. - RT is a negative control, where no reverse transcriptase was added to the cDNA synthesis reaction.

### DETAILED DESCRIPTION OF THE INVENTION

### Definitions:

As used herein the term "classification" shall be taken to mean the action or process of categorising an object according to pre-determined parameters. Said categorization may be performed by humans means or by means of a computer or computer implemented means. It is particularly preferred that said parameters are phenotypic parameters, accordingly it is particularly preferred that said categorization is the assignment of a biological sample to a particular phenotypic class. In one embodiment of the method said phenotypic parameter or class is selected from the group consisting cell type, organ type, tissue type and disease status.

As used herein the term "expression" shall be taken to mean the transcription and translation of a gene. The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.

Furthermore the activity of the transcribed gene may be affected by genetic variations such as but not limited genetic mutations (including but not limited to SNPs, point mutations, deletions, insertions, repeat length, rearrangements and other polymorphisms).

The term "Observed/Expected Ratio" ("O/E Ratio") refers to the frequency of CpG dinucleotides within a particular DNA sequence, and corresponds to the [number of CpG sites / (number of C bases x number of G bases)].

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "methylation level" or "level of methylation" refers to the degree of 5-methylcytosine ("5-mCyt") at one or a plurality of CpG dinucleotides within a DNA sequence wherein one ore more DNA molecules are considered.

The term "hemi-methylation" or "hemimethylation" refers to the methylation state of a palindromic CpG methylation site, where only a single cytosine in one of the two CpG dinucleotide sequences of the palindromic CpG methylation site is methylated (*e.g.,* 5'-CC^{M}GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity).The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1 % SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The term polypeptide as used hereunder shall be taken to encompass all peptides, proteins and/or fragments thereof.

### Overview:

The present invention provides for molecular genetic markers that have novel utility for the classification of biological samples. In particular embodiments classification is according to at least one parameter selected from the group consisting cell type, organ type, tissue type and disease status. It is particularly preferred that the method and nucleic acids according to the invention are utilized for at least one of histological analysis, pathological analysis, detection and/or characterization of cell proliferative disorders and monitoring of cellular or tissue differentiation.

In a particularly preferred embodiment the invention provides a method for the classification of biological samples, comprising the following steps:
i) determining the expression levels of one or more genes or gene sequences according to Table 1 and/or regulatory regions thereof; and
ii) classifying said biological sample according to said expression status. Said expression level may be determined by any means standard in the art including but not limited to methylation analysis, loss of heterozygosity (hereinafter also referred to as LOH), RNA expression levels and protein expression levels.

Accordingly, said method may be enabled by means of any analysis of the expression of a RNA transcribed therefrom or polypeptide or protein translated from said RNA, preferably by means of mRNA expression analysis or polypeptide expression analysis. Accordingly the present invention also provides classification assays and methods, both quantitative and qualitative for detecting the expression of the genes, genomic sequences and/or regulatory regions according to Table 1 and providing therefrom a classification of said biological sample.

Expression of mRNA transcribed from the genes or genomic regions according to Table 1, are associated with specific organ and cell types.

To detect the presence of mRNA encoding a gene or genomic sequence, a sample is obtained from a patient. The sample may be any suitable sample comprising cellular matter of the tumor, most preferably the primary tumor. Suitable sample types include cells or cell lines, histological slides, biopsies, paraffin-embedded tissue, bodily fluids, sputum, stool, nipple aspirate, cerebrospinal fluid, ejaculate, urine, blood or any other suitable biological sample and all possible combinations thereof.

The sample may be treated to extract the RNA contained therein. The resulting nucleic acid from the sample is then analyzed. Many techniques are known in the state of the art for determining absolute and relative levels of gene expression, commonly used techniques suitable for use in the present invention include in situ hybridization (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridization probes (e.g. TaqMan, Lightcycler, Molecular Beacons & Scorpion) or SYBR green. The detected signal from the probes or SYBR green is then quantified either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labeled probe in the dried gel itself or on a membrane. The resulting signal is proportional to the amount of target RNA in the RNA population.

Comparing the signals from two or more cell populations or tissues reveals relative differences in gene expression levels. Absolute quantification can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labeled (e.g. radioactive labels, mass labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used.

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantification of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labeled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression.

DNA arrays can be generated by immobilizing presynthesized oligomers onto prepared glass slides. In this case, representative gene sequences are manufactured and prepared using standard oligomer synthesis and purification methods. These synthesized gene sequences are complementary to the genes of interest and tend to be shorter sequences in the range of 25-70 nucleotides. Alternatively, immobilized oligomers can be chemically synthesized in-situ on the surface of the slide. In situ oligomer synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labeled cDNA via a reverse transcription reaction. Fluorescent labeling of the cDNA can be accomplished by either direct labeling or indirect labeling methods. During direct labeling, fluorescently modified nucleotides (e.g., Cy^{®}3- or Cy^{®}5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabeled, but may be detectable by specific binding with a ligand which is labeled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labeled with Cy^{®}3. The resulting labeled cDNA is purified to remove unincorporated nucleotides, free dye and residual RNA. Following purification, the labeled cDNA samples are hybridized to the microarray. The stringency of hybridization is determined by a number of factors during hybridization and during the washing procedure, including temperature, ionic strength, length of time and concentration of fromamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression for that gene; bright spots correspond to strongly expressed genes, while dim spots indicate weak expression..

Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides methods for the detection of the presence of the polypeptide encoded by said gene sequences in a sample obtained from a patient.

Levels of polypeptide expression of the polypeptides encoded by the genes and/or genomic regions according to Table 1 are associated with the classification of biological samples.

Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labeled polypeptide or derivative thereof.

Certain embodiments of the present invention comprise the use of antibodies specific to the polypeptide encoded by the genes and/or genomic regions according to Table 1 .

Such antibodies are useful for the classification of biological samples. In certain embodiments production of monoclonal or polyclonal antibodies can be induced by the use of the coded polypeptide as an antigene. Such antibodies may in turn be used to detect expressed polypeptides as classification markers for biological samples. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabeled, for example as used in agglutination tests, or labeled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a generic protein (e.g. milk protein) to bind remaining sticky places on the membrane. An antibody specific to the protein of interest is then added, said antibody being detectably labeled for example by dyes or enzymatic means (e.g. alkaline phosphatase or horseradish peroxidase) . The location of the antibody on the membrane is then detected.

In an alternative embodiment of the method the proteins may be detected by means of immunochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to colored deposits at the antibody-antigen binding sites. There is a wide range of suitable sample types, antigen-antibody affinity, antibody types, and detection enhancement methods. Thus optimal conditions for immunochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In the final step of the method the biological sample is classified, as specified below.

Another aspect of the invention provides a kit for use in classifying a biological sample, comprising: a means for detecting polypeptides of a gene or genomic region according to Table 1. The means for detecting the polypeptides comprise preferably antibodies, antibody derivatives, or antibody fragments. The polypeptides are most preferably detected by means of Western blotting utilizing a labeled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit for use in classifying biological samples, comprises: (a) a means for detecting polypeptides of a gene or genomic region according to Table 1; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Another aspect of the invention relates to a kit for use in classifying a biological sample, said kit comprising: a means for measuring the level of transcription of a gene or genomic region according to Table 1 . In a preferred embodiment the means for measuring the level of transcription comprise oligomers or polynucleotides able to hybridize under stringent or moderately stringent conditions to the transcription products of a gene or genomic region according to Table 1 . In a most preferred embodiment the level of transcription is determined by techniques selected from the group of Northern blot analysis, reverse transcriptase PCR, real-time PCR, RNAse protection, and microarray. In another embodiment of the invention the kit further comprises means for obtaining a biological sample of the patient. Preferred is a kit, which further comprises a container suitable for containing the means for measuring the level of transcription and the biological sample of the patient, and most preferably further comprises instructions for use and interpretation of the kit results.

In a preferred embodiment the kit for use in classifying biological samples comprises (a) a plurality of oligomers or polynucleotides able to hybridize under stringent or moderately stringent conditions to the transcription products of a gene or genomic region according to Table 1; (b) a container suitable for containing the oligomers or polynucleotides and a biological sample of the patient comprising the transcription products wherein the oligomers or polynucleotide can hybridize under stringent or moderately stringent conditions to the transcription products, (c) means to detect the hybridization of (b); and optionally, (d) instructions for use and interpretation of the kit results.

The kit may also contain other components such as hybridization buffer (where the oligomers are to be used as a probe) packaged in a separate container. Alternatively, where the oligomers are to be used to amplify a target region, the kit may contain, packaged in separate containers, a polymerase and a reaction buffer optimized for primer extension mediated by the polymerase, such as PCR.

Most preferably a kit according to the embodiments of the present invention is used for the determination of expression step of the methods according to other aspects of the invention.

In a particularly preferred embodiment the expression level of the genes, genomic sequences and/or regulatory regions according to Table 1 is determined by analysis of the level of methylation of said genes, genomic sequences and/or regulatory regions thereof. It is preferred that the level of methylation of said genes, genomic sequences and/or regulatory regions thereof is determined by determining the methylation status or level of at least one CpG dinucleotide thereof. It is further preferred that the level of methylation of said genes, genomic sequences and/or regulatory regions thereof is determined by determining the methylation status or level of a plurality of CpG dinucleotides thereof. It is further preferred that the methylation state of CpG positions within regions of the sequences of Table 1, as shown in Table 3 are analyzed. Said analysis comprises the following steps:
i) contacting genomic DNA obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) classifying the biological sample according to the methylation status of said target regions analyzed in i).

Genomic DNA may be isolated by any means standard in the art, including the use of commercially available kits. Briefly, wherein the DNA of interest is encapsulated in by a cellular membrane the biological sample must be disrupted and lyzed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analyzed in order to determine the methylation state of one or more target gene sequences (prior to the treatment) suitable for the classification of biological samples. It is preferred that the target region comprises, or hybridizes under stringent conditions to at least 16 contiguous nucleotides of the converted sequence of at least one gene or genomic sequence selected from the group consisting the genes and genomic sequences as listed in Table 1, and more preferably to the sub-regions thereof according to Table 3. It is preferred that at least one target region is selected from each of Tables 8A to 8L. It is further preferred that the sequences of said genes as described in the accompanying sequence listing are analyzed. The method of analysis may be selected from those known in the art, including those listed herein. Particularly preferred are MethyLight^{™}, MSP^{™} and the use of blocking oligonucleotides as will be described herein. It is preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more converted sequences selected from Table 2 and sequences complementary thereto.

### FURTHERIMPROVEMENTS

The present invention provides novel uses for the genes, genomic sequences and/or regulatory regions thereof according to Table 1. Additional embodiments (see Table 2) provide modified variants of SEQ ID NO: 413 TO SEQ ID NO: 824, as well as oligonucleotides and/or PNA-oligomers for analysis of cytosine methylation patterns within the group consisting SEQ ID NO: 413 TO SEQ ID NO: 824.

An objective of the invention comprises analysis of the methylation state of one or more CpG dinucleotides within at least one of the genomic sequences selected from the group consisting of SEQ ID NO: 413 TO SEQ ID NO: 824 and sequences complementary thereto.

The disclosed invention provides treated nucleic acids, derived from genomic SEQ ID NO: 413 TO SEQ ID NO: 824, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the converted sequences of Table 2. It is particularly preferred that said nucleic acid is a non-naturally occurring modified nucleic acid that is not identical to or complementary to the genomic sequences of Table 1 or other human genomic DNA.

It is further preferred that said sequence comprises at least one CpG, TpA or CpA dinucleotide and sequences complementary thereto. The sequences of SEQ ID NO: 1650 TO SEQ ID NO: 4120 provide non-naturally occurring modified versions of the nucleic acid according to SEQ ID NO: 413 TO SEQ ID NO: 824, wherein the modification of each genomic sequence results in the synthesis of a nucleic acid having a sequence that is unique and distinct from said genomic sequence as follows. For each sense strand genomic DNA, *e.g*., SEQ ID NO:1, four converted versions are disclosed. A first version wherein "C" is converted to "T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (*i.e*. *antisense* strand), wherein "C" is converted to "T," but "CpG" remains "CpG" (*i.e*., corresponds to case where, for all "C" residues of CpG dinucleotide sequences are methylated and are thus not converted). The `upmethylated' converted sequences of SEQ ID NO: 413 TO SEQ ID NO: 824 correspond to SEQ ID NO: 1650 TO SEQ ID NO: 2472. A third chemically converted version of each genomic sequences is provided, wherein "C" is converted to "T" for all "C" residues, including those of "CpG" dinucleotide sequences (*i.e*., corresponds to case where, for the genomic sequences, all "C" residues of CpG dinucleotide sequences are unmethylated); a final chemically converted version of each sequence, discloses the complement of the disclosed genomic DNA sequence (*i.e*. *anti*sense strand), wherein "C" is converted to "T" for all "C" residues, including those of "CpG" dinucleotide sequences (*i.e*., corresponds to case where, for the complement (*anti*sense strand) of each genomic sequence, all "C" residues of CpG dinucleotide sequences are unmethylated). The 'downmethylated' converted sequences of SEQ ID NO: 413 TO SEQ ID NO: 824 correspond to SEQ ID NO: 3297 TO SEQ ID NO: 4120.

It is particularly preferred that, fragments of the converted DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO: 1650 TO SEQ ID NO: 4120 and sequences complementary thereto.

In an alternate embodiment of the method, the methylation status of preselected CpG positions within the nucleic acid sequences comprising one or more of SEQ ID NO: 413 TO SEQ ID NO: 824 may be detected by use of methylation-specific primer oligonucleotides. This technique (MSP) has been described in United States Patent No. 6,265,171 to Herman. The use of methylation status specific primers for the amplification of bisulfite converted DNA allows the differentiation between methylated and unmethylated nucleic acids. MSP primers pairs contain at least one primer which hybridizes to a bisulfite converted CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a converted nucleic acid sequence according to one of SEQ ID NO: 1650 TO SEQ ID NO: 4120 and sequences complementary thereto, wherein the base sequence of said oligomers comprises at least one CpG dinucleotide.

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite converted nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite converted nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g*., with excess blocker), degradation of the blocker oligonucleotide will be substantially precluded. This is because the polymerase will not extend the primer toward, and through (in the 5'-3' direction) the blocker—a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a converted nucleic acid sequence according to one of SEQ ID NO: 1650 TO SEQ ID NO: 4120 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the from of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, *e.g.*, matrix assisted laser desorption/ionization mass spectrometry (MALDI) or using electron spray mass spectrometry (ESI).

Matrix Assisted Laser Desorption/lonization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionately with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *next step* of the method, the amplificates obtained are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised are subsequently hybridized to an array or a set of oligonucleotides and/or PNA oligomers. In this context, the hybridization takes place in the following manner: the set of probes used during the hybridization is preferably composed of at least 2 oligonucleotides or PNA-oligomers; in the process, the amplificates serve as probes which hybridize to oligonucleotides or PNA oligomers previously bonded to a solid phase; the non-hybridized fragments are subsequently removed; said oligonucleotides or PNA oligomers contain at least one base sequence having a length of at least 9 nucleotides which is reverse complementary or identical to a segment of a sequence selected from SEQ ID NO: 1650 TO SEQ ID NO: 4120; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO: 413 TO SEQ ID NO: 4120. It is preferred that at least one oligonucleotide is used to determine the status of at least one CpG dinucleotide of a gene selected from each of Tables 8A to 8L. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In a particularly preferred embodiment of the method, the genomic methylation status of the CpG positions may be ascertained by means of oligonucleotide probes that are hybridized to the bisulfite converted DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (*e.g*., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite converted DNA, and moreover for methylation analysis within CpG dinucleotides.

A further suitable method for the use of probe oligonucleotides for the assessment of methylation by analysis of bisulfite converted nucleic acids In a further preferred, the method comprises the use of template-directed oligonucleotide extension, such as MS-SNuPE as described by Gonzalgo & Jones, Nucleic Acids Res. 25:2529-2531, 1997.

In yet a further embodiment of the method, said step comprises sequencing and subsequent sequence analysis of the amplificate (Sanger F., et al., Proc Natl Acad Sci USA 74:5463-5467, 1977).

In a preferred embodiment, the methylation analysis comprises the use of an oligonucleotide or oligomer for detecting the cytosine methylation state within genomic or treated (chemically modified) DNA, according to SEQ ID NO: 413 TO SEQ ID NO: 4120 . Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO: 1650 TO SEQ ID NO: 4120 and/or sequences complementary thereto, or to a genomic sequence according to SEQ ID NO: 413 TO SEQ ID NO: 824 and/or sequences complementary thereto.

Thus, the present invention includes nucleic acid molecules (*e.g*., oligonucleotides and peptide nucleic acid (PNA) molecules (PNA-oligomers)) that hybridize under moderately stringent and/or stringent hybridization conditions to all or a portion of the sequences SEQ ID NO: 413 TO SEQ ID NO: 4120 , or to the complements thereof. Particularly preferred is a nucleic acid molecule that hybridizes under moderately stringent and/or stringent hybridization conditions to all or a portion of the sequences SEQ ID NO: 1650 TO SEQ ID NO: 4120 but is not identical to or complementary to the equivalent genomic DNA selected from SEQ ID NO: 413 TO SEQ ID NO: 4120 or other human genomic DNA. The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 413 TO SEQ ID NO: 4120, or to the complements thereof.

Hybridizing nucleic acids of the type described herein can be used, for example, as a primer (*e.g*., a PCR primer), or a probe. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 413 TO SEQ ID NO: 4120 (such as allelic variants and SNPs), rather than identical, it is useful to first establish the lowest temperature at which only homologous hybridization occurs with a particular concentration of salt (*e.g.*, SSC or SSPE). Then, assuming that 1 % mismatching results in a 1 °C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1 % mismatch.

Examples of inventive oligonucleotides of length X (in nucleotides), as indicated by polynucleotide positions with reference to, *e.g*., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1, 2, 3,...(Y-(X-1));
where Y equals the length (nucleotides or base pairs) of SEQ ID NO: 1 (444);
where X equals the common length (in nucleotides) of each oligonucleotide in the set (*e.g*., X=20 for a set of consecutively overlapping 20-mers); and
where the number (Z) of consecutively overlapping oligomers of length X for a given SEQ ID NO of length Y is equal to Y-(X-1 For example Z= 444-19= 425 for either sense or antisense sets of SEQ ID NO:1, where X=20.

Preferably, the set is limited to those oligomers that comprise at least one CpG, TpG or CpA dinucleotide.

Examples of inventive 20-mer oligonucleotides include the following set of oligomers (and the antisense set complementary thereto), indicated by polynucleotide positions with reference to SEQ ID NO:1:
1-20, 2-21, 3-22, 4-23, 5-24, ......425 -444.

Preferably, the set is limited to those oligomers that comprise at least one CpG, TpG or CpA dinucleotide.

Likewise, examples of inventive 25-mer oligonucleotides include the following set of oligomers (and the antisense set complementary thereto), indicated by polynucleotide positions with reference to SEQ ID NO:1:
1-25, 2-26, 3-27, 4-28, 5-29, ...... 420-444.

Preferably, the set is limited to those oligomers that comprise at least one CpG, TpG or CpA dinucleotide.

The present invention encompasses, for each of SEQ ID NO: 413 TO SEQ ID NO: 4120 (sense and antisense), multiple consecutively overlapping sets of oligonucleotides or modified oligonucleotides of length X, where, *e.g*., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO: 413 TO SEQ ID NO: 824. Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the from of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g.,* a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of at least one of the CpG dinucleotides of genomic sequences SEQ ID NO: 413 TO SEQ ID NO: 824 and sequences complementary thereto, or to the corresponding CpG, TpG or CpA dinucleotide within a sequence of the treated nucleic acids according to SEQ ID NO: 1650 TO SEQ ID NO: 4120 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 1650 TO SEQ ID NO: 4120), or in genomic DNA (SEQ ID NO: 413 TO SEQ ID NO: 824) and sequences complementary thereto. These probes enable the classification of biological samples. The set of oligomers may also be used for detecting single nucleotide polymorphisms (SNPs) in treated genomic DNA (SEQ ID NO: 1650 TO SEQ ID NO: 4120), or in genomic DNA (SEQ ID NO: 413 TO SEQ ID NO: 824 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO: 413 TO SEQ ID NO: 4120 and sequences complementary thereto, or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the from of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the from of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

It is particularly preferred that the oligomers according to the invention are utilised for at least one of: in determining the presence or absence of specific organ, tissue or cell types, the detection and/or classification of a cell proliferative disorder and/or analysis of cellular differentiation.

In one embodiment of the method, this is achieved by analysis of the methylation status of at least one target sequence comprising, or hybridizing under stringent conditions to at least 16 contiguous nucleotides of a gene or sequence selected from the group consisting the genes and sequences according to Table 1 and complements thereof.

### Best mode

In a particularly preferred embodiment of the invention, the expression at least one of the genes, genomic sequences and/or regulatory regions thereof from each of Tables 8A to 8L is used in the classification of said sample. It is particularly preferred that said biological sample is classified according to at least one parameter selected from the group consisting of the cell, organ or tissue type of said biological sample or features thereof such as disease state.

Accordingly, in a particularly preferred embodiment the invention provides a method for the classification of biological samples, comprising the following steps:
i) determining the expression levels of one or more genes or gene sequences of each of Tables 8A to 8L and/or regulatory regions thereof; and
ii) classifying said biological sample according to said expression status.

In a the most preferred embodiment thereof, the present invention provides a method for classifying a biological sample, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of at least one gene or genomic sequence selected from each of Tables 8A to 8L said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the equivalent converted sequence selected from Tables 8A to 8L, and contiguous regions thereof corresponding to the target sequence.

In the final step of the method the classification of the biological sample according to the measured expression is determined. Table 6 and 7 provide information for the correlation of measured methylation with cell, tissue and/or organ types. The person skilled in the art will be able to interpret RNA and/or protein expression on this basis. It is generally appreciated that there is an inverse correlation between methylation and mRNA and/or polypeptide expression.

A person with ordinary skills in the art will be able to utilize the information provided in Tables 6 to 9 in order to select suitable markers according to his specific requirements. The information provided in said tables enables the selection of one or a plurality of genes or genomic sequences in order to enable the classification of biological samples.

The information provided in Tables 6 to 9 will also enable a person with ordinary skills in the art to combine suitable markers in order to classify or identify of biological samples. This can be done for example, but not limited to, by combing at least two genes or genomics regions which have a complementary and/or overlapping expression pattern. In one embodiment of the invention the information provided by Table 6 and 7 is used to for the classification of one or more biological samples. In a particularly preferred embodiment the information of Table 6 is used for the classification of a biological sample according to cell, tissue and/or organ type. In an alternative embodiment the information according to Table 7 is used for the confirmation and/or monitoring of cell, tissue and/or organ type which were derived by means of cell culturing or tissue engineering processes.

### Kits

The described invention further provides a composition of matter useful for the classification of biological samples. Said composition comprising at least one nucleic acid 18 base pairs in length of a segment of a nucleic acid sequence selected from the group consisting SEQ ID NO: 1650 TO SEQ ID NO: 4120, and one or more substances taken from the group comprising : magnesium chloride, dNTP, Taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of SEQ ID NO: 1650 TO SEQ ID NO: 4120 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

### Utility

The subject matter of the invention has specific utility in the fields of medicine and/or molecular biology. In particular aspects, the subject matter are one or more markers or comprises at least parts of one or more markers. Thereby a marker is a gene, a genomic sequence, a regulatory region of a gene according to Table 1 or its mRNA, cDNA or polypeptide (protein, peptide). herein also referred as molecular biological marker. According to the invention, the provided markers have novel utility for the classification of biological samples.

A utility of the present invention is to provide molecular markers and methods for the analysis thereof that may be considered an alternative to traditional histological or pathological analysis. Said molecular biological markers accordingly offer an alternative to current means such as staining and microscopic analysis.

The present invention, in particular said markers are of use in determining the presence or absence of specific organ, tissue or cell types in a biological sample. Wherein said sample is heterogeneous in nature, the method according to the present invention may be used for the identification of a population or subpopulation of specific organs, tissue or cell types. One application of this is for the determination of the tumor content of a biopsy sample which may heterogeneously comprise both tumor and normal tissue. The determination of the relative tumor content of sample is of particular interest when quantifying the presence of molecular markers by reference to the tumor content of the sample, as for example described in EP 05090318 (which is hereby incorporated by reference in its entirety).

The present invention, in particular said markers have further utility in the detection and/or classification of a cell proliferative disorder, for example but not limited to cancer. It is known in the art that increased levels of circulating cells or cellular matter are a characteristic of cell proliferative disorders. The methods or markers according to the present invention enable the detection and identification of atypical levels of cells or cellular matter derived from specific organ, tissue or cell types and thereby enable the determination of the primary location of said proliferative disorder. They also enable the detection and identification of atypical levels of expression which is a sign of dedifferentiation and breakdown of the cellular regulation mechanisms. Furthermore wherein the presence of a proliferative disorder has already been detected the primary location thereof may be determined according to the methods of the present invention.

The methods or markers of the present invention have a further alternative utility in the analysis of cellular differentiation, for example in the field of tissue engineering. The molecular characterization of a biological sample as opposed to traditional histological analysis enables the improved monitoring of differentiating cell or tissue cultures. The invention solves this longstanding need in the art by providing markers i.e. genes, genomic sequences and/or regulatory regions thereof according to Table 1 (or to one or more of those), the expression thereof at the mRNA, cDNA, protein or peptide level being indicative of the class of said biological sample. In particular, the genes, genomic sequences and/or regulatory regions thereof according to Table 8A are of use in the differentiation and/or detection of T-lymphocytes. In particular the genes, genomic sequences and/or regulatory regions thereof according to Table 8B are of use in the differentiation and/or detection of embryonic liver. In particular the genes, genomic sequences and/or regulatory regions thereof according to Table 8C are of use in the differentiation and/or detection of embryonic skeletal muscle. In particular the genes, genomic sequences and/or regulatory regions thereof according to Table 8D are of use in the differentiation and/or detection of fibroblasts. In particular the genes, genomic sequences and/or regulatory regions thereof according to Table 8E are of use in the differentiation and/or detection of heart muscle. In particular the genes, genomic sequences and/or regulatory regions thereof according to Table 8F are of use in the differentiation of heart muscle from skeletal muscle. In particular the genes, genomic sequences and/or regulatory regions thereof according to Table 8G are of use in the differentiation and/or detection of keratinocytes. In particular the genes, genomic sequences and/or regulatory regions thereof according to Table 8H are of use in the differentiation and/or detection of liver. In particular the genes, genomic sequences and/or regulatory regions thereof according to Table 81 are of use in the differentiation and/or detection of melanocytes. In particular the genes, genomic sequences and/or regulatory regions thereof according to Table 8J are of use in the differentiation and/or detection of placenta. In particular the genes, genomic sequences and/or regulatory regions thereof according to Table 8K are of use in the differentiation and/or detection of skeletal muscle. In particular the genes, genomic sequences and/or regulatory regions thereof according to Table 8L are of use in the differentiation and/or detection of sperm.

The genomic sequences (and corresponding genes) according to Table 9 are of particular use in the fields of cell culture and tissue engineering, for the confirmation and/or monitoring of particular cell, tissue and/organ types. In particular the genomic sequences according to Table 9A are suitable for the confirmation and/or monitoring of T-lymphocytes. In particular the genomic sequences according to Table 9B are suitable for the confirmation and/or monitoring of embryonic liver. In particular the genomic sequences according to Table 9C are suitable for the confirmation and/or monitoring of embryonic skeletal muscle. In particular the genomic sequences according to Table 9D are suitable for the confirmation and/or monitoring of fibroblasts. In particular the genomic sequences according to Table 9E are suitable for confirmation and/or monitoring of heart muscle. In particular the genomic sequences according to Table 9F are suitable for the confirmation and/or monitoring of keratinocytes.

In particular the genomic sequences according to Table 9G are suitable for the confirmation and/or monitoring of liver. In particular the genomic sequences according to Table 9H are suitable for the confirmation and/or monitoring of melanocytes. In particular the genomic sequences according to Table 91 are suitable for the confirmation and/or monitoring of placenta. In particular the genomic sequences according to Table 9J are suitable for the confirmation and/or monitoring of skeletal muscle.

According to a particularly preferred embodiment of the invention, the methylation status of CpG positions of genes, genomic sequences and/or regulatory regions thereof according to Table 1 is used in the classification of said sample. It is particularly preferred that said biological sample is classified according to at least one parameter selected from the group consisting of the cell, organ or tissue type of said biological sample or features thereof such as disease state. According to a particular preferred embodiment, the subject matter of the invention, in particular the methods, compositions, kits, or markers are utilised for at least one of histological analysis, pathological analysis, detection and/or characterization of cell proliferative disorders and monitoring of cellular or tissue differentiation.

According to the invention, the provided markers, in particular the genes, genomic sequences, regulatory regions, and corresponding mRNAs, cDNAs, proteins or peptides have a particular utility in the following aspects. Thereby a single marker is used either alone or in combination with other marker or markers herein provided or not.

The herein provided markers have utility (i) for the characterization of the marker corresponding tissue or cell, (ii) for the identification of marker corresponding tissue or cell, (iii) for the isolation of marker corresponding tissue or cell, (iv) for the purification of the corresponding tissue or cell, or (v) combinations thereof. Therefore known methods, so far unreported methods, or combinations thereof are useable. Said application is useful in the field of research, diagnostics as well as therapeutics.

In addition, the herein provided markers have utility for the prospective profiling, retrospective profiling, or both of donors and/or recipients in organ transplantation procedures. The correct characterization, identification, or both of the donor and/or the recipient is mandatory during organ transplantation procedures to assure the success of the intervention. The use of the markers of the invention enables the profiling of both, donor and recipient, form which prospective or retrospective observations or conclusions about the feasibility of the procedure are drawn.

In addition, the herein provided markers have utility for histological, chemical and/or immunohistochemical analysis. Accordingly, they have utility in the fields of research as well as diagnostics, in particular for histological or pathological analysis.

In addition, the herein provided markers have utility for phylogenetic profiling of species or tissues. The ontogenetic origin or the developmental lineage is then determined by comparison of the determined profiles.

In addition, the herein provided markers have utility for quality control of a genetically modified organism, tissue, group of cells or cell.

In addition, the herein provided markers have utility for controlling side effects in in vivo gene therapy procedures wherein genetically modified organism, tissue, group of cells or cell is used.

In addition, the herein provided markers have utility for the characterization, identification, or labelling of corresponding tissue or combinations thereof. This is of particular utility in the field of tissue bank storage and proliferation. Furthermore it has utility in a prospective as well as in a retrospective manner. The provided markers allow the individualization of samples by a precise molecular method. This is mandatory in storing biological material from patients or healthy individuals. In addition, this also advantageous for isolation or purification of tissues cells.

In addition, the herein provided markers have utility for controlling cell differentiation in stem-cell research and/or therapeutics. Cells undergo many genetic and/or epigenetic changes throughout differentiation. These changes influence the physiology of the cell and their control is mandatory in any procedure involving stem-cell in research and/or therapeutics. The provided markers allow to control this changes by giving a reference of the adult (completely differentiated) and embryonic (partially differentiated) status of the cells.

### CD4+ and CD8+ lymphocytes:

The herein provided markers of Table 8A and Table 9A have util ity for the quantification of lymphocytes, in particular in peripheral blood. The said markers enable the identification of CD4+ and CD8+ lymphocytes among other cells in blood samples. A low number of leucocytes in blood (leucopenia) may indicate bone marrow failure (for example, due to infection, tumor, fibrosis); presence of cytotoxic substance; collagen-vascular diseases (such as lupus erythematosus); disease of the liver or spleen; or radiation. A high number of leucocytes in blood (leucocytosis) may indicate infectious diseases; inflammatory disease (such as rheumatoid arthritis or allergy); leukemia; severe emotional or physical stress; tissue damage (for example, bums); or anemia.

In addition, the herein provided markers of Table 8A and Table 9A have utility for the study of CD4 and/or CD8 T-lymphocyte infiltration in other tissues healthy or diseased. Infiltration of lymphocytes in healthy or diseased tissues is an indication of several diseases such immunological malignances or even in tumor development. The said markers represent a target for the development of molecular probes that coupled to any detection method (e.g. Fluorescent dye) allow the identification of these cells in histological preparations.

In addition, the herein provided markers of Table 8A and Table 9A have utility for identification, isolation and/or purification of CD4 T-lymphocytes and/or CD8 T-lymphocytes, in particular from surrounding tissue infiltrated by the T-lymphocytes; from blood; and/or from other body fluids.

In addition, the herein provided markers of Table 8A and Table 9A have utility for the identification of an individual. Thereby at least two samples are used. One samples is obtained from an individual and another sample is a forensic sample, in particular traces of body cells, tissues or fluids, for example but not limited to, traces of blood and/or body fluids. This is of particular utility in the field of forensic medicine or of legal medicine. As constituent of blood or body fluids, CD4 T-lymphocytes and CD8 T-lymphocytes are part of the mentioned traces. The said markers have the advantage of being stable over time because they are DNA based. In addition said markers have the advantage that they enable a highly detailed and accurate characterization of samples. Through this an unambiguous identification of an individual is enabled.

In addition, the herein provided markers of Table 8A and Table 9A have utility for diagnosing the presence or absence of a disease. Thereby the number of CD4 T-lymphocytes, CD8 T-lymphocytes or both is quantified in normalized samples of healthy individuals. The determined number of CD4 T-lymphocytes, CD8 T-lymphocytes or both are then considered as indicative for healthy condition or a diseased condition with respect to an individual. Preferably, large amount of normalized samples are considered to generate reference values of CD4 T-lymphocytes, CD8 T-lymphocytes or both for a healthy condition and/or for one or more diseased conditions. The diseased condition can be any kind of diseased condition. Preferably, the diseased condition is a disease which causes a immune reaction. For example but not limited to the diseased condition is a cancer disease, a cell proliferation disease, or HIV. Preferably the total number of cells present in a sample is determined. The number of CD4 T-lymphocytes, CD8 T-lymphocytes or both are then normalized to the total number of cells.

### Embryonic

The herein provided markers of Table 8B, Table 8C, Table 9B and Table 9C have utility for the study, identification and/or quantification of fetal cells or fetal DNA circulating in maternal blood and /or amniotic fluid. During pregnancy cells and DNA from the fetus are continuously brought to the maternal blood stream as well as the amniotic fluid. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101).

In addition, the herein provided markers of Table 8B, Table 8C, Table 9B and Table 9C have utility for the study, identification and/or quantification of fetal cells or fetal DNA from amniocentesis and/or chorionic villus sampling. This is of particular utility in the field of prenatal diagnosis. Prenatal diagnosis procedures involve the study of fetal cells obtained by amniocentesis and chorionic villus biopsies.

### Skin

The herein provided markers of Tables 8D, G, I and Tables 9D, F, H have utility for identifying individuals from traces of skin and/ or adjacent tissues (such as hair, nail pieces, etc). This is of particular utility in forensic medicine and/or legal medicine. Skin or skin adjacent tissue is usually used as study material in forensic and legal medicine. The markers provided in Table 8G and 9F have a particular utility because of the following reason. Keratinocytes constitute the external layer of the skin and therefore are the first cell type to be de-attached and a high number of these cells is expected in skin traces. Variations of one marker alone or in combination with other markers herein provided or not enable the accurate assessment of identity.

In addition, the herein provided markers of Tables 8D, G, I and Tables 9D, F, H have utility for characterizing the skin, hair, nail, or adjacent tissue of an individual.

In addition, the herein provided markers of Tables 8D, G, I and Tables 9D, F, H have utility for determining the composition of the skin, hair, nail, or adjacent tissue of an individual. Said composition being dependent from the content of at least one of the three major constituting cell types of the skin (fibroblasts, keratinocytes and melanocytes).

In addition, the herein provided markers of Tables 8D, G, I and Tables 9D, F, H have utility in the field of drugs. They have particular utility for the development of drugs as well as for the treatment with drugs. The skin, hair, nail or adjacent tissue of an individual can be characterized by means of the provided markers of Tables 8D, G, I and Tables 9D, F, H. This information can then be used to develop new drugs or to access already existing drugs with regard to skin, hair, nail etc. of an individual or to subgroups of individuals. These subgroups are for example but not limited to be characterized by a disease and/or a defined type of skin or hair, etc. The efficiency of said drugs i.e. the presence or absence of the desired effect is also characterized or monitored by means of the provided markers of Tables 8D, G, I and Tables 9D, F, H.

In addition, the herein provided markers of Tables 8D, G, I and Tables 9D, F, H have utility as prognostic and/or diagnostic markers for wound healing, in particular in the field of surgery procedures wherein the skin is affected.

### Liver

The herein provided markers of Tables 8H and Tables 9G have utility for deducing the presence of absence of an event which affects the liver. For example but not limited to it, said event is at least one select from the group comprising liver cirrhosis; liver cancer; hepatitis A; hepatitis B; hepatitis C; healthy condition, recently or longer chemical, physical or biological exposure; recently or longer exposure to a drug, or alcohol; or treatment procedures. In the case the event is adverse, said event affecting the liver leads to the death of liver cells. In the case the event is benign, said event leads to a reduction of liver cell death. The genomic DNA of dead liver cells can then be found in the body fluids in particular in the blood of a affected individual.

In addition, the herein provided markers of Tables 8H and Tables 9G have utility for deducing the sensitivity of an individual to alcohol. Alcohol consumption may change the DNA methylation status as reviewed by Poschl et al, 2004 (Poschl G, Stickel F, Wang XD, Seitz HK. Alcohol and cancer: genetic and nutritional aspects. Proc Nutr Soc. 2004 Feb;63(1 ):65-71.).

### Heart Muscle

The herein provided markers of Tables 8E, Table 8F and Tables 9E have utility for deducing the presence of absence of an event or condition affecting the heart. For example but not limited to it, said event or condition is at least one select from the group comprising heart failure; heart attack; athletic capacity; healthy condition; recently or longer chemical, physical or biological exposure; recently or longer exposure to a drug; or treatment procedure. In the case the event is adverse, said event or condition affecting the heart leads to death of heart cells. In the case the event is benign, said event leads to a reduction of heart cell death. The genomic DNA of dead heart cells can then be found in the body fluids in particular in the blood of an affected individual.

### Placenta

The herein provided markers of Table 8J and Table 91 have utility for the study, monitoring, identification and/or quantification of placental cells or placental DNA circulating in maternal blood and /or amniotic fluid. In this respect, the said markers have also utility for the isolation or purification of placental cell or placental genomic DNA. Placenta constitute an extra-embryonic fetal tissue and as such, it shares many genetic characteristics with the fetal tissue. Therefore, cells from the placenta as well as DNA from placental cells can surrogate fetal cells and fetal DNA for diagnostic means. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101). During pregnancy placenta cells are de-attached and brought to the maternal blood stream as well as the amniotic fluid.

In addition, the herein provided markers of Table 8J and Table 91 have utility for the monitoring of embryonic development or the monitoring of placental development, in particular of extra-embryonic tissue or of interaction of extra-embryonic tissue with maternal placental tissue.

In addition, the herein provided markers of Table 8J and Table 91 have utility for the study, monitoring, identification and/or quantification of placental cells in regenerative medicine, in particular in the field of tissue engineering. Corresponding methods for the study, monitoring, identification and/or quantification of placental cells are applied in particular before and after storage, before and after cell differentiation, before and after cell proliferation, before and after cell culture expansion, and before and after tissue expansion as well as before and after transplantation.

### Sperm

The herein provided markers of Table 8L have utility for diagnosing a male infertility related disease. A major cause of male infertility is either a low amount of sperm cells (spermatozoa) in the ejaculate (oligospermia) or a complete lack of sperm cells (spermatozoa) in the ejaculate (azoospermia). Thus, methods for the quantification of sperm cells are widely useable in diagnosing male infertility.

In addition, the herein provided markers of Table 8L have utility as a tool to access the viability of the sperm cells.

In addition, the herein provided markers of Table 8L have utility for increasing the fertility of a male individual. As said above male fertility is often limited by the amount of sperm cells in the ejaculate. Thus, male fertility can be enhanced by enriching, isolating or purifying sperm cells.

In addition, the herein provided markers of Table 8L have utility for assisted fertilization procedures. Assisted fertilization procedures are for example but not limited to intracytoplasmic sperm injection (ICSI) or in vitro fertilization (IVF). All assisted fertilization procedures require the management of sperm cells prior to the procedure. Such management comprises at least the characterization, identification, quantification, enrichment, isolation, purification of sperm cells or combinations thereof.

In addition, the herein provided markers of Table 8L have utility in the fields of forensic and/or legal medicine. By use of the said markers it is possible to determine the presence or absence of sperm in a sample. Furthermore, it is possible to identify an individual by use of said markers.

### Skeletal Muscle

The herein provided markers of Table 8F, 8K and Table 9J have utility for characterizing the efficiency of skeletal muscle cells. This utility is of particular value in the field of sports medicine.

In addition, the herein provided markers of Table 8F, 8K and Table 9J have utility for identifying fully differentiated muscle cells in cell culture. This is of particular utility in the field of tissue engineering. Muscle cells are generate in cell culture by cultivation and differentiation of muscle cell progenitor cells. Fully differentiated skeletal muscle cells can be identified by means of the provided markers of Table 8F, 8K and Table 9J.

In addition, the herein provided markers of Table 8F, 8K and Table 9J have utility for diagnosing muscle cell associated diseases, in particular disease which are characterized by a death of muscle cells like muscular distrophy. The DNA of dead muscle cells is found in body fluids such as blood or urine. This DNA can be identified by means of the herein provided markers of Table 8F, 8K and Table 9J.

### CD8 T-lymphocytes

The herein provided markers of Table 8A specific only for CD8 T-lymphocytes have utility for quantifying CD8 T-lymphocytes, in particular for monitoring the immune system of individuals infected with HIV. The periodically determining of the number of CD8 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection.

### CD4+ lymphocytes

The herein provided markers of Table 8A specific only for CD4 T-lymphocytes have utility for quantifying CD4 T-lymphocytes, in particular for monitoring the immune system of individuals infected with HIV. The periodically determining of the number of CD4 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection.

According to a preferred embodiment of the invention, the provided markers, in particular the genes, genomic sequences, regulatory regions, and corresponding mRNAs, cDNAs, proteins or peptides are used in the following aspects. Thereby a single marker is used either alone or in combination with other marker or markers herein provided or not.

In a preferred embodiment, at least one of the provided markers is used (i) for the characterization of the marker corresponding tissue or cell, (ii) for the identification of marker corresponding tissue or cell, (iii) for the isolation of marker corresponding tissue or cell, (iv) for the purification of the corresponding tissue or cell, or (v) combinations thereof. Therefore known methods, so far unreported methods, or combinations thereof are useable. Said application is useful in the field of research, diagnostics as well as therapeutics. As an example, but not limited to it, this application is illustrated in more detail for the marker PDGFB SEQ ID NO: 508. All the herein provided markers can be applied and used in the same way like PDGFB SEQ ID NO: 508 correspondingly to their assignment to respective tissues, organs or cells according to Tables 8 A-L or Tables 9 A-J. Of course, corresponding markers can be alternatively used. Thereby a corresponding marker of PDGFB SEQ ID NO: 508 is, for example but not limited to, genomic DNA derived from or associated with PDGFB SEQ ID NO: 508; methylation specifically converted DNA derived from PDGFB SEQ ID NO: 508; mRNA, cDNA, protein, or peptide each of which derived at least in parts from PDGFB SEQ ID NO: 508. If the case may be, a person skilled in the art knows how to adjust the presented procedure. As shown in Table 6, PDGFB SEQ ID NO: 508 is a marker for adult liver because the CpG dinucleotides of PDGFB SEQ ID NO: 508 are methylated within the range of 75-100% in liver and only within the range of 0-25% in T-lymphocytes (CD4, CD8), embryonic tissue (embryonic liver, embryonic skeletal muscle), skin (melanocytes, keratinocytes, fibroblasts), heart muscle, placenta, sperm, or skeletal muscle.

Correspondingly, for example but not limited to it, a method for characterization and/or identification of a cell or tissue type of a sample comprises the following steps:
1. Providing of a sample, the sample being derived from an individual and comprising genomic DNA. Preferably, the genomic DNA is purified by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing a cell or tissue type by determining the methylation state or the methylation level of at least one CpG position within the sequence of PDGFB SEQ ID NO: 508 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMeth™ real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™}method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, *e.g*. anti-5-methylcytosine antibodies.
3. Optional, deducing the presence or absence of a cell type or tissue type within the provided sample from the presence or absence of a methylation state or methylation level for said at least one CpG.

For example but not limited to, a method for isolation and/or purification of a cell or group of cells comprises the following steps:
1. Providing of a sample, the sample being derived from an individual and comprising one or more cells.
2. Binding of at least one probe to one or more CpG positions within the sequence of PDGFB SEQ ID NO: 508 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells.
3. Isolating and/or purifying a cell or group of cells from the provided sample by means of the attached probes and their corresponding tags, respectively. A person skilled in the art is aware of suitable methods. Said methods are based on chemical, physical or biological properties of the attached probes or corresponding tags. For example but not limited to, the isolation is performed (i) by means of affinity cromatography, wherein the probe is attached to a tag that is recognized by an antibody immobilized on a column; (ii) by means of magnetic beads, wherein a magnetic bead is directly or indirectly bound to an attached probe and wherein a magnetic field is applied; or (iii) by means of fluorescent activated cell sorting, wherein the used tag is a fluorescent dye.

In a preferred embodiment, at least one of the provided markers is used for the prospective profiling, retrospective profiling, or both of donors and/or recipients in organ transplantation procedures. The correct characterization, identification, or both of the donor and/or the recipient is mandatory during organ transplantation procedures to assure the success of the intervention. The use of the markers of the invention enables the profiling of both, donor and recipient, form which prospective or retrospective observations or conclusions about the feasibility of the procedure are drawn. As an example, but not limited to it, this application is illustrated in more detail for the marker FOXC1 SEQ ID NO: 579. All other markers of Table 8E, Table 8F and Table 9F are applied and used like FOXC1 SEQ ID NO: 579 for heart muscle. The other herein provided markers are applied and used according their assignment to the Tables 8 A-L or Tables 9 A-J. Of course, corresponding markers can also be alternatively used. Thereby corresponding markers of FOXC1 SEQ ID NO: 579 are, for example but not limited to, genomic DNA derived from or associated with FOXC1 SEQ ID NO: 579; methylation specifically converted DNA derived from FOXC1 SEQ ID NO: 579; mRNA, cDNA, protein, or peptide each of which derived at least in parts from FOXC1 SEQ ID NO: 579. If the case may be, a person skilled in the art knows how to adjust the presented procedures. As shown in Table 6, FOXC1 SEQ ID NO: 579 is a marker for heart muscle because the CpG dinucleotides of FOXC1 SEQ ID NO: 579 are methylated within the range of 25-75% in heart muscle and only within the range of 0-25% in T-lymphocytes (CD4, CD8), embryonic tissue (embryonic liver, embryonic skeletal muscle), skin (melanocytes, keratinocytes, fibroblasts), liver, placenta, sperm, or skeletal muscle.

Correspondingly, for example but not limited to it, a method for the prospective profiling, retrospective profiling, or both of a donor and/or recipient in organ or tissue transplantation procedures comprises the following steps:
1. Providing of at least one sample, at least one sample being derived from a donor individual and/or at least one sample being derived from a recipient individual. Genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each of the at least one sample by determining the methylation state or the methylation level of at least one CpG position within the sequence of FOXC1 SEQ ID NO: 579 of the provided samples. Thereby a profile is generated comprising the methylation information of all characterized CpG positions of FOXC1 SEQ ID NO: 579. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™}method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, *e.g.* anti-5-methylcytosine anti-bodies.
3. Comparing the determined profiles, in particular the methylation state or methylation level of the analyzed at least one CpG position of the donor sample(s) and/or the recipient sample(s). Thereby the profiling is prospective or retrospective depending from the point in time the samples were collected i.e. before or after the transplantation procedure. A person skilled in the art knows then how to interpret the profiling to give an estimate on the probability of success of the intervention.

In a preferred embodiment, at least one of the provided markers is detected in studies by histological, chemical and/or immunohistochemical means. Said embodiment is useful in the fields of research as well as diagnostics, in particular for histological or pathological analysis. According to the said embodiment, the detection occurs by one or more probes that specifically bind to an epitop, peptide, protein, cDNA, mRNA, and/or at least one methylation state or level of at least one of the provided markers according to Tables 8 A-L or Table 9 A-J. Thereby a probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative. In addition, the probe is labeled directly or indirectly with a dye, protein, enzyme, metal, bead or chemical compound suitable for detection. As an example, but not limited to it, this embodiment is illustrated in more detail for the marker CMAH SEQ ID NO: 570. All other markers of Table 8G and Table 9F are applied and used like CMAH SEQ ID NO: 570 for keratinocytes. The other herein provided markers are applied and used according their assignment to the Tables 8 A-L or Tables 9 A-J. Of course, corresponding markers can also be alternatively used. Thereby corresponding markers of CMAH SEQ ID NO: 570 are, for example but not limited to, genomic DNA derived from or associated with CMAH SEQ ID NO: 570; methylation specifically converted DNA derived from CMAH SEQ ID NO: 570; mRNA, cDNA, protein, or peptide each of which derived at least in parts from CMAH SEQ ID NO: 570. If the case may be, a person skilled in the art knows how to adjust the presented procedure. As shown in Table 6, CMAH SEQ ID NO: 570 is a marker for keratinocytes and sperm because the CpG dinucleotides of CMAH SEQ ID NO: 570 are methylated only within the range of 0-25% in keratinocytes and sperm and within the range of 75-100% in T-lymphocytes (CD4, CD8), embryonic tissue (embryonic liver, embryonic skeletal muscle), melanocytes, fibroblasts, heart muscle, liver, or skeletal muscle. Keratinocytes and sperm can easily be distinguished by their different morphological appearance and physiological occurrence.

Correspondingly, for example but not limited to it, a method for histological or pathological analysis, comprises
1. Providing of a sample, comprising genomic DNA;
2. Contacting the genomic DNA or a derivative of it with at least one probe which is specific for at least one differentially methylated CpG position of the marker CMAH SEQ ID NO: 570. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art is aware of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen).

In a preferred embodiment, at least one of the provided markers is applied to phylogenetic profiling of species or tissues. The ontogenetic origin or the developmental lineage is then determined by comparison of the determined profiles. As an example, but not limited to it, this application is illustrated in more detail for the marker AIM1 SEQ ID NO: 538. All other markers of Table 8J and Table 91 are applied and used like AIM1 SEQ ID NO: 538 for placenta. The other herein provided markers are applied and used according their assignment to the Tables 8 A-L or Tables 9 A-J. Of course, corresponding markers can also be alternatively used. Thereby corresponding markers of AIM1 SEQ ID NO: 538 are, for example but not limited to, genomic DNA derived from or associated with AIM1 SEQ ID NO: 538; methylation specifically converted DNA derived from AIM1 SEQ ID NO: 538; mRNA, cDNA, protein, or peptide each of which derived at least in parts AIM1 SEQ ID NO: 538. If the case may be, a person skilled in the art knows how to adjust the presented procedures. As shown in Table 6, AIM1 SEQ ID NO: 538 is a marker for placenta because the CpG dinucleotides of AIM1 SEQ ID NO: 538 are methylated within the range of 25-75% in placenta and only within the range of 0-25% in T-lymphocytes (CD4, CD8), embryonic tissue (embryonic liver, embryonic skeletal muscle), skin (melanocytes, keratinocytes, fibroblasts), liver, heart muscle, sperm, or skeletal muscle.

Correspondingly, for example but not limited to it, a method for phylogenetic profiling of species or tissues, comprises
1. Providing of at least one sample, each sample comprising genomic DNA. Genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each of the at least one sample by determining the methylation state or the methylation level of at least one CpG position within the sequence of AIM1 SEQ ID NO: 538 of the provided samples. Thereby a profile is generated comprising the methylation information of all characterized CpG positions of AIM1 SEQ ID NO: 538. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine anti-bodies.

The profiles of the one or more respective samples are then compared with each other or with at least one reference profile. According to methods or algorithms known to those skilled in the art, the ontogenetic origin of a cell, group of cells, tissue or organ or the developmental lineage of a cell, group of cells, tissue or organ is determined.

In a preferred embodiment, at least one of the provided markers is applied for quality control of a genetically modified organism, tissue, group of cells or cell. As an example, but not limited to it, this embodiment is illustrated in more detail for the marker TBC1D10A SEQ ID NO: 700. All other markers of Table 8E, 8F and Table 9E are applied and used like TBC1D10A SEQ ID NO: 700 for heart muscle. The other herein provided markers are applied and used according their assignment to the Tables 8 A-L or Tables 9 A-J. Of course, corresponding markers can also be alternatively used. Thereby corresponding markers of TBC1D10A SEQ ID NO: 700 are, for example but not limited to, genomic DNA derived from or associated with TBC1D10A SEQ ID NO: 700; methylation specifically converted DNA derived from TBC1D10A SEQ ID NO: 700; mRNA, cDNA, protein, or peptide each of which derived at least in parts from TBC1D10A SEQ ID NO: 700. If the case may be, a person skilled in the art knows how to adjust the presented procedures. TBC1D10A SEQ ID NO: 700 is a marker for heart muscle because a) the CpG dinucleotides of TBC1D10A SEQ ID NO: 700 are methylated to a significantly higher extend in the heart muscle and sperm than in other tissues (see Table 6); and b) heart muscle and sperm can be easily distinguished morphologically or by means of other markers. According to Table 6 75-100% of the CpG dinucleotides of the marker TBC1D10A SEQ ID NO: 700 is methylated in heart muscle and sperm; 0-25% is methylated in T-lymphocytes (CD4, CD8), embryonic tissue (embryonic liver, embryonic skeletal muscle), skin (melanocytes, keratinocytes, fibroblasts), and placenta; and 25-75% is methylated in liver and skeletal muscle.

Correspondingly, for example but not limited to it, a method for quality control of a genetically modified organism, tissue, group of cells or cell, comprises
1. Providing of at least one sample of or derived from the genetically modified organism, tissue, group of cells or cell, each sample comprising genomic DNA. The genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each of the at least one sample by determining the methylation state or the methylation level of at least one CpG position within the sequence of TBC1D10A SEQ ID NO: 700 of the provided samples. Thereby a profile is generated comprising the methylation information of all characterized CpG positions of TBC1D10A SEQ ID NO: 700. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine anti-bodies.
3. Comparing the said profiles with each other or with reference profiles. A person skilled in the art knows to interpret correspondingly said comparison and to deduce therefrom the quality of the genetically modified organism, tissue, group of cells or cell. Thereby a person skilled in the art is enabled to draw prospectively or retrospectively conclusions on the presence or absence of side effects if said genetically modified organism, tissue, group of cells or cell is brought into contact with other organisms, tissues, groups of cells or cells.

For example but not limited to, a method for quality control of an genetically modified organism, tissue, group of cells or cell, comprises
1. Providing of at least one first sample of or derived from an organism, tissue, group of cells or cell being not genetically modified and at least one second sample of or derived from a correspondent organism, tissue, group of cells or cell being genetically modified. Thereby each sample comprises genomic DNA. The genomic DNA is purified from said first and second samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each of the at least one first and second samples by determining the methylation state or the methylation level of at least one CpG position within the sequence of TBC1D10A SEQ ID NO: 700 of the provided samples. Thereby a first and second profile is generated comprising the methylation information of all characterized CpG positions of TBC1D10A SEQ ID NO: 700 of the respective samples. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine anti-bodies.
3. Comparing the said first and second profile with each other or with reference profiles.
4. Deducing the presence or absence of side effects, wherein said side effects are characterized in that changes are introduced into regions of the genome which were not target of the genetic modification. This application is of particular interest to exclude unwanted physiological alterations of the cell, group of cells, tissue or organism. The reason for this is unwanted physiological alterations are mainly caused by genetic modification of non-target genomic DNA regions.

According to a particular preferred embodiment, the at least one first sample is derived from an organism, tissue, group of cells or cell before a genetic modification and the at least one second sample is derived thereof after said genetic modification.

In a preferred embodiment, at least one of the provided markers is applied for controlling side effects in in vivo gene therapy procedures wherein genetically modified organism, tissue, group of cells or cell is used. As an example, but not limited to it, this embodiment is illustrated in more detail for the marker GPX5 SEQ ID NO: 574. All other markers of Table 8A and Table 9A are applied and used correspondingly as GPX5 SEQ ID NO: 574 for T-lymphocytes. The other herein provided markers are applied and used according their assignment to the Tables 8 A-L or Tables 9 A-J. Of course, corresponding markers can also be alternatively used. Thereby corresponding markers of GPX5 SEQ ID NO: 574 are, for example but not limited to, genomic DNA derived from or associated with GPX5 SEQ ID NO: 574; methylation specifically converted DNA derived from GPX5 SEQ ID NO: 574; mRNA, cDNA, protein, or peptide each of which derived at least in parts from GPX5 SEQ ID NO: 574. If the case may be, a person skilled in the art knows how to adjust the presented procedures. As shown in Table 6, GPX5 SEQ ID NO: 574 is a marker for T-lymphocytes because the CpG dinucleotides of GPX5 SEQ ID NO: 574 are methylated within the range of 0-25% in CD4 T-lymphocytes as well as in CD 8 T-lymphocytes and within the range of 75-100% in embryonic tissue (embryonic liver, embryonic skeletal muscle), skin (melanocytes, keratinocytes, fibroblasts), placenta, liver, heart muscle, sperm, or skeletal muscle.

For example but not limited to, a method for controlling side effects in in vivo gene therapy procedures, comprises
1. Providing of at least one untreated sample derived from an individual and at least one treated sample of said individual. Thereby the samples are derived from respective body regions and each of the samples comprises genomic DNA. The genomic DNA is purified from said first and second samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each of the at least one first and second samples by determining the methylation state or the methylation level of at least one CpG position within the sequence of GPX5 SEQ ID NO: 574 of the provided samples. Thereby a first and second profile is generated comprising the methylation information of all characterized CpG positions of GPX5 SEQ ID NO: 574 of the respective samples. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine anti-bodies.
3. Comparing the said first and second profile with each other or with reference profiles.
4. Deducing the presence or absence of side effects, wherein said side effects are characterized in that changes are introduced into regions of the genome which were not target of the genetic modification. This application is of particular interest to exclude unwanted physiological alterations for the individual. The reason for this unwanted physiological alterations are mainly caused by genetic modification of non-target genomic DNA regions.

According to a particular preferred embodiment, the at least one untreated sample is derived before the gene therapy and the at least one treated sample is derived after said gene therapy.

In a preferred embodiment, at least one of the provided markers is applied for the characterization, identification, or labelling of corresponding tissue or combinations thereof. Said embodiment is of particular use in the field of tissue bank storage and proliferation. Furthermore it can be used in a prospective as well as in a retrospective manner. The provided markers allow the individualization of samples by a precise molecular method. This is mandatory in storing biological material from patients or healthy individuals. In addition, this also advantageous for isolation or purification of tissues cells. As an example, but not limited to it, this application is illustrated in more detail for the marker TCN2 SEQ ID NO: 470. All other markers of Table 8L are applied and used like TCN2 SEQ ID NO: 470 for sperm. The other herein provided markers are applied and used according their assignment to the Tables 8 A-L or Tables 9 A-J. Of course, corresponding markers can also be alternatively used. Thereby corresponding markers of TCN2 SEQ ID NO: 470 are, for example but not limited to, genomic DNA derived from or associated with TCN2 SEQ ID NO: 470; methylation specifically converted DNA derived from TCN2 SEQ ID NO: 470; mRNA, cDNA, protein, or peptide each of which derived at least in parts from TCN2 SEQ ID NO: 470. If the case may be, a person skilled in the art knows how to adjust the presented procedures. As shown in Table 6, TCN2 SEQ ID NO: 470 is a marker for sperm because the CpG dinucleotides of TCN2 SEQ ID NO: 470 are methylated within the range of 75-100% in sperm and to only an extend of 0-25% in T-lymphocytes (CD4, CD8), embryonic tissue (embryonic liver, embryonic skeletal muscle), skin (melanocytes, keratinocytes, fibroblasts), placenta, liver, heart muscle, or skeletal muscle.

Correspondingly, for example but not limited to it, a method for characterizing a tissue or cell, comprises
1. Providing of at least one sample comprising genomic DNA. The genomic DNA is purified from said sample(s), preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each sample by determining the methylation state or the methylation level of at least one CpG position within the sequence of TCN2 SEQ ID NO: 470 of the provided sample(s). Thereby a profile is generated comprising the methylation information of all characterized CpG positions of TCN2 SEQ ID NO: 470 of the respective sample(s). A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

For example but not limited to it, a method for labelling a tissue or cell, comprises in addition to the said method for characterizing a tissue or cell:
3. Labelling said tissue or cell by means of the methylation status or level of one or more of the analyzed CpG positions of TCN2 SEQ ID NO: 470. According to a preferred embodiment the labelling is achieved by assigning at least one distinct methylation status or level of at least one analyzed CpG dinucleotide of TCN2 SEQ ID NO: 470 to said tissue or cell. Thereby the assigned methylation status or level(s) are specific for said tissue or cell.

For example but not limited to it, a method for identifying a tissue or cell, comprises in addition to the said method for characterizing a tissue or cell:
3. Identifying a tissue or cell or an individual from whom the sample is derived from by comparison of the determined TCN2 profile of said samples with a reference TCN2 profile.

For example but not limited to it, a method for profiling a tissue type or cell type, comprises in addition to the said method for characterizing a tissue or cell:
3. Comparing the determined TCN2 profiles of said samples with each other and/or with at least one reference TCN2 profile and considering the group of same methylation status or levels of correspondent CpG dinucleotides of different samples as a tissue type or cell type profile.

A person skilled in the art knows to combine the said methods for characterizing a tissue or cell; for labelling a tissue or cell; for identifying a tissue or cell; and for profiling a tissue type or cell type with state of the art methods for tissue or cell isolation or purification.

In a preferred embodiment, at least one of the provided markers is applied for controlling cell differentiation in stem-cell research and/or therapeutics. Cells undergo many genetic and/or epigenetic changes throughout differentiation. These changes influence the physiology of the cell and their control is mandatory in any procedure involving stem-cell in research and/or therapeutics. The provided markers enable to control this changes by giving a reference of the adult (completely differentiated) and embryonic (partially differentiated) status of the cells. As an example, but not limited to it, this application is illustrated in more detail for the marker RPL3 SEQ ID NO: 529. All other markers of Table 8H and Table 9G are applied and used like RPL3 SEQ ID NO: 529 for adult liver. The other herein provided markers are applied and used according their assignment to the Tables 8 A-L or Tables 9 A-J. Of course, corresponding markers can also be alternatively used. Thereby corresponding markers of RPL3 SEQ ID NO: 529 are, for example but not limited to, genomic DNA derived from or associated with RPL3 SEQ ID NO: 529; methylation specifically converted DNA derived from RPL3 SEQ ID NO: 529; mRNA, cDNA, protein, or peptide each of which derived at least in parts from RPL3 SEQ ID NO: 529. If the case may be, a person skilled in the art knows how to adjust the presented procedures. As shown in Table 6, RPL3 SEQ ID NO: 529 is a marker for sperm because the CpG dinucleotides of RPL3 SEQ ID NO: 529 are methylated within the range of 25-75% in adult liver and within the range of 75-100% in T-lymphocytes (CD4, CD8), embryonic tissue (embryonic liver, embryonic skeletal muscle), skin (melanocytes, keratinocytes, fibroblasts), placenta, sperm, heart muscle, or skeletal muscle.

Correspondingly, for example but not limited to it, a method for controlling cell differentiation in stem- cell research and/or therapeutics, comprises
1. Providing of a sample comprising genomic DNA. The genomic DNA is purified from said sample(s), preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing said sample by determining the methylation state or the methylation level of at least one CpG position within the sequence of RPL3 SEQ ID NO: 529 of the provided sample. Thereby a profile is generated comprising the methylation information of all characterized CpG positions of RPL3 SEQ ID NO: 529 of the respective sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Comparing said RPL3 profile of said sample with at least one reference RPL3 profile and deducing therefrom if a desired differentiation status of one or more cells of the sample is met or not. Through this a controlling of cell differentiation is achieved.

According to Table 6, the herein provided markers are assigned to different tissues (see Tables 8 A-L or Tables 9 A-J). In the following, applications are described which are only applicable for the named tissue(s).

### CD4+ and CD8+ lymphocytes:

In a preferred embodiment, the herein provided markers of Table 8A and Table 9A are used for the quantification of lymphocytes, in particular in peripheral blood.

Low number of leucocytes in blood (leucopenia) may indicate:
- bone marrow failure (for example, due to infection, tumor, fibrosis)
- presence of cytotoxic substance
- collagen-vascular diseases (such as lupus erythematosus)
- disease of the liver or spleen
- radiation

High number of leucocytes in blood (leucocytosis) may indicate:
- infectious diseases
- inflammatory disease (such as rheumatoid arthritis or allergy)
- leukemia
- severe emotional or physical stress
- tissue damage (for example, burns)
- anemia

Said markers enable the identification of CD4+ and CD8+ lymphocytes among other cells in blood samples. For example but not limited to it, the differential methylation of FBLN1 SEQ ID NO: 426 is used. According to Table 6, the differential methylation of FBLN1 SEQ ID NO: 426 is marker for CD4 T-lymphocytes as well as for CD8 T-lymphocytes because the CpG dinucleotides of FBLN1 SEQ ID NO: 426 are methylated within the range of 25-75% in CD4 and CD8 T-lymphocytes while other tissues show a different extend of methylation. Of course, corresponding markers can also be alternatively used. Thereby corresponding markers of FBLN1 SEQ ID NO: 426 are, for example but not limited to, genomic DNA derived from or associated with FBLN1 SEQ ID NO: 426; methylation specifically converted DNA derived from FBLN1 SEQ ID NO: 426; mRNA, cDNA, protein, or peptide each of which derived at least in parts from FBLN1 SEQ ID NO: 426. If the case may be, a person skilled in the art knows how to adjust the presented procedures.

Correspondingly, for example but not limited to it, a method for the quantification of CD4 and/or CD8 T-lymphocytes, comprises:
1. Providing of a sample, comprising genomic DNA;
2. Contacting the genomic DNA or a derivative of it with at least one probe which is specific for at least one differentially methylated CpG position of the marker FBLN1 SEQ ID NO: 426. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art is aware of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen). A person skilled in the art knows further suitable methods for detection.
4. Quantifying the detection reaction in a manner so that the detected signal is indicative for the amount of probe or label therewith also for the amount of CD4 and/or CD8 T-lymphocytes. A person knows suitable methods for quantification.

In a particular preferred embodiment, the said method is also a method for isolation of CD4 and CD8 T-lymphocytes. Said method additional comprises the separation of CD4 and CD8 T-lymphocytes from other cells, tissue, or molecules of a sample by means of the said probes and/or labels attached to one or more differentially methylated CpG position of FBLN1 SEQ ID NO: 426 in CD4 and CD8 T-lymphocytes. A person skilled in the art knows suitable methods for separation of labeled cells form unlabeled cells, tissue, or molecules.

Alternatively, for example but not limited to, a method for quantifying the number of CD4 and/or CD8 T-lymphocytes comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of FBLN1 SEQ ID NO: 426 of the provided sample. A person skilled in the art knows how to determine the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies. 3. Quantifying the number of CD4 and/or CD8 T-lymphocytes by comparing the determined one or more methylation levels of the sample with the respective herein provided one or more methylation levels of CD4 and/or CD8 T-lymphocytes and with at least one methylation level representing the correspondent tissue, group of cells, or cell of a healthy individual.

In a preferred embodiment, the markers of Table 8A and Table 9A are used to study the CD4 and/or CD8 T-lymphocyte infiltration in other tissues healthy or diseased. Infiltration of lymphocytes in healthy or diseased tissues is an indication of several diseases such immunological malignances or even in tumor development. The said markers represent a target for the development of molecular probes that coupled to any detection method (e.g. Fluorescent dye) allow the identification of these cells in histological preparations. For example but not limited to it, the differential methylation of HLA-DPB SEQ ID NO: 416 is used. According to Table 6, the differential methylation of HLA-DPB SEQ ID NO: 416 is marker for CD4 T-lymphocytes as well as for CD8 T-lymphocytes because the CpG dinucleotides of HLA-DPB SEQ ID NO: 416 are methylated within the range of 75-100% in CD4 and CD8 T-lymphocytes while other tissues show a different extend of methylation. Of course, corresponding markers can also be alternatively used. Thereby corresponding markers of HLA-DPB SEQ ID NO: 416 are, for example but not limited to, genomic DNA derived from or associated with HLA-DPB SEQ ID NO: 416; methylation specifically converted DNA derived from HLA-DPB SEQ ID NO: 416; mRNA, cDNA, protein, or peptide each of which derived at least in parts from HLA-DPB SEQ ID NO: 416. If the case may be, a person skilled in the art knows how to adjust the presented procedures.

Correspondingly, for example but not limited to it, a method for detection of infiltrated CD4 and/or CD8 T-lymphocytes comprises:
1. Providing of a sample, comprising genomic DNA;
2. Contacting the genomic DNA or a derivative of it with at least one probe which is specific for at least one differentially methylated CpG position of the marker HLA-DPB SEQ ID NO: 416. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art knows of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen).

According to a preferred embodiment, said method is performed in a histological manner. A person skilled in the art knows how to carried such a method. For example, but not limited to, the providing of the sample comprises the making of sample sections suitable for histological analysis.

In a preferred embodiment, the markers of Table 8A and Table 9A are used to identify, isolate and/or purify CD4 T-lymphocytes and/or CD8 T-lymphocytes, in particular from surrounding tissue infiltrated by the T-lymphocytes; from blood; and/or from other body fluids. For example but not limited to it, the differential methylation of APOBEC3B SEQ ID NO: 474 is used. According to Table 6, the differential methylation of APOBEC3B SEQ ID NO: 474 is a marker for CD4 T-lymphocytes as well as for CD8 T-lymphocytes because the CpG dinucleotides of APOBEC3B SEQ ID NO: 474 are methylated within the range of 0-25% in CD4 and CD8 T-lymphocytes while other tissues show a different extend of methylation. Of course, corresponding markers can also be alternatively used. Thereby corresponding markers of APOBEC3B SEQ ID NO: 474 are, for example but not limited to, genomic DNA derived from or associated with APOBEC3B SEQ ID NO: 474; methylation specifically converted DNA derived from APOBEC3B SEQ ID NO: 474; mRNA, cDNA, protein, or peptide each of which derived at least in parts from APOBEC3B SEQ ID NO: 474. If the case may be, a person skilled in the art knows how to adjust the presented procedures.

Correspondingly, for example but not limited to, a method for identifying CD4 T-lymphocytes and/or CD8 T-lymphocytes comprises:
1. Providing of a sample, the sample comprising genomic DNA.
2. Binding of at least one probe to one or more CpG positions within the sequence of APOBEC3B SEQ ID NO: 474 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying CD4 and/or CD8 T-lymphocytes by detecting the bound probes and/or their respective label(s). A person skilled in the art knows suitable methods for detection of said probes or labels.

For example but not limited to, a method for isolating and/or purifying CD4 and/or CD8 T-lymphocytes comprises in addition to the steps of the said method for identifying CD4 T-lymphocytes and/or CD8 T-lymphocytes:
3. Isolating and/or purifying of the identified CD4 and/or CD8 T-lymphocytes from the provided sample by means of the attached probes and their corresponding tags, respectively. A person skilled in the art knows suitable methods. Said methods are based on chemical, physical or biological properties of the attached probes or corresponding tags. For example but not limited to, the isolation is performed (i) by means of affinity cromatography, wherein the probe is attached to a tag that is recognized by an antibody immobilized on a column; (ii) by means of magnetic beads, wherein a magnetic bead is directly or indirectly bound to an attached probe and wherein a magnetic field is applied; or (iii) by means of fluorescent activated cell sorting, wherein the used tag is a fluorescent dye.

According to a preferred embodiment, the isolated or purified CD4 and/or CD8 T-lymphocytes are quantified by means of the attached probes and/or their corresponding tags. A person skilled in the art knows suitable methods. For example, but not limited to by cell counting manually or by automatic means.

In a preferred embodiment, the markers of Table 8A and Table 9A are used for the identification of an individual. Thereby at least two samples are used. One samples is obtained from an individual and another sample is a forensic sample, in particular traces of body cells, tissues or fluids for example but not limited to traces of blood and/or body fluids. This embodiment is of particular use in the field of forensic medicine or of legal medicine. As constituent of blood or body fluids, CD4 T-lymphocytes and CD8 T-lymphocytes are part of the mentioned traces. The said markers have the advantage of being stable over time because they are DNA based. In addition said markers have the advantage that they enable a highly detailed and accurate characterization of samples. Through this an unambiguous identification of an individual is enabled. For example but not limited to it, the differential methylation of GPX5 SEQ ID NO: 574 is used. According to Table 6, the differential methylation of GPX5 SEQ ID NO: 574 is a marker for CD4 T-lymphocytes as well as for CD8 T-lymphocytes because the CpG dinucleotides of GPX5 SEQ ID NO: 574 are methylated within the range of 0-25% in CD4 and CD8 T-lymphocytes while other tissues show a different extend of methylation. Of course, corresponding markers can also be alternatively used. Thereby corresponding markers of GPX5 SEQ ID NO: 574 are, for example but not limited to, genomic DNA derived from or associated with GPX5 SEQ ID NO: 574; methylation specifically converted DNA derived from GPX5 SEQ ID NO: 574; mRNA, cDNA, protein, or peptide each of which derived at least in parts from GPX5 SEQ ID NO: 574. If the case may be, a person skilled in the art knows how to adjust the presented procedures.

Correspondingly, for example but not limited to it, a method for the identification of an individual, comprises
1. Providing at least two samples. One sample is collected from an individual. Another sample is a forensic sample. Each of the provided samples comprises genomic DNA. The genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing each sample by determining the methylation state or the methylation level of at least one CpG position within the sequence of GPX5 SEQ ID NO: 574 of the provided samples. Thereby a profile is generated comprising the methylation information of all characterized CpG positions GPX5 SEQ ID NO: 574 of the respective samples. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3.Comparing the GPX5 profile of the forensic sample with one or more profiles of the samples collected from individual. An individual is identified wherein the GXP5 profile matches the profile of the sample of said individual. In alternative preferred embodiments, the forensic sample and the samples collected from individuals are collected or processed not at the same time.

In a preferred embodiment, the markers of Table 8A and Table 9A are used to diagnose the presence or absence of a disease. Thereby the number of CD4 T-lymphocytes, CD8 T-lymphocytes or both is quantified in normalized samples of healthy individuals. The determined number of CD4 T-lymphocytes, CD8 T-lymphocytes or both are then considered as indicative for healthy condition or a diseased condition with respect to an individual. Preferably, large amount of normalized samples are considered to generate reference values of CD4 T-lymphocytes, CD8 T-lymphocytes or both for a healthy condition and/or for one or more diseased conditions. The diseased condition can be any kind of diseased condition. Preferably, the diseased condition is a disease which causes a immune reaction. For example but not limited to the diseased condition is a cancer disease, a cell proliferation disease, or HIV. Preferably the total number of cells present in a sample is determined. The number of CD4 T-lymphocytes, CD8 T-lymphocytes or both are then normalized to the total number of cells. For example but not limited to it, the differential methylation of SYNE1 SEQ ID NO: 558 is used. According to Table 6, the differential methylation of SYNE1 SEQ ID NO: 558 is a marker for CD4 T-lymphocytes, for CD8 T-lymphocytes as well as sperm because the CpG dinucleotides of SYNE1 SEQ ID NO: 558 are methylated within the range of 75-100% in CD4 T-lymphocytes, CD8 T-lymphocytes and sperm while other tissues show a different extend of methylation. Because sperm can be easily morphologically distinguished from CD4 or CD8 T-lymphocytes, this marker can be used in the following described application. Of course, corresponding markers can also be alternatively used. Thereby corresponding markers of SYNE1 SEQ ID NO: 558 are, for example but not limited to, genomic DNA derived from or associated with SYNE1 SEQ ID NO: 558; methylation specifically converted DNA derived from SYNE1 SEQ ID NO: 558; mRNA, cDNA, protein, or peptide each of which derived at least in parts from SYNE1 SEQ ID NO: 558. If the case may be, a person skilled in the art knows how to adjust the presented procedures.

Correspondingly, for example but not limited to it, a method for diagnosing the absence or presence of a diseased or healthy condition for an individual, comprises
1. Providing a sample, comprising genomic DNA.
2. Determining the number of CD4 T-lymphocytes, CD8 T-lymphocytes or both by detecting the methylation status or level of at least one CpG position within the sequence of SYNE1 SEQ ID NO: 558 of the provided samples. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies. 3. Normalizing the determined number of CD4 T-lymphocytes, CD8 T-lymphocytes or CD4 T-lymphocytes and CD8 T-lymphocytes. A person skilled in the art knows suitable methods for normalization. For example but not limited to the determined number of CD4 T-lymphocytes, CD8 T-lymphocytes or CD4 T-lymphocyte and CD8 T-lymphocytes is normalized to the number of total cell contained the provided sample.
1. In a preferred embodiment, step 2 is performed in a histological, immunohistological and/or immunocytological manners. A person skilled in the art knows suitable methods for carrying out step 2 in histological, immunohistological and/or immunocytological manners for example but not limited to in situ hybridization, antibody stainings, or FACS. In another preferred embodiment, step 2 is performed by means of molecular biological methods. A person skilled in the art knows also suitable methods therefor for example but not limited to real time PCR based methods or hybridization based methods (Microarrays).

### Embryonic

In a preferred embodiment, the herein provided markers of Table 8B, Table 8C, Table 9B and Table 9C are used for the study, identification and/or quantification of fetal cells or fetal DNA circulating in maternal blood and /or amniotic fluid. During pregnancy cells and DNA from the fetus are continuously brought to the maternal blood stream as well as the amniotic fluid. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101). For example but not limited to it, the differential methylation of CTA-384D8.15 SEQ ID NO: 509 is used. According to Table 6, the differential methylation of CTA-384D8.15 SEQ ID NO: 509 is a marker for embryonic liver because the CpG dinucleotides of CTA-384D8.15 SEQ ID NO: 509 are methylated within the range of 25-75% in embryonic liver while other tissues show a different extend of methylation. Of course, corresponding markers can also be alternatively used. Thereby corresponding markers of CTA-384D8.15 SEQ ID NO: 509 are, for example but not limited to, genomic DNA derived from or associated with CTA-384D8.15 SEQ ID NO: 509; methylation specifically converted DNA derived from CTA-384D8.15 SEQ ID NO: 509; mRNA, cDNA, protein, or peptide each of which derived at least in parts from CTA-384D8.15 SEQ ID NO: 509. If the case may be, a person skilled in the art knows how to adjust the presented procedures.

Correspondingly, for example but not limited to, a method for identifying fetal cells or fetal DNA comprises:
1. Providing of a sample, the sample comprising one or more cells or genomic DNA.
2. Binding of at least one probe to one or more CpG positions within the sequence of CTA-384D8.15 SEQ ID NO: 509 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying fetal cells or fetal genomic DNA by detecting the bound probes and/or their respective label(s). A person skilled in the art knows suitable methods for detection of said probes or labels.

In preferred embodiments, a probe of step 2 binds specifically with respect to the methylation status of 1, 2, 3, 4, 5, 6, 7, 8, 9, 10, 11, 12, 13, 14, 15, 16, 17, 18, 19, 20, 22, 25, or 30 CpG dinucleotides.

For example but not limited to, a method for isolating and/or purifying fetal cells or fetal genomic DNA comprises in addition to the steps of the said method for identifying fetal cells or fetal genomic DNA:
3. Isolating and/or purifying of the identified fetal cells or fetal genomic DNA from the provided sample by means of the attached probes and their corresponding tags, respectively. A person skilled in the art knows suitable methods. Said methods are based on chemical, physical or biological properties of the attached probes or corresponding tags. For example but not limited to, the isolation is performed (i) by means of affinity cromatography, wherein the probe (e.g. a nucleic acid) is directly or indirectly bound to a solid surface; (ii) by means of affinity cromatography, wherein the probe is attached to a tag that is recognized by an antibody immobilized on a column; (iii) by means of magnetic beads, wherein a magnetic bead is directly or indirectly bound to an attached probe and wherein a magnetic field is applied; or (iv) by means of fluorescent activated cell sorting, wherein the used tag is a fluorescent dye.

According to a preferred embodiment, the isolated or purified fetal cells or fetal genomic DNA are quantified by means of the attached probes and/or their corresponding tags. A person skilled in the art knows suitable methods. For example, but not limited to by cell counting manually or by automatic means. According to a preferred embodiment, the isolated or purified fetal genomic DNA is quantified by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method.

Alternatively, for example but not limited to, a method for quantifying the number of fetal cells or the amount of fetal genomic DNA comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of CTA-384D8.15 SEQ ID NO: 509 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of fetal cells or the amount of fetal genomic DNA by comparing the determined one or more methylation levels of the sample with the respective herein provided one or more methylation levels specific for embryonic liver and/or embryonic skeletal muscle and with at least one methylation level representing the correspondent tissue, group of cells, or cell comprising no placental DNA.

For example but not limited to it, a method for characterizing one or more fetal cells or fetal genomic DNA comprises
1. Providing of a sample comprising one or more fetal cells or fetal genomic DNA. The fetal cell(s) or the fetal genomic DNA are isolated for example but not limited to the methods described herein.
2. Characterizing said sample by determining the methylation state or the methylation level of at least one CpG position within the sequence of CTA-384D8.15 SEQ ID NO: 509 of the provided sample. Thereby a profile is generated comprising the methylation information of all characterized CpG positions of CTA-384D8.15 SEQ ID NO: 509 of the respective sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

In a preferred embodiment of step 1, the genomic DNA comprising fetal genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).

In a preferred embodiment the determined CTA-384D8.15 profile is compared with one or more CTA-384D8.15 profiles of obtained from other samples and/or with one or more CTA-384D8.15 reference profiles.

In a preferred embodiment, the herein provided markers of Table 8B, Table 8C, Table 9B and Table 9C are used for the study, identification and/or quantification of fetal cells or fetal DNA from amniocentesis and/or chorionic villus sampling. Said embodiment is of particular use in the field of prenatal diagnosis. Prenatal diagnosis procedures involve the study of fetal cells obtained by amniocentesis and chorionic villus biopsies. For example but not limited to it, the differential methylation of CRYBA4 SEQ ID NO: 476 is used. According to Table 6, the differential methylation of CRYBA4 SEQ ID NO: 476 is a marker for embryonic liver and embryonic skeletal muscle because the CpG dinucleotides of CRYBA4 SEQ ID NO: 476 are methylated within the range of 25-75% in embryonic liver or embryonic skeletal muscle while other tissues show a different extend of methylation. Of course, corresponding markers can also be alternatively used. Thereby corresponding markers of CRYBA4 SEQ ID NO: 476 are, for example but not limited to, genomic DNA derived from or associated with CRYBA4 SEQ ID NO: 476; methylation specifically converted DNA derived from CRYBA4 SEQ ID NO: 476; mRNA, cDNA, protein, or peptide each of which derived at least in parts from CRYBA4 SEQ ID NO: 476. If the case may be, a person skilled in the art knows how to adjust the presented procedures.

Correspondingly, for example but not limited to, a method for identifying fetal cells or fetal DNA comprises:
1. Providing of a sample, the sample comprising one or more cells or genomic DNA.
2. Binding of at least one probe to one or more CpG positions within the sequence of CRYBA4 SEQ ID NO: 476 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying fetal cells or fetal genomic DNA by detecting the bound probes and/or their respective label(s). A person skilled in the art knows suitable methods for detection of said probes or labels.

In preferred embodiments, a probe of step 2 binds specifically with respect to the methylation status of 1, 2, 3, 4, 5, 6, 7, 8, 9, 10, 11, 12, 13, 14, 15, 16, 17, 18, 19, 20, 22, 25, or 30 CpG dinucleotides.

For example but not limited to, a method for isolating and/or purifying fetal cells or fetal genomic DNA comprises in addition to the steps of the said method for identifying fetal cells or fetal genomic DNA:
3. Isolating and/or purifying of the identified fetal cells or fetal genomic DNA from the provided sample by means of the attached probes and their corresponding tags, respectively. A person skilled in the art knows suitable methods. Said methods are based on chemical, physical or biological properties of the attached probes or corresponding tags. For example but not limited to, the isolation is performed (i) by means of affinity cromatography, wherein the probe (e.g. a nucleic acid) is directly or indirectly bound to a solid surface; (ii) by means of affinity cromatography, wherein the probe is attached to a tag that is recognized by an antibody immobilized on a column; (iii) by means of magnetic beads, wherein a magnetic bead is directly or indirectly bound to an attached probe and wherein a magnetic field is applied; or (iv) by means of fluorescent activated cell sorting, wherein the used tag is a fluorescent dye.

According to a preferred embodiment, the isolated or purified fetal cells or fetal genomic DNA are quantified by means of the attached probes and/or their corresponding tags. A person skilled in the art knows suitable methods. For example, but not limited to by cell counting manually or by automatic means. According to a preferred embodiment, the isolated or purified fetal genomic DNA is quantified by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method.

Alternatively, for example but not limited to, a method for quantifying the number of fetal cells or the amount of fetal genomic DNA comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of CRYBA4 SEQ ID NO: 476 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™}method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of fetal cells or the amount of fetal genomic DNA by comparing the determined methylation levels of the sample with the respective herein provided methylation levels of embryonic liver and/or embryonic skeletal muscle.

For example but not limited to it, a method for characterizing one or more fetal cells or fetal genomic DNA comprises
1. Providing of a sample comprising one or more fetal cells or fetal genomic DNA. The fetal cell(s) or the fetal genomic DNA are isolated for example but not limited to the methods described herein.
2. Characterizing said sample by determining the methylation state or the methylation level of at least one CpG position within the sequence of CRYBA4 SEQ ID NO: 476 of the provided sample. Thereby a profile is generated comprising the methylation information of all characterized CpG positions of CRYBA4 SEQ ID NO: 476 of the respective sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

In a preferred embodiment of step 1, the genomic DNA comprising fetal genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).

In a preferred embodiment the determined CRYBA4 profile is compared with one or more CRYBA4 profiles of obtained from other samples and/or with one or more CRYBA4 reference profiles.

### Skin

In a preferred embodiment, the herein provided markers of Tables 8D, G, I and Tables 9D, F, H are used for identifying individuals from traces of skin and/ or adjacent tissues (such as hair, nail pieces, etc). This embodiment is of particular use in forensic medicine and/or legal medicine. Skin or skin adjacent tissue is usually used as study material in forensic and legal medicine. Preferably the markers provided in Table 8G and 9F are used because of the following reason. Keratinocytes constitute the external layer of the skin and therefore are the first cell type to be de-attached and a high number of these cells is expected in skin traces. Variations of one marker alone or in combination with other markers herein provided or not enable the accurate assessment of identity. For example but not limited to it, the differential methylation of NP_612444.1 SEQ ID NO: 689 is used. According to Table 6, the differential methylation of NP_612444.1 SEQ ID NO: 689 is a marker for keratinocytes as well as for sperm because the CpG dinucleotides of NP_612444.1 SEQ ID NO: 689 are methylated within the range of 0-25% in keratinocytes and sperm while other tissues show a different extend of methylation. Of course, corresponding markers can also be alternatively used. Thereby corresponding markers of NP_612444.1 SEQ ID NO: 689 are, for example but not limited to, genomic DNA derived from or associated with NP_612444.1 SEQ ID NO: 689; methylation specifically converted DNA derived from NP_612444.1 SEQ ID NO: 689; mRNA, cDNA, protein, or peptide each of which derived at least in parts from NP_612444.1 SEQ ID NO: 689. If the case may be, a person skilled in the art knows how to adjust the presented procedures.

Correspondingly, for example but not limited to it, a method for the identification of an individual, comprises
1. Providing a sample, comprising skin, hair, nail pieces and/or adjacent tissue. Genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing the sample by determining the methylation state or the methylation level of at least one CpG position within the sequence of NP_612444.1 SEQ ID NO: 689 of the provided sample. Thereby a profile is generated comprising the methylation information of all characterized CpG positions of NP_612444.1 SEQ ID NO: 689 of the sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3.Comparing the determined NP_612444.1 profile of the sample with one or more NP_612444.1 profiles of individuals, the profiles obtained correspondingly or in a different manner. An individual is identified wherein the NP_612444.1 profile matches the profile of an individual. In preferred embodiments, the forensic sample and the sample collected from an individual are collected and/or processed simultaneously or not.

In a preferred embodiment, the herein provided markers of Tables 8D, G, I and Tables 9D, F, H are used to characterize the skin, hair, nail, or adjacent tissue of an individual. For example but not limited to it, the differential methylation of SEQ ID NO: 640 (no gene associated) is used. According to Table 6, the differential methylation of SEQ ID NO: 640 is a marker for skin, hair, nail, or adjacent tissue because the CpG dinucleotides of SEQ ID NO: 640 are methylated within the range of 25-75% in keratinocytes, melanocytes and fibroblasts while other tissues show a different extend of methylation. Of course, corresponding markers can also be used alternatively. Thereby corresponding markers of SEQ ID NO: 640 are, for example but not limited to, genomic DNA derived from or associated with SEQ ID NO: 640; methylation specifically converted DNA derived from SEQ ID NO: 640; mRNA, cDNA, protein, or peptide each of which derived at least in parts from SEQ ID NO: 640. If the case may be, a person skilled in the art knows how to adjust the presented procedures.

Correspondingly, for example but not limited to, a method for characterizing skin, hair, nail and/or adjacent tissue comprises the characterization of at least keratinocytes, melanocytes, fibroblasts or combinations thereof, in addition comprising:
1. Providing a sample, comprising skin, hair, nail and/or adjacent tissue. Genomic DNA is purified from said samples, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing the sample by determining the methylation state or the methylation level of at least one CpG position within the sequence of SEQ ID NO: 640 of the provided sample. Thereby a profile is generated comprising the methylation information of all characterized CpG positions of SEQ ID NO: 640 of the sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

In a preferred embodiment, the herein provided markers of Tables 8D, G, I and Tables 9D, F, H are used to determine the composition of the skin, hair, nail, or adjacent tissue of an individual. Said composition being dependent from the content of at least one of the three major constituting cell types of the skin (fibroblasts, keratinocytes and melanocytes). For example but not limited to it, the differential methylation of SEQ ID NO: 644 (no gene associated), SEQ ID NO: 648 (no gene associated), PTPNS1 SEQ ID NO: 649, or combinations thereof are used. According to Table 6, the differential methylation of SEQ ID NO: 644 is a marker for melanocytes because the CpG dinucleotides of SEQ ID NO: 644 are methylated within the range of 0-25% in melanocytes while other tissues show a different extend of methylation. Of course, corresponding markers can also be used alternatively. Thereby corresponding markers of SEQ ID NO: 644 are, for example but not limited to, genomic DNA derived from or associated with SEQ ID NO: 644; methylation specifically converted DNA derived from SEQ ID NO: 644; mRNA, cDNA, protein, or peptide each of which derived at least in parts from SEQ ID NO: 644. According to Table 6, the differential methylation of SEQ ID NO: 648 is a marker for fibroblasts because the CpG dinucleotides of SEQ ID NO: 648 are methylated within the range of 0-25% in fibroblast while other tissues show a different extend of methylation. Of course, corresponding markers can also be used alternatively. Thereby corresponding markers of SEQ ID NO: 648 are, for example but not limited to, genomic DNA derived from or associated with SEQ ID NO: 648; methylation specifically converted DNA derived from SEQ ID NO: 648; mRNA, cDNA, protein, or peptide each of which derived at least in parts from SEQ ID NO: 648. According to Table 6, the differential methylation of PTPNS1 SEQ ID NO: 649 is a marker for keratinocytes because the CpG dinucleotides of PTPNS1 SEQ ID NO: 649 are methylated within the range of 0-25% in keratinocytes while other tissues show a different extend of methylation. Of course, corresponding markers can also be used alternatively. Thereby corresponding markers of PTPNS1 SEQ ID NO: 649 are, for example but not limited to, genomic DNA derived from or associated with PTPNS1 SEQ ID NO: 649; methylation specifically converted DNA derived from PTPNS1 SEQ ID NO: 649; mRNA, cDNA, protein, or peptide each of which derived at least in parts from PTPNS1 SEQ ID NO: 649. If the case may be, a person skilled in the art knows how to adjust the presented procedures.

Correspondingly, for example but not limited to, a method for quantifying the number of keratinocytes, fibroblast, melanocytes or combinations thereof comprises:
1. Providing of a sample, the sample comprising one or more cells or genomic DNA.
2. Binding of at least one probe to one or more CpG positions within the sequence of SEQ ID NO: 644, SEQ ID NO: 648, PTPNS1 SEQ ID NO: 649 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying keratinocytes, fibroblasts, melanocytes or combinations thereof by detecting the bound probes and/or their respective label(s). A person skilled in the art knows suitable methods for detection of said probes or labels.
4. Quantifying the isolated or purified cells by means of the attached probes and/or their corresponding tags. A person skilled in the art knows suitable methods. For example, but not limited to by cell counting manually or by automatic means. According to a preferred embodiment, the isolated or purified fetal genomic DNA is quantified by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™}method, MethyLight^{™} Algo^{™}method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method.

Alternatively, for example but not limited to, a method for quantifying the number of keratinocytes, fibroblast, melanocytes or combinations thereof comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of SEQ ID NO: 644, SEQ ID NO: 648, PTPNS1 SEQ ID NO: 649 or combinations thereof of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™}Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of keratinocytes, fibroblasts, melanocytes, or combinations thereof by comparing the determined methylation levels of the sample with the respective herein provided methylation levels of keratinocytes, fibroblasts or melanocytes.

In a preferred embodiment, the herein provided markers of Tables 8D, G, I and Tables 9D, F, H are used in the field of drugs. Said embodiment is of particular use for the development of drugs as well as for the treatment with drugs. The skin, hair, nail or adjacent tissue of an individual can be characterized by means of the provided markers of Tables 8D, G, I and Tables 9D, F, H. This information can then be used to develop new drugs or to access already existing drugs with regard to skin, hair, nail etc. of an individual or to subgroups of individuals. These subgroups are for example but not limited to be characterized by a disease and/or a defined type of skin or hair, etc. The efficiency of said drugs i.e. the presence or absence of the desired effect is also characterized or monitored by means of the provided markers of Tables 8D, G, I and Tables 9D, F, H. For example but not limited to it, the differential methylation of SEQ ID NO: 773 (no gene associated) is used. According to Table 6, the differential methylation of SEQ ID NO: 773 is a marker for skin, hair, nail, or adjacent tissue because the CpG dinucleotides of SEQ ID NO: 773 are methylated within the range of 0-25% in keratinocytes, melanocytes and fibroblasts while other tissues show a different extend of methylation. Of course, corresponding markers can also be used alternatively. Thereby corresponding markers of SEQ ID NO: 773 are, for example but not limited to, genomic DNA derived from or associated with SEQ ID NO: 773; methylation specifically converted DNA derived from SEQ ID NO: 773; mRNA, cDNA, protein, or peptide each of which derived at least in parts from SEQ ID NO: 773. If the case may be, a person skilled in the art knows how to adjust the presented procedures.

Correspondingly, for example but not limited to, a method for developing a drug and/or for treating an individual with a drug comprises:
1. Providing a sample obtained from an individual comprising genomic DNA of keratinocytes, melanocytes, fibroblasts or combinations thereof. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing said sample by determining the methylation status or level of at least one CpG position within the sequence of SEQ ID NO: 773 of the provided sample. Thereby a SEQ ID NO: 773 profile specific for said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Treating said individual with a drug.
4. Providing a sample of said individual after drug treatment.
5. Characterizing said sample after drug treatment by determining the methylation status or level of at least one CpG position within the sequence of SEQ ID NO: 773 of the provided sample. Thereby a SEQ ID NO: 773 profile specific for said sample after drug treatment is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies. 6. Comparing the determined SEQ ID NO: 773 profiles with drug treatment and without drug treatment with each other.

According to a preferred embodiment, guidelines are drawn from the comparison for the further drug development. According to another preferred embodiment, guidelines are drawn from the comparison for the treatment of an individual with said drug.

In a preferred embodiment, the herein provided markers of Tables 8D, G, I and Tables 9D, F, H are used as prognostic and/or diagnostic markers for wound healing, in particular in the field of surgery procedures wherein the skin is affected. For example but not limited to it, the differential methylation of SLC35E4 SEQ ID NO: 751 is used. According to Table 6, the differential methylation of SLC35E4 SEQ ID NO: 751 is a marker for wound healing because the CpG dinucleotides of SLC35E4 SEQ ID NO: 751 are methylated within the range of 0-25% in keratinocytes, melanocytes and fibroblasts while other tissues show a different extend of methylation. Of course, corresponding markers can also be used alternatively. Thereby corresponding markers of SLC35E4 SEQ ID NO: 751 are, for example but not limited to, genomic DNA derived from or associated with SLC35E4 SEQ ID NO: 751; methylation specifically converted DNA derived from SLC35E4 SEQ ID NO: 751; mRNA, cDNA, protein, or peptide each of which derived at least in parts from SLC35E4 SEQ ID NO: 751. If the case may be, a person skilled in the art knows how to adjust the presented procedures.

Correspondingly, for example but not limited to, a method for prognosing or diagnosing would healing comprises:
1. Providing a sample obtained from an individual comprising genomic DNA of keratinocytes, melanocytes, fibroblasts or combinations thereof. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing said sample by determining the methylation status or level of at least one CpG position within the sequence of SLC35E4 SEQ ID NO: 751 of the provided sample. Thereby a SLC35E4 profile specific for said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Comparing the determined SLC35E4 profile with at least one reference profile obtained from the same individual and/or from one or more other individuals. Preferably, said reference profiles are obtained according to steps 1 and 2. Preferably, said reference profiles comprises pairs of profiles each pair being obtained from an individual. Thereby said pair consists of a first profile specific for the condition of healthy skin (no lesion of skin) and of a second profile specific for a healing state of the wound or affected skin area after lesion. A person skilled in the art knows to deduce the possible grade of scare building and or the time required for healing therefrom.

In a preferred embodiment, said method is a method for diagnosing wound healing wherein the determined SLC35E4 profile matches a reference profile. In another preferred embodiment, said method is a method for prognosing wound healing wherein the determined SLC35E4 profile matches a first reference profile and wherein a second reference profile exists which was obtained from the same individual as the first reference profile. Thereby the second reference profile was obtained after the first reference profile. Accordingly, the prognosis for the individual for whom the SLC35E4 profile was determined is the condition of wound healing characterized by the second reference profile. In preferred embodiments, at least 1, 2, 4, 6, 10, 15, 25 or 50 reference profiles are considered. The more reference profiles are considered the merrier is the diagnosis or prognosis.

### Liver

In a preferred embodiment, the herein provided markers of Tables 8H and Tables 9G are used for deducing the presence of absence of an event which affects the liver. For example but not limited to it, said event is at least one select from the group comprising liver cirrhosis; liver cancer; hepatitis A; hepatitis B; hepatitis C; healthy condition, recently or longer chemical, physical or biological exposure; recently or longer exposure to a drug, or alcohol; or treatment procedures. In the case the event is adverse, said event affecting the liver leads to the death of liver cells. In the case the event is benign, said event leads to a reduction of liver cell death. The genomic DNA of dead liver cells can then be found in the body fluids in particular in the blood of a affected individual. As an example but not limited to, the differential methylation of VARS SEQ ID NO: 415 is used. According to Table 6, the differential methylation of VARS SEQ ID NO: 415 is a marker for diseases affecting the liver because the CpG dinucleotides of VARS SEQ ID NO: 415 are methylated within the range of 25-75% in liver while other tissues show a different extend of methylation. Of course, corresponding markers can also be used alternatively. Thereby corresponding markers of VARS SEQ ID NO: 415 are, for example but not limited to, genomic DNA derived from or associated with VARS SEQ ID NO: 415; methylation specifically converted DNA derived from VARS SEQ ID NO: 415; mRNA, cDNA, protein, or peptide each of which derived at least in parts from VARS SEQ ID NO: 415. If the case may be, a person skilled in the art knows how to adjust the presented procedures.

Correspondingly, for example but not limited to, a method for detecting a liver affecting event comprises:
1. Providing a sample comprising genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega). Preferably said sample is a blood sample, plasma sample, or urine sample.
2. Determining the methylation level of at least one CpG position within the sequence of VARS SEQ ID NO: 415 of the provided sample. Thereby a VARS profile specific of said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Deducing the presence or absence of a liver affecting event from the comparison of the determined VARS profile with one or more VARS reference profiles. Said reference profiles are specific for a healthy condition or a condition specific for an event. In the case the determined VARS profile matches or is similar to a reference profile specific for an event, said liver affecting event is present. In case the determined VARS profiles is not similar to a reference profile specific for an event, said liver affecting event is absent.

In addition, for example but not limited to it, a method for detecting a liver affecting event comprises the quantification of the amount of free floating genomic DNA of liver cells. Said method comprises:
1. Providing of a sample, comprising genomic DNA;
2. Contacting the genomic DNA or a derivative of it with at least one probe which is specific for at least one differentially methylated CpG position of the marker VARS SEQ ID NO: 415. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art is aware of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen). A person skilled in the art knows further suitable methods for detection.
4. Quantifying the detection reaction in a manner so that the detected signal is indicative for the amount of probe or label and therewith for the number of dead liver cell. A person knows suitable methods for quantification.
5. Deducing the presence or absence of a liver affecting event from the comparison of the determined number of dead liver cells with reference numbers of dead liver cells that are specific for an event. In the case the determined number matches or is similar to a reference number specific for a healthy condition, a liver affecting event is absent. In the case the determined number matches or is similar to a reference number specific for a condition specific for an event, a liver affecting event is present.

Alternatively, for example but not limited to, a method for detecting a liver affecting event comprises the quantification of the amount of free floating genomic DNA of liver cells. Said method comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of VARS SEQ ID NO: 415 of the provided sample. A person skilled in the art knows how to determine the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of dead liver cells by comparing the determined one or more methylation levels of the sample with the respective herein provided one or more methylation levels of liver cells and with at least one methylation level representing the correspondent tissue, group of cells, or cell of a healthy individual.

In a preferred embodiment, the herein provided markers of Tables 8H and Tables 9G are used for deducing the sensitivity of an individual to alcohol. Alcohol consumption changes the DNA methylation status as reviewed by Poschl et al, 2004 (Poschl G, Stickel F, Wang XD, Seitz HK. Alcohol and cancer: genetic and nutritional aspects. Proc Nutr Soc. 2004 Feb;63(1):65-71.). As an example but not limited to, the differential methylation of BMP7 SEQ ID NO: 684 is used. According to Table 6, the differential methylation of BMP7 SEQ ID NO: 684 is a marker for diseases affecting the liver because the CpG dinucleotides of BMP7 SEQ ID NO: 684 are methylated within the range of 25-75% in liver while other tissues show a different extend of methylation. Of course, corresponding markers can also be used alternatively. Thereby corresponding markers of BMP7 SEQ ID NO: 684 are, for example but not limited to, genomic DNA derived from or associated with BMP7 SEQ ID NO: 684; methylation specifically converted DNA derived from BMP7 SEQ ID NO: 684; mRNA, cDNA, protein, or peptide each of which derived at least in parts from BMP7 SEQ ID NO: 684. If the case may be, a person skilled in the art knows how to adjust the presented procedures.

Correspondingly, for example but not limited to, a method for deducing the sensitivity of an individual to alcohol comprises:
1. Providing a sample derived from said individual comprising genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega). Preferably said sample is a blood sample, plasma sample, or urine sample.
2. Determining the methylation level of at least one CpG position within the sequence of BMP7 SEQ ID NO: 684 of the provided sample. Thereby a BMP7 profile specific of said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Deducing the extend of a sensitivity of said individual to alcohol from the comparison of the determined BMP7 profile with one or more BMP7 reference profiles. The reference profiles are specific for different conditions of sensitivity. In the case the determined BMP7 profile matches or is similar to a reference profile, the sensitivity of said individual is identical or similar to the sensitivity for which the reference profile is specific for. In a preferred embodiment, the sensitivity to alcohol is determined for a cell, group of cell, or tissue according to the above described procedure.

### Heart Muscle

In a preferred embodiment, the herein provided markers of Tables 8E, Table 8F and Tables 9E are used for deducing the presence of absence of an event or condition affecting the heart. For example but not limited to it, said event or condition is at least one select from the group comprising heart failure; heart attack; athletic capacity; healthy condition; recently or longer chemical, physical or biological exposure; recently or longer exposure to a drug; or treatment procedure. In the case the event is adverse, said event or condition affecting the heart leads to death of heart cells. In the case the event is benign, said event leads to a reduction of heart cell death. The genomic DNA of dead heart cells can then be found in the body fluids in particular in the blood of an affected individual. As an example but not limited to, the differential methylation of TBC1D10A SEQ ID NO: 700 is used. According to Table 6, the differential methylation of TBC1D10A SEQ ID NO: 700 is a marker for diseases affecting the heart because the CpG dinucleotides of TBC1D10A SEQ ID NO: 700 are methylated within the range of 75-100% in heart while other tissues show a different extend of methylation. Of course, corresponding markers can also be used alternatively. Thereby corresponding markers of TBC1D10A SEQ ID NO: 700 are, for example but not limited to, genomic DNA derived from or associated with TBC1D10A SEQ ID NO: 700; methylation specifically converted DNA derived from TBC1D10A SEQ ID NO: 700; mRNA, cDNA, protein, or peptide each of which derived at least in parts from TBC1D10A SEQ ID NO: 700. If the case may be, a person skilled in the art knows how to adjust the presented procedures.

Correspondingly, for example but not limited to, a method for detecting a heart affecting event or condition comprises:
1. Providing a sample comprising genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega). Preferably said sample is a blood sample, plasma sample, or urine sample.
2. Determining the methylation level of at least one CpG position within the sequence of TBC1 D10A SEQ ID NO: 700 of the provided sample. Thereby a TBC1 D10A profile specific of said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Deducing the presence or absence of a heart affecting event or condition from the comparison of the determined TBC1D10A profile with one or more TBC1D10A reference profiles. Said reference profiles are specific for a healthy condition or a condition specific for an event. In case the determined TBC1D10A profile matches or is similar to a reference profile specific for an event or condition, the said heart affecting event or condition is present. In case the determined TBC1D10A profile is not similar to a reference profile specific for an event or condition, the said heart affecting event or condition is absent.

In addition, for example but not limited to it, a method for detecting a heart affecting event comprises the quantification of the amount of free floating genomic DNA of heart cells. Said method comprises:
1. Providing of a sample, comprising genomic DNA;
2. Contacting the genomic DNA or a derivative of it with at least one probe which is specific for at least one differentially methylated CpG position of the marker TBC1D10A SEQ ID NO: 700. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art is aware of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen). A person skilled in the art knows further suitable methods for detection.
4. Quantifying the detection reaction in a manner so that the detected signal is indicative for the amount of probe or label and therewith for the number of dead liver cell. A person knows suitable methods for quantification.
5. Deducing the presence or absence of a heart affecting event from the comparison of the determined number of dead heart cells with reference numbers of dead heart cells that are specific for an event. In the case the determined number matches or is similar to a reference number specific for an event or condition, said heart affecting event is present. In the case the determined number is not similar to a reference number specific for an event or condition, said heart affecting event is absent.

Alternatively, for example but not limited to, a method for detecting a heart affecting event comprises the quantification of the amount of free floating genomic DNA of heart cells. Said method comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of TBC1D10A SEQ ID NO: 700 of the provided sample. A person skilled in the art knows how to determine the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of dead heart cells by comparing the determined one or more methylation levels of the sample with the respective herein provided one or more methylation levels of heart cells and with at least one methylation level representing the correspondent tissue, group of cells, or cell of a healthy individual.

### Placenta

In a preferred embodiment, the herein provided markers of Table 8J and Table 91 are used for the study, monitoring, identification and/or quantification of placental cells or placental DNA circulating in maternal blood and /or amniotic fluid. Placenta constitute an extra-embryonic fetal tissue and as such, it shares many genetic characteristics with the fetal tissue. Therefore, cells from the placenta as well as DNA from placental cells can surrogate fetal cells and fetal DNA for diagnostic means. Fetal cells and fetal DNA have a diagnostic potential in monitoring the health status of the fetus as reviewed by Bianchi D, 2004 (Bianchi DW. Circulating fetal DNA: its origin and diagnostic potential-a review. Placenta. 2004 Apr;25 Suppl A:S93-S101 During pregnancy placenta cells are de-attached and brought to the maternal blood stream as well as the amniotic fluid. For example but not limited to it, the differential methylation of PRAME SEQ ID NO: 419 is used. According to Table 6, the differential methylation of PRAME SEQ ID NO: 419 is a marker for placenta because the CpG dinucleotides of PRAME SEQ ID NO: 419 are methylated within the range of 0-25% in placenta while other tissues show a different extend of methylation. Of course, corresponding markers can also be alternatively used. Thereby corresponding markers of PRAME SEQ ID NO: 419 are, for example but not limited to, genomic DNA derived from or associated with PRAME SEQ ID NO: 419; methylation specifically converted DNA derived from PRAME SEQ ID NO: 419; mRNA, cDNA, protein, or peptide each of which derived at least in parts from PRAME SEQ ID NO: 419. If the case may be, a person skilled in the art knows how to adjust the presented procedures.

Correspondingly, for example but not limited to, a method for identifying one or more placental cells or placental DNA comprises:
1. Providing of a sample, the sample comprising one or more cells or genomic DNA.
2. Binding of at least one probe to one or more CpG positions within the sequence of PRAME SEQ ID NO: 419 of the provided sample. Thereby a probe binds specifically with respect to the methylation status of said one or more CpG positions. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying placental cells or placental genomic DNA by detecting the bound probes and/or their respective label(s). A person skilled in the art knows suitable methods for detection of said probes or labels.

In preferred embodiments, a probe of step 2 binds specifically with respect to the methylation status of 1, 2, 3, 4, 5, 6, 7, 8, 9, 10, 11, 12, 13, 14, 15, 16, 17, 18, 19, 20, 22, 25, or 30 CpG dinucleotides.

For particular applications it is necessary to isolate or purify placental cell or placental genomic DNA. Accordingly, as an example but not limited to, a method for isolating and/or purifying placental cells or placental genomic DNA comprises in addition to the steps of the said method for identifying placental cells or placental genomic DNA:
3. Isolating and/or purifying of the identified placental cells or placental genomic DNA from the provided sample by means of the attached probes and their corresponding tags, respectively. A person skilled in the art knows suitable methods. Said methods are based on chemical, physical or biological properties of the attached probes or corresponding tags. For example but not limited to, the isolation is performed (i) by means of affinity cromatography, wherein the probe (e.g. a nucleic acid) is directly or indirectly bound to a solid surface; (ii) by means of affinity cromatography, wherein the probe is attached to a tag that is recognized by an antibody immobilized on a column; (iii) by means of magnetic beads, wherein a magnetic bead is directly or indirectly bound to an attached probe and wherein a magnetic field is applied; or (iv) by means of fluorescent activated cell sorting, wherein the used tag is a fluorescent dye.

According to a preferred embodiment, the isolated or purified placental cells or placental genomic DNA are quantified by means of the attached probes and/or their corresponding tags. A person skilled in the art knows suitable methods. For example, but not limited to by cell counting manually or by automatic means. According to a preferred embodiment, the isolated or purified placental genomic DNA is quantified by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method.

Alternatively, for example but not limited to, a method for quantifying the number of placental cells or the amount of placental genomic DNA comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of PRAME SEQ ID NO: 419 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of placental cells or the amount of placental genomic DNA by comparing the determined one or more methylation levels of the sample with the respective herein provided one or more methylation levels specific for placenta and with at least one methylation level representing the correspondent tissue, group of cells, or cell comprising no placental DNA.

For example but not limited to it, a method for characterizing one or more placental cells or placental genomic DNA comprises
1. Providing of a sample comprising one or more placental cells or placental genomic DNA. The placental cell(s) or the placental genomic DNA are isolated for example but not limited to the methods described herein.
2. Characterizing said sample by determining the methylation state or the methylation level of at least one CpG position within the sequence of PRAME SEQ ID NO: 419 of the provided sample. Thereby a profile is generated comprising the methylation information of all characterized CpG positions of PRAME SEQ ID NO: 419 of the respective sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

In a preferred embodiment of step 1, the genomic DNA comprising placental genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).

In a preferred embodiment, the determined PRAME profile is compared with one or more PRAME profiles obtained from other samples of the same individual and/or with one or more PRAME reference profiles of a normal pregnancy.

In a preferred embodiment, the herein provided markers of Table 8J and Table 9I are used for to the monitoring of embryonic development or the monitoring of placental development, in particular of extra-embryonic tissue or of interaction of extra-embryonic tissue with maternal placental tissue. Said embodiment comprises one or more of said methods for identifying one or more placental cells or placental DNA; one or more of said methods for isolating and/or purifying one or more placental cells or placental genomic DNA; one or more of said methods for quantifying the number of placental cells or the amount of placental genomic DNA; one or more of said methods for characterizing one or more placental cells or placental genomic DNA; or combinations thereof. application is for example but not limited

In a preferred embodiment, the herein provided markers of Table 8J and Table 9I are used for the study, monitoring, identification and/or quantification of placental cells in regenerative medicine, in particular in the field of tissue engineering. Therefore the above described methods (a method for identifying one or more placental cells or placental DNA; a method for isolating and/or purifying one or more placental cells or placental genomic DNA; a method for quantifying the number of placental cells or the amount of placental genomic DNA; a method for characterizing one or more placental cells or placental genomic DNA; a method for monitoring one or more placental cells) or combinations thereof are used. Thereby the said methods are applied to placental cells or to cells derived from placental cells. Furthermore the methods are applied in particular before and after storage, before and after cell differentiation, before and after cell proliferation, before and after cell culture expansion, and before and after tissue expansion as well as before and after transplantation. For example but not limited to, the differential methylation of GPR24 SEQ ID NO: 436 is used. According to Table 6, the differential methylation of GPR24 SEQ ID NO: 436 is a marker for placenta because the CpG dinucleotides of GPR24 SEQ ID NO: 436 are methylated within the range of 25-75% in placenta while other tissues show a different extend of methylation. Said methods (a method for identifying one or more placental cells or placental DNA; a method for isolating and/or purifying one or more placental cells or placental genomic DNA; a method for quantifying the number of placental cells or the amount of placental genomic DNA; a method for characterizing one or more placental cells or placental genomic DNA; a method for monitoring one or more placental cells) are carried out as described above, wherein PRAME SEQ ID NO: 419 is substituted by GPR24 SEQ ID NO: 436. Of course, corresponding markers can also be alternatively used. Thereby corresponding markers of GPR24 SEQ ID NO: 436 are, for example but not limited to, genomic DNA derived from or associated with GPR24 SEQ ID NO: 436; methylation specifically converted DNA derived from GPR24 SEQ ID NO: 436; mRNA, cDNA, protein, or peptide each of which derived at least in parts from GPR24 SEQ ID NO: 436. If the case may be, a person skilled in the art knows how to adjust the said methods.

### Sperm

The herein provided markers of Table 8L are used for diagnosing a male infertility related disease. A major cause of male infertility is either a low amount of sperm cells (spermatozoa) in the ejaculate (oligospermia) or a complete lack of sperm cells (spermatozoa) in the ejaculate (azoospermia). Thus, methods for the quantification of sperm cells are widely used in diagnosis of male infertility. In addition methylation analysis of sperm cells can be used as a tool to access the viability of the said. For example but not limited to, the differential methylation of GAL3ST1 SEQ ID NO: 437 is used. According to Table 6, the differential methylation of GAL3ST1 SEQ ID NO: 437 is a marker for sperm because the CpG dinucleotides of GAL3ST1 SEQ ID NO: 437 are methylated within the range of 0-25% in sperm while other tissues show a different extend of methylation. Of course, corresponding markers can also be alternatively used. Thereby corresponding markers of GAL3ST1 SEQ ID NO: 437 are, for example but not limited to, genomic DNA derived from or associated with GAL3ST1 SEQ ID NO: 437; methylation specifically converted DNA derived from GAL3ST1 SEQ ID NO: 437; mRNA, cDNA, protein, or peptide each of which derived at least in parts from GAL3ST1 SEQ ID NO: 437. If the case may be, a person skilled in the art knows how to adjust the presented procedures.

Correspondingly, for example but not limited to, a method for diagnosing a male infertility related disease comprises the quantification and/or characterization of sperm cells in the ejaculate.

For example, but not limited to, a method for characterization of sperm cells in the ejaculate comprises:
1. Providing of a sample comprising ejaculate or genomic DNA derived from an ejaculate. The genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing said sample by determining the methylation state or the methylation level of at least one CpG position within the sequence of GAL3ST1 SEQ ID NO: 437 of the provided sample. Thereby a profile is generated comprising the methylation information of all characterized CpG positions of GAL3ST1 SEQ ID NO: 437 of the respective sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.

According to a preferred embodiment, the determined GAL3ST1 profile of a provided sample is compared with at least one GAL3ST1 reference profile. Said reference profile is either obtained from a different individual or from the same individual. The reference profile is characterized in that it is specific for a defined state of viability of sperm cells. In a preferred preferred embodiment several reference profiles are used each of which is specific for a defined state of viability of sperm cells in the ejaculate and therefore for a defined value of infertility or fertility. In case a determined GAL3ST1 profile matches or is similar to a reference profile, it is deduced that the viability of sperm cells of the correspondent individual is characterized by the said defined state of viability of the reference.

Alternatively, for example, but not limited to, a method for characterizing sperm cells in an ejaculate comprises:
1. Providing of a sample comprising ejaculate or genomic DNA derived from an ejaculate.
2. Binding of at least one probe to one or more CpG positions within the sequence of GAL3ST1 SEQ ID NO: 437 of the provided sample. Thereby a probe binds specifically with respect to the methylation status of said one or more CpG positions creating a characteristic binding pattern. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.

According to a preferred embodiment, the determined GAL3ST1 binding pattern is compared with at least one GAL3ST1 reference binding pattern. Said reference binding pattern is either obtained from a different individual or from the same individual. The reference binding pattern is characterized in that it is specific for a defined state of viability of sperm cells. In a preferred embodiment several reference binding patterns are used each of which is specific for a defined state of viability of sperm cells in the ejaculate and therefore for a defined value of infertility or fertility. In case a determined GAL3ST1 binding pattern matches or is similar to a reference binding pattern, it is deduced that the viability of sperm cells of the correspondent individual is characterized by the said defined state of viability of the reference.

For example, but not limited to, a method for quantification of sperm cells in the ejaculate comprises the identification of sperm cells. For example, but not limited to, a method for identification of sperm cells comprises:
1. Providing of a sample comprising ejaculate or genomic DNA derived from an ejaculate.
2. Binding of at least one probe to one or more CpG positions within the sequence of GAL3ST1 SEQ ID NO: 437 of the provided sample. Thereby a probe binds specifically with respect to the methylation status of said one or more CpG positions creating a characteristic binding pattern. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying sperm cells or sperm genomic DNA by detecting the bound probes and/or their respective label(s). A person skilled in the art knows suitable methods for detection of said probes or labels.

In preferred embodiments, a probe of step 2 binds specifically with respect to the methylation status of 1, 2, 3, 4, 5, 6, 7, 8, 9, 10, 11, 12, 13, 14, 15, 16, 17, 18, 19, 20, 22, 25, or 30 CpG dinucleotides.

The said method for quantification of sperm cells in the ejaculate comprising the identification of sperm cells additional comprises:
Quantifying the identified sperm cells by counting or quantifying the labeled sperm genomic DNA by quantifying the bound probes and/or their respective label(s). A person skilled in the art knows suitable methods for quantification and/or counting.

Alternatively, for example but not limited to, a method for quantification of sperm cells in the ejaculate comprises:
1. Providing of a sample comprising ejaculate or genomic DNA derived from an ejaculate. The genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega)..
2. Determining the methylation level of at least one CpG position within the sequence of GAL3ST1 SEQ ID NO: 437 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of sperm cells or the amount of sperm genomic DNA by comparing the determined one or more methylation levels of the sample with the respective herein provided one or more methylation levels specific for sperm and with at least one methylation level representing an ejaculate comprising no sperm cell or a defined amount of sperm cells.

For example but not limited to, a method for diagnosing fertility or infertility for a male individual comprises:
1. Quantifying the amount of sperm cells in the ejaculate of said individual.
2. Comparing said amount with at least one reference value. The reference value is specific for a defined fertility or infertility state. In case the quantified amount of sperm cells matches or is similar to the reference value, it is deduced that the fertility or infertility of said individual has the same fertility or infertility which is specific for the reference value.

In a preferred embodiment, the herein provided markers of Table 8L are used for increasing the fertility of a male individual. As said above male fertility is often limited by the amount of sperm cells in the ejaculate. Thus, male fertility can be enhanced by enriching, isolating or purifying sperm cells. For example but not limited to, the differential methylation of APOL4 SEQ ID NO: 486 is used. According to Table 6, the differential methylation of APOL4 SEQ ID NO: 486 is a marker for sperm because the CpG dinucleotides of APOL4 SEQ ID NO: 486 are methylated within the range of 75-100% in sperm while other tissues show a different extend of methylation. Of course, corresponding markers can also be alternatively used. Thereby corresponding markers of APOL4 SEQ ID NO: 486 are, for example but not limited to, genomic DNA derived from or associated with APOL4 SEQ ID NO: 486; methylation specifically converted DNA derived from APOL4 SEQ ID NO: 486; mRNA, cDNA, protein, or peptide each of which derived at least in parts from APOL4 SEQ ID NO: 486. If the case may be, a person skilled in the art knows how to adjust the presented procedures.

Correspondingly, for example but not limited to, a method for increasing the fertility of a male individual comprises:
1. Identification of sperm cells in an ejaculate by means of the above described method for identification of sperm cells, wherein GAL3ST1 SEQ ID NO: 437 is substitutes by APOL4 SEQ ID NO: 486.
2. Enriching, isolating or purifying sperm cells by means of the attached probes and their corresponding tags, respectively. A person skilled in the art knows suitable methods. Said methods are based on chemical, physical or biological properties of the attached probes or corresponding tags. For example but not limited to, the isolation is performed (i) by means of affinity cromatography, wherein the probe (e.g. a nucleic acid) is directly or indirectly bound to a solid surface; (ii) by means of affinity cromatography, wherein the probe is attached to a tag that is recognized by an antibody immobilized on a column; (iii) by means of magnetic beads, wherein a magnetic bead is directly or indirectly bound to an attached probe and wherein a magnetic field is applied; or (iv) by means of fluorescent activated cell sorting, wherein the used tag is a fluorescent dye.

In a preferred embodiment, the herein provided markers of Table 8L are used for assisted fertilization procedures. Assisted fertilization procedures are for example but not limited to intracytoplasmic sperm injection (ICSI) or in vitro fertilization (IVF). All assisted fertilization procedures require the management of sperm cells prior to the procedure. Such management comprises at least the characterization, identification, quantification, enrichment, isolation, purification of sperm cells or combinations thereof. Therefore the above described methods for characterizing, identifying, quantifying, enriching, isolating and purifying sperm cells are applied.

In a preferred embodiment, the herein provided markers of Table 8L are used in the fields of forensic and/or legal medicine. By use of the said markers it is possible to determine the presence or absence of sperm in a sample. Furthermore, it is possible to identify an individual by use of said markers. For example but not limited to, the differential methylation of TCN2 SEQ ID NO: 470 is used. According to Table 6, the differential methylation of TCN2 SEQ ID NO: 470 is a marker for sperm because the CpG dinucleotides of TCN2 SEQ ID NO: 470 are methylated within the range of 75-100% in sperm while other tissues show a different extend of methylation. Of course, corresponding markers can also be alternatively used. Thereby corresponding markers of TCN2 SEQ ID NO: 470 are, for example but not limited to, genomic DNA derived from or associated with TCN2 SEQ ID NO: 470; methylation specifically converted DNA derived from TCN2 SEQ ID NO: 470; mRNA, cDNA, protein, or peptide each of which derived at least in parts from TCN2 SEQ ID NO: 470. If the case may be, a person skilled in the art knows how to adjust the presented procedures.

Correspondingly, for example but not limited to, a method for identifying sperm cells in a sample and/or for identifying an individual comprises:
1. Providing a sample comprising genomic DNA.
2. Binding of at least one probe to one or more CpG positions within the sequence of TCN2 SEQ ID NO: 470 of the provided sample. Thereby a probe binds specifically with respect to the methylation status of said one or more CpG positions and/or with respect to the methylation status of said one or more CpG positions and an individual. A probe is either a protein, peptide, nucleic acid, RNA or DNA for example but not limited to, an antibody specific for 5-methylcytosine (e.g. AbCAM Cat. No. ab1884); a methyl-binding protein such as the proteins MeCP2, MBD1, MBD2, MBD4, Kaiso or any domain thereof; or a nucleic acid probe that is specific for the methylated sequence. According to some preferred embodiments, the said probe(s) are labeled with a tag suitable for detection of the probe, isolation of one or more cells, and/or purification of one or more cells. A person skilled in the art knows suitable methods to carry out this step.
3. Identifying sperm cells or sperm genomic DNA or identifying sperm cells or sperm genomic DNA of an individual by detecting the bound probes and/or their respective label(s). A person skilled in the art knows suitable methods for detection of said probes or labels.

In addition, for example but not limited to, an alternative method for identifying an individual comprises:
1. Providing of a sample comprising genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of TCN2 SEQ ID NO: 470 of the provided sample. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Comparing the determined methylation level(s) of said sample with respective TCN2 SEQ ID NO: 470 methylation level(s) of at least one sample obtained from at least one candidate individual. Wherein the determined methylation level(s) matches or is similar to the respective methylation level(s) of an individual, it its deduced that the provided sample comprises sperm genomic DNA or sperm cells of said individual. Thereby an individual is identified.

### Skeletal Muscle

In a preferred embodiment, the herein provided markers of Table 8F, 8K and Table 9J are used for characterizing the efficiency of skeletal muscle cells. This embodiment is of particular value in the field of sports medicine. For example but not limited to, the differential methylation of CARD10 SEQ ID NO: 498 is used. According to Table 6, the differential methylation of CARD10 SEQ ID NO: 498 is a marker for skeletal muscle because the CpG dinucleotides of CARD10 SEQ ID NO: 498 are methylated within the range of 0-25% in skeletal muscle while other tissues show a different extend of methylation. Of course, corresponding markers can also be alternatively used. Thereby corresponding markers of CARD10 SEQ ID NO: 498 are, for example but not limited to, genomic DNA derived from or associated with CARD10 O SEQ ID NO: 498; methylation specifically converted DNA derived from CARD10 SEQ ID NO: 498; mRNA, cDNA, protein, or peptide each of which derived at least in parts from CARD10 SEQ ID NO: 498. If the case may be, a person skilled in the art knows how to adjust the presented procedures.

Correspondingly, for example but not limited to, a method for characterizing the efficiency of a skeletal muscle comprises:
1. Providing of a sample comprising genomic DNA of a skeletal muscle. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing the provided sample by Determining the methylation level of at least one CpG position within the sequence of CARD10 SEQ ID NO: 498 of the provided sample. Thereby a CARD 10 profile is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Comparing the generated CARD10 profile with at least one CARD10 reference profile. Thereby each reference profile is characteristic for a defined efficiency. Wherein the determined CARD10 profile matches or is similar to a CARD10 reference profile, the skeletal muscle from whom the analyzed sample is provided has the same efficiency as the said reference.

In a preferred embodiment, the herein provided markers of Table 8F, 8K and Table 9J are used for identifying fully differentiated muscle cells in cell culture. This is of particular value in the field of tissue engineering. Muscle cells are generate in cell culture by cultivation and differentiation of muscle cell progenitor cells. Fully differentiated skeletal muscle cells can be identified by means of the provided markers of Table 8F, 8K and Table 9J. For example but not limited to, the differential methylation of HTF9C_HUMAN SEQ ID NO: 500 is used. According to Table 6, the differential methylation of HTF9C_HUMAN SEQ ID NO: 500 is a marker for skeletal muscle because the CpG dinucleotides of HTF9C_HUMAN SEQ ID NO: 500 are methylated within the range of 25-75% in skeletal muscle while other tissues show a different extend of methylation. Of course, corresponding markers can also be alternatively used. Thereby corresponding markers of HTF9C_HUMAN SEQ ID NO: 500 are, for example but not limited to, genomic DNA derived from or associated with HTF9C_HUMAN SEQ ID NO: 500; methylation specifically converted DNA derived from HTF9C_HUMAN SEQ ID NO: 500; mRNA, cDNA, protein, or peptide each of which derived at least in parts from HTF9C_HUMAN SEQ ID NO: 500. If the case may be, a person skilled in the art knows how to adjust the presented procedures.

Correspondingly, for example but not limited to, a method for identifying fully in vitro differentiated muscle cell comprises:
1. Providing of a sample comprising genomic DNA of a skeletal muscle. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Characterizing the provided sample by Determining the methylation level of at least one CpG position within the sequence of HTF9C_HUMAN SEQ ID NO: 500 of the provided sample. Thereby a HTF9C_HUMAN profile is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl™ MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Comparing the generated HTF9C_HUMAN profile with a HTF9C_HUMAN reference profile which is characteristic for a fully differentiated muscle cell. Wherein the determined HTF9C_HUMAN profile matches or is similar to the reference profile, the analyzed skeletal muscle cell is considered as fully differentiated.

In a preferred embodiment, the herein provided markers of Table 8F, 8K and Table 9J are used for diagnosing muscle cell associated diseases, in particular disease which are characterized by a death of muscle cells like muscular distrophy. The DNA of dead muscle cells is found in body fluids such as blood or urine. This DNA is identified by means of the herein provided markers of Table 8F, 8K and Table 9J. For example but not limited to, the differential methylation of EYA2 SEQ ID NO: 678 is used. According to Table 6, the differential methylation of EYA2 SEQ ID NO: 678 is a marker for skeletal muscle because the CpG dinucleotides of EYA2 SEQ ID NO: 678 are methylated within the range of 25-75% in skeletal muscle while other tissues show a different extend of methylation. Of course, corresponding markers can also be alternatively used. Thereby corresponding markers of EYA2 SEQ ID NO: 678 are, for example but not limited to, genomic DNA derived from or associated with EYA2 SEQ ID NO: 678; methylation specifically converted DNA derived from EYA2 SEQ ID NO: 678; mRNA, cDNA, protein, or peptide each of which derived at least in parts from EYA2 SEQ ID NO: 678. If the case may be, a person skilled in the art knows how to adjust the presented procedures.

Correspondingly, for example but not limited to, a method for diagnosing muscle cell associated diseases comprises:
1. Providing of a sample derived from blood or urine, the sample comprising genomic DNA. The genomic DNA is isolated/purified from the provided sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega)..
2. Characterizing the sample by Determining the methylation level of at least one CpG position within the sequence of EYA2 SEQ ID NO: 678 of the provided sample. Thereby a EAY2 profile is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Deducing the presence or absence of a muscle associated disease from comparison of the determined EYA2 profile with at least one EYA2 reference profile. A EYA2 reference profile comprises the same position(s) as the determined profile. In addition a EYA2 profile is specific for either skeletal muscle cells (as herein provided by Table 8F, 8K and Table 9J), blood or urine comprising no skeletal muscle cell DNA, or blood or urine derived from a healthy individual.

### CD8 T-lymphocytes

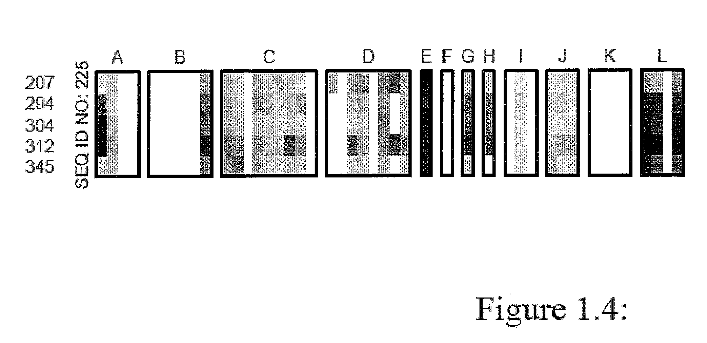

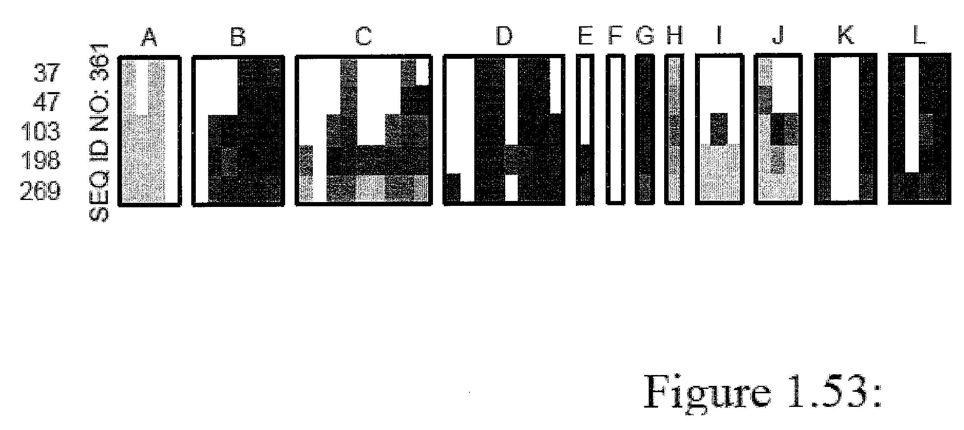

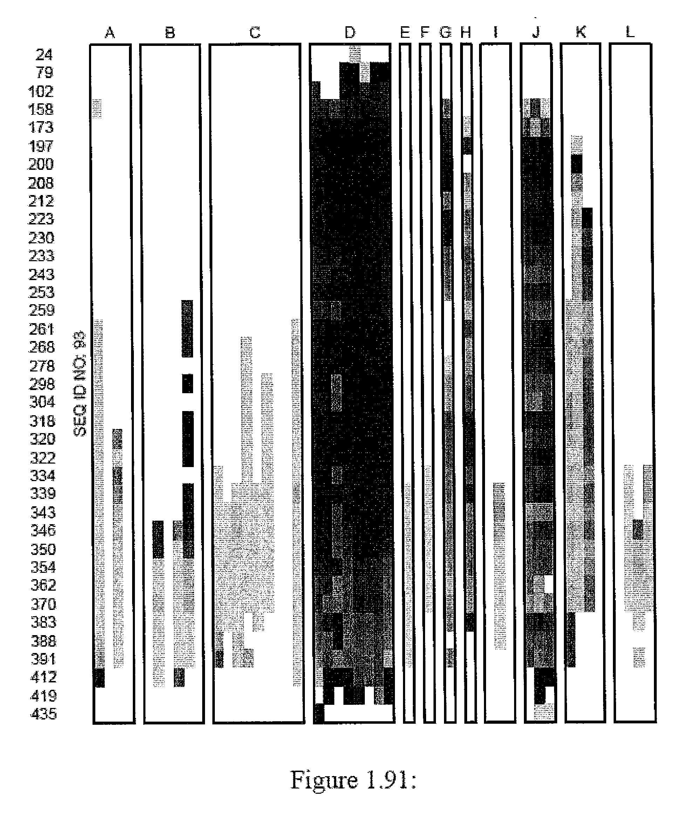

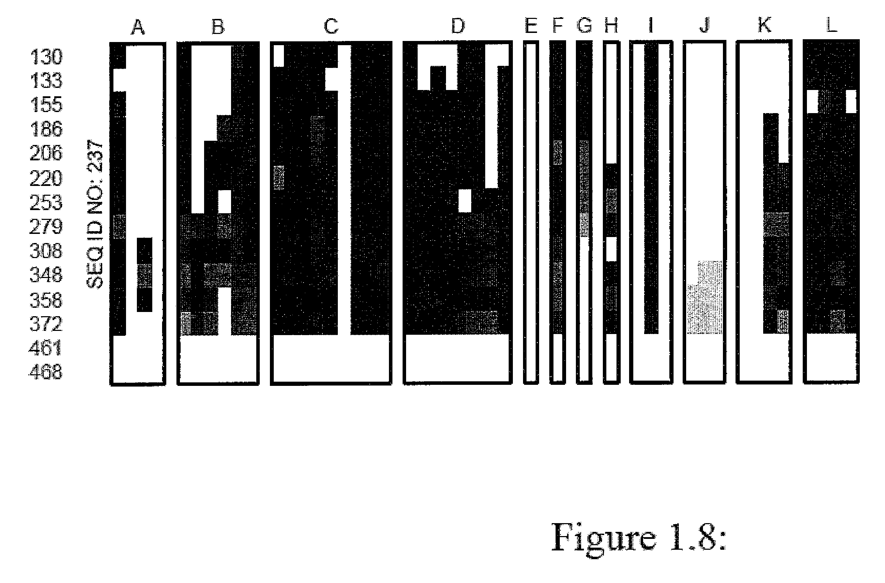

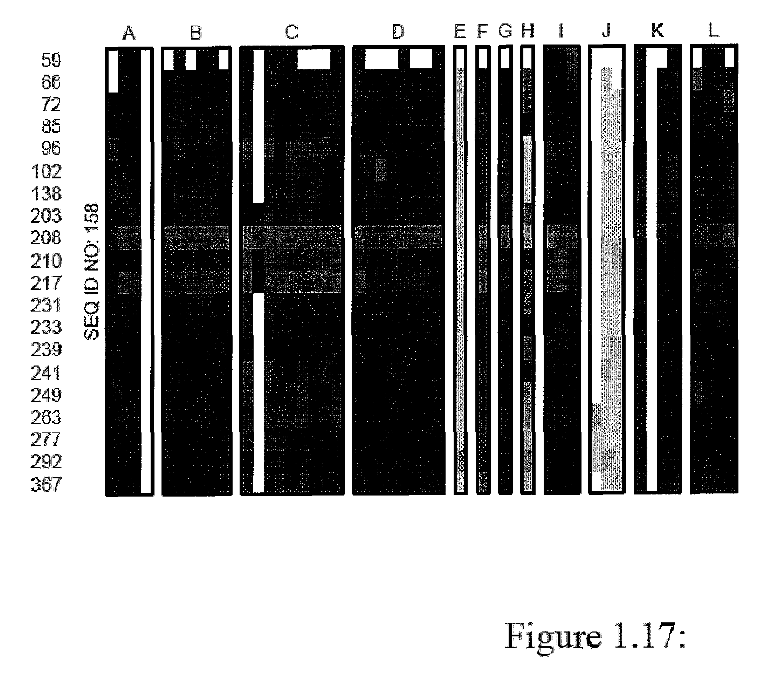

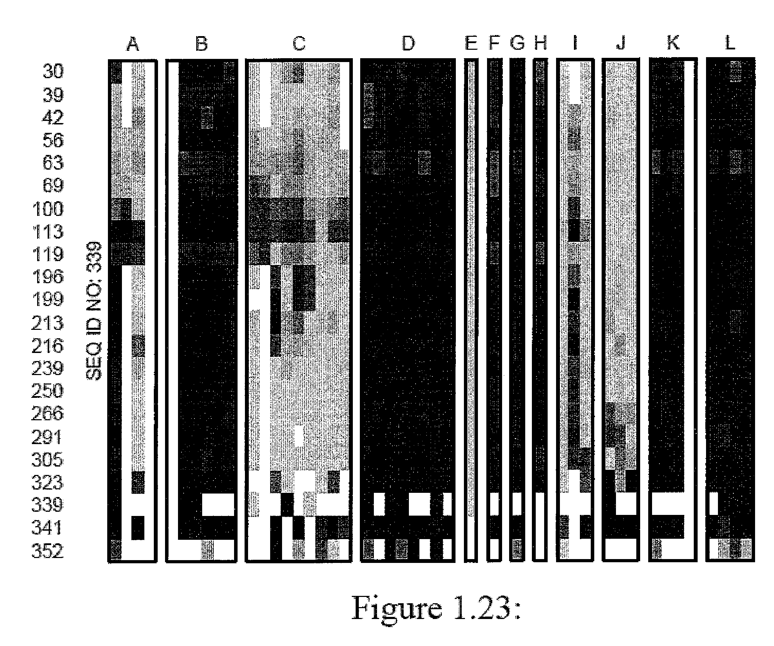

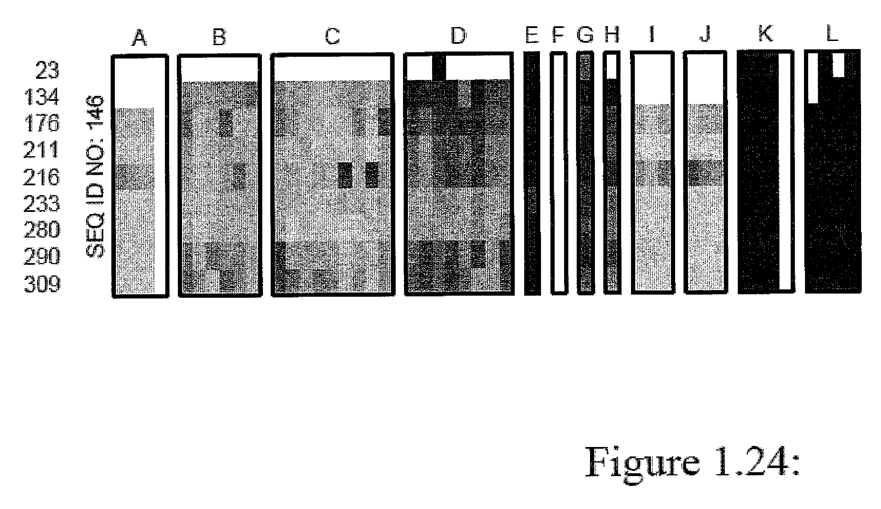

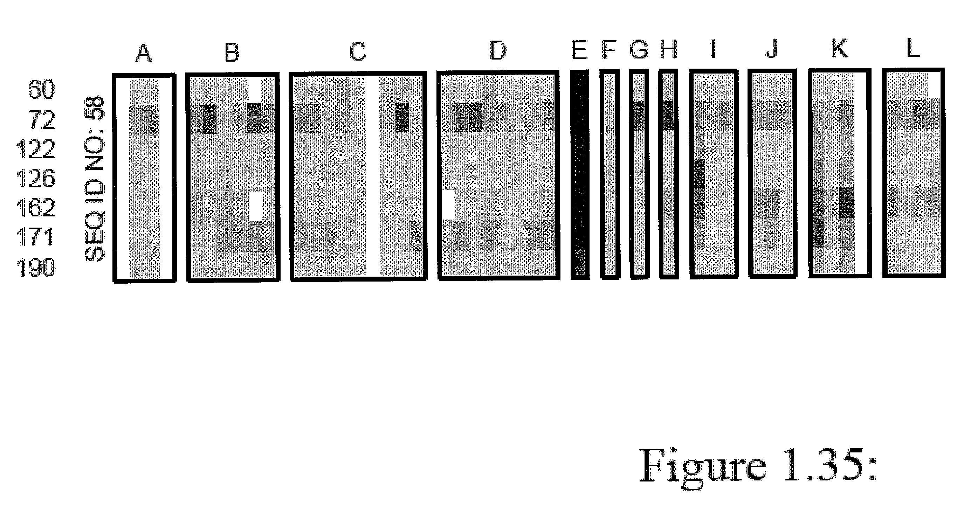

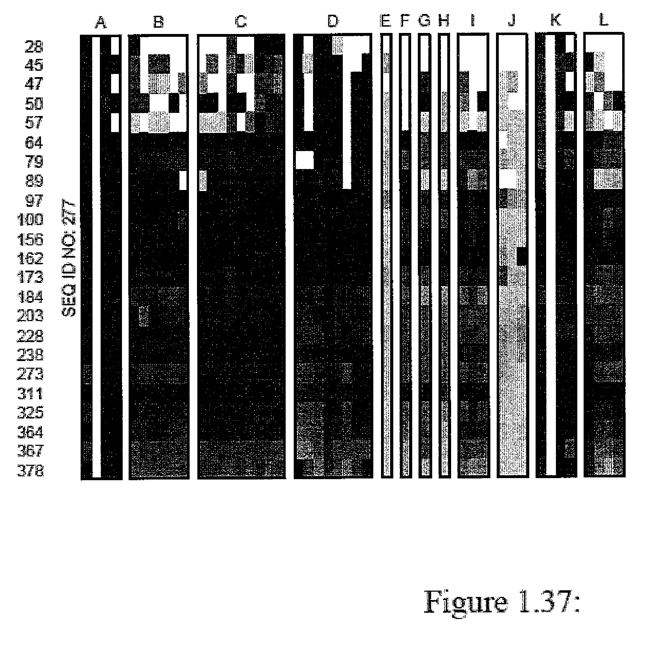

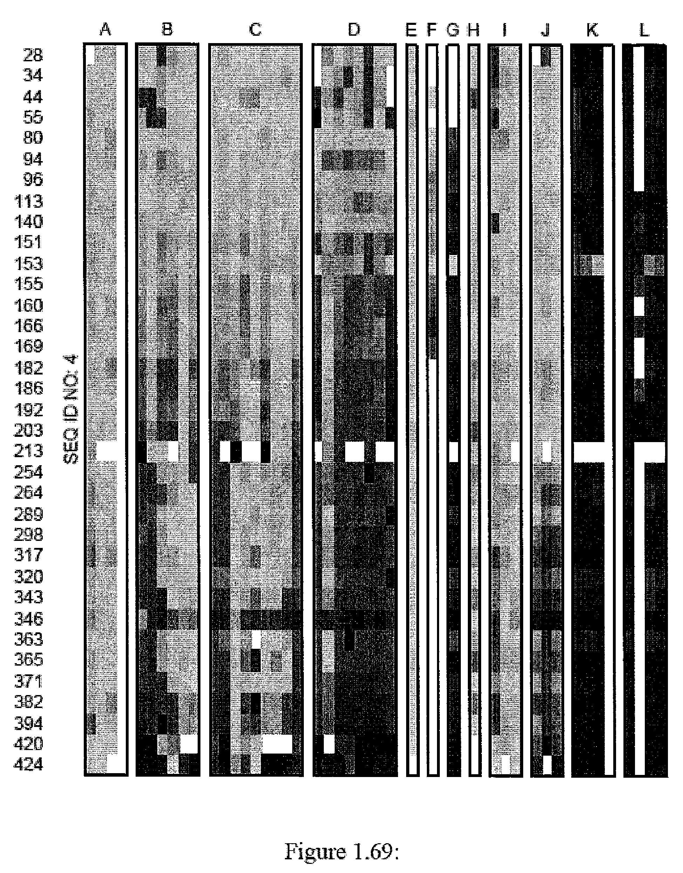

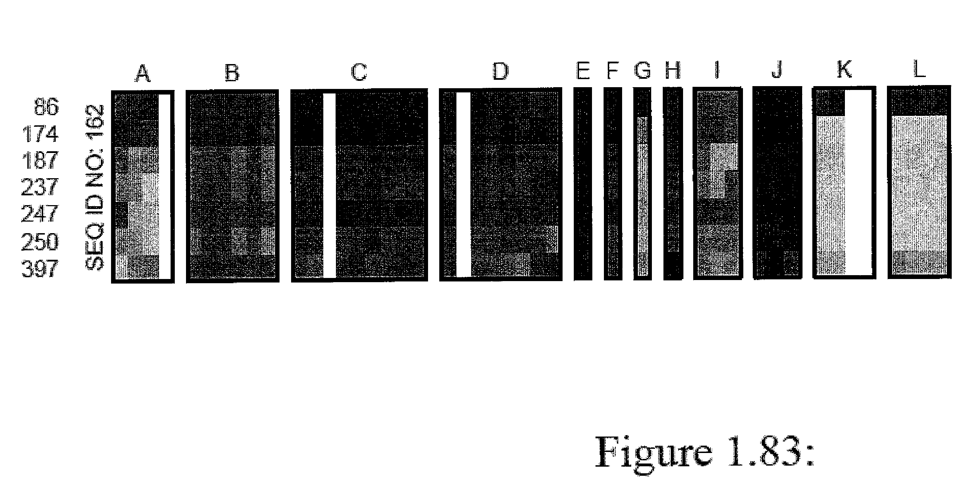

In a preferred embodiment, the herein provided markers of Table 8A specific only for CD8 T-lymphocytes are used for quantifying CD8 T-lymphocytes, in particular for monitoring the immune system of individuals infected with HIV. The periodically determining of the number of CD8 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection. For example but not limited to, the differential methylation of RP4-695O20_B.9 SEQ ID NO: 706 is used. According to Figure 1.296 and to Table 6, the differential methylation of RP4-695O20_B.9 SEQ ID NO: 706 is a marker for CD8 T-lymphocytes because the CpG dinucleotides of RP4-695O20_B.9 SEQ ID NO: 706 are methylated within the range of 75-100% in CD8 T-lymphocytes, sperm and liver while other tissues show a different extend of methylation. Furthermore CD8 T-lymphocytes can easily be distinguished from sperm or liver morphologically or by means of other markers. Of course, corresponding markers can also be alternatively used. Thereby corresponding markers of RP4-695O20_B.9 SEQ ID NO: 706 are, for example but not limited to, genomic DNA derived from or associated with RP4-695O20_B.9 SEQ ID NO: 706; methylation specifically converted DNA derived from RP4-695O20_B.9 SEQ ID NO: 706; mRNA, cDNA, protein, or peptide each of which derived at least in parts from RP4-695O20_B.9 SEQ ID NO: 706. If the case may be, a person skilled in the art knows how to adjust the presented procedures.

Correspondingly, for example but not limited to, a method for quantifying CD8 T-lymphocytes comprises:
1. Providing a sample comprising genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega). Preferably said sample is a blood sample, plasma sample, or urine sample.
2. Determining the methylation level of at least one CpG position within the sequence of RP4-695O20_B.9 SEQ ID NO: 706 of the provided sample. Thereby a RP4-695O20_B.9 profile specific of said sample is generated. A person skilled in the art knows how to determine the methylation state or the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl^{™} method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Deducing the number of CD8 T-lymphocytes from the comparison of the determined RP4-695O20_B.9 profile with one or more RP4-695O20_B.9 reference profiles. Said reference profiles are specific for a defined number of CD8 T-lymphocytes. In addition the reference profiles are obtained from the same sample type as the provided sample. For example but not limited to, blood is the sample type for the provided sample and the reference profiles. In a preferred embodiment, wherein the determined RP4-695O20_B.9 profile matches or is similar to a reference profile specific for a defined number of CD8 T-lymphocytes, the said number of CD8 T-lymphocytes is present in the analyzed sample. In a preferred embodiment, a calibration curve is prepared form the reference profiles. The number of CD8 T-lymphocytes is then deduced by comparing the determined RP4-695O20_B.9 profile with the calibration curve. Of course, other algorithms as the are known in the art are also preferred.

In addition, for example but not limited to it, a method for quantifying CD8 T-lymphocytes comprises:
1. Providing of a sample, comprising genomic DNA;
2. Contacting the genomic DNA or a derivative of it with at least one probe which is specific for at least one differentially methylated CpG position of the marker RP4-695O20_B.9 SEQ ID NO: 706. Said probe is selected from the group comprising antibody; 5-methylcytosine specific antibody (e.g. AbCam Cat. No. ab1884); affinity binding protein; protein binding specifically methylated or unmethylated DNA like MeCP2, MBD1, MBD2, MBD4, Kaiso or any domain thereof; nucleic acid; DNA, RNA, PNA or nucleic acid derivative specific for the methylated sequence. In addition, the probe is labeled directly or indirectly with a dye, protein, enzyme, metal, bead or chemical compound suitable for detection.
3. Performing a detection reaction by means of the probe and/or the label. A person skilled in the art is aware of suitable detection reactions. For example, but not limited to, the detection reaction comprises Rabbit Peroxidase Anti-Peroxidase (PAP) Soluble Complex (Rockland Catalog#: P300-002); radioactive labeled probes; or probes fluorescently labeled like DNA probes coupled with Cy5 (Invitrogen). A person skilled in the art knows further suitable methods for detection.
4. Quantifying the detection reaction in a manner so that the detected signal is indicative for the amount of probe or label and therewith for the number of dead liver cell. A person knows suitable methods for quantification.

Alternatively, for example but not limited to, a method for quantifying CD8 T-lymphocytes comprises:
1. Providing of a sample, the sample comprising one or more cells or genomic DNA. The genomic DNA is purified from said sample, preferably by means of a kit. A person skilled in the art is aware of suitable kits. For example, but not limited to it, the kit for purification of genomic DNA is the DNeasy Tissue Kit (Qiagen) or the Wizard Genomic DNA Purification kit (Promega).
2. Determining the methylation level of at least one CpG position within the sequence of RP4-695O20_B.9 SEQ ID NO: 706 of the provided sample. A person skilled in the art knows how to determine the methylation level of one or more CpG positions. For example, but not limited to, the methylation state or level is determined by means of at least one selected from the group comprising amplification method, PCR method, isothermal amplification method, NASBA method, LCR method, methylation specific amplification method, MSP (Methylation Specific PCR) method, nested MSP method, HeavyMethyl™ method, detection method, methylation specific detection method, bisulfite sequencing method, detection by means of DNA-arrays, detection by means of oligonucleotide microarrays, detection by means of CpG-island-microarrays, detection by means of restriction enzymes, detection by means of methylation sensitive restriction enzymes simultaneous methylation specific amplification and detection method, COBRA method, real-time PCR, HeavyMethyl^{™} real time PCR method, MSP MethyLight^{™} method, MethyLight^{™} method, MethyLight^{™} Algo^{™} method, QM method, Headloop MethyLight^{™} method, HeavyMethyl^{™} MethyLight^{™} method, HeavyMethyl^{™} Scorpion^{™} method, MSP Scorpion^{™} method, Headloop Scorpion^{™} method, methylation sensitive primer extension, Ms-SNuPE (Methylation-sensitive Single Nucleotide Primer Extension) method, and proteins binding specifically methylated or unmethylated DNA like the proteins MeCP2, MBD1, MBD2, MBD4, Kaiso or any domain thereof like but not limited to the CXXC-3 domain of the MBD1 protein or methylation-specific antibodies, e.g. anti-5-methylcytosine antibodies.
3. Quantifying the number of CD8 T-lymphocytes by comparing the determined one or more methylation levels of the sample with the respective herein provided one or more methylation levels of CD8 T-lymphocytes and with at least one methylation level representing the correspondent tissue, group of cells, or cell. Thereby said correspondent tissue, group of cells, or cell is derived from a healthy individual or is completely free of CD8 T-lymphocytes.

### CD4+ lymphocytes

In a preferred embodiment, the herein provided markers of Table 8A specific only for CD4 T-lymphocytes are used for quantifying CD4 T-lymphocytes, in particular for monitoring the immune system of individuals infected with HIV. The periodically determining of the number of CD4 T-lymphocytes for patients infected with HIV is a standard procedure in the art. It is necessary to decide whether and when a drug or treatment is necessary, whether a drug or treatment is still effective, and which drug or treatment can be selected. The said is necessary with respect to the HIV infection itself but also with respect to secondary infection. For example but not limited to, the differential methylation of SEQ ID NO: 652 (no gene associated) is used. According to Table 6, the differential methylation of SEQ ID NO: 652 is a marker for CD4 T-lymphocytes because the CpG dinucleotides of SEQ ID NO: 652 are methylated within the range of 25-75% in CD4 T-lymphocytes while other tissues show a different extend of methylation. For example but not limited to, the quantification of CD4 T-lymphocytes is carried out as the above described methods for quantifying CD8 T-lymphocytes wherein the marker RP4-695O20_B.9 SEQ ID NO: 706 is substituted by SEQ ID NO: 652. Of course, corresponding markers can also be alternatively used. Thereby corresponding markers of SEQ ID NO: 652 are, for example but not limited to, genomic DNA derived from or associated with SEQ ID NO: 652; methylation specifically converted DNA derived from SEQ ID NO: 652; mRNA, cDNA, protein, or peptide each of which derived at least in parts from SEQ ID NO: 652. If the case may be, a person skilled in the art knows how to adjust the presented procedures.

All methods named in the above embodiments are known in the art. They are described for example in EP06075376.1 or in PCT/US2006/014667 both of which is hereby incorporated by reference.

While the present invention has been described with specificity in accordance with certain of its preferred embodiments, the following EXAMPLES and TABLES serve only to illustrate the invention and are not intended to limit the invention within the principles and scope of the broadest interpretations and equivalent configurations thereof.

### TABLES

**Table 1. Overview of the genes or genomic regions according ot the present invention, with HUGO ID and the SEQ ID NO.**

| **Gene** | **HUGO ID** | **SEQ ID NO: Genomic** |
|---|---|---|
| VARS, valyl-tRNA synthetase, ENSG00000096171 | VARS | 415 |
| major histocompatibility complex, class II, DP beta 1, OTTHUMG00000031076, HLA-DPB1 | HLA-DPB1 | 416 |
| PRAME, Melanoma antigen preferentially expressed in tumors (Preferentially expressed antigen of melanoma) (OPA-interacting protein 4) (OIP4),ENSG00000185686 | PRAME | 419 |
| ZNRF3 protein (Fragment), ENSG00000183579, ZNRF3 zinc and ring finger 3 (ZNRF3) | ZNRF3 | 420 |
| Myosin-18B (Myosin XVIIIb), ENSG00000133454, MYO18B | MYO18B | 424 |
| FBLN1; fibulin 1; ENSG00000077942 | FBLN1 | 426 |
| no gene associated | | 430 |
| GPR24; G protein-coupled receptor 24; ENSG00000128285 | GPR24 | 436 |
| TCN2; transcobalamin II; macrocytic anemia; ENSG00000185339 | TCN2 | 470 |
| APOBEC3B; apolipoprotein B mRNA editing enzyme, catalytic polypeptide-like 3B; ENSG00000179750 | APOBEC3B | 474 |
| CRYBA4; crystallin, beta A4; ENSG00000196431 | CRYBA4 | 476 |
| Caspase recruitment domain protein 10 (CARD-containing MAGUK protein 3) (Carma 3).ENSG00000100065, CARD10 | CARD10 | 498 |
| HTF9C; Hpall tiny fragments locus 9C; ENSG00000099899 | HTF9C_HUMAN (UniProt/Swiss-Prot ID) | 500 |
| transmembrane protease, serine 6 | TMPRSS6 | 505 |
| Platelet-derived growth factor B chain precursor (PDGF B- chain), ENSG00000100311 , PDGFB | PDGFB | 508 |
| OTTHUMG00000030815, CTA-384D8.15 | CTA-384D8.15 (Vega gene ID) | 509 |
| RPL3; ribosomal protein L3; ENSG00000100316 | RPL3 | 529 |
| AIM1; absent in melanoma1; ENSG00000112297 | AlM1 | 538 |
| Disheveled associated activator of morphogenesis 2, ENSG00000146122, DAAM2 | DAAM2 | 540 |
| Nesprin-1 (Nuclear envelope spectrin repeat protein 1) (Synaptic nuclear envelope protein 1) (Syne-1) (Myocyte nuclear envelope protein 1) (Myne-1) (Enaptin), ENSG00000131018, SYNE1 | SYNE1 | 558 |
| cytidine monophosphate-N-acetylneuraminic acid hydroxylase (CMP-N-acetylneuraminate monooxygenase); CMAH | CMAH | 570 |
| glutathione peroxidase 5, OTTHUMG00000016307, GPX5 | GPX5 | 574 |
| forkhead box C1, OTTHUMG00000016182, FOXC1 | FOXC1 | 579 |
| KAAG1; kidney associated antigen 1; ENSG00000146049 | KAAG1 | 591 |
| no gene associated | | 615 |
| no gene associated | | 634 |
| no gene associated | | 637 |
| no gene associated | | 640 |
| no gene associated | | 644 |
| no gene associated | | 648 |
| PTPNS1; protein tyrosine phosphatase, non-receptor type substrate 1; ENSG00000198053 | PTPNS1 | 649 |
| ENSG00000149346, NP _001009608.1, hypothetical protein LOC128710, chromosome 20 open reading frame 94 | C20orf94 | 652 |
| EYA2; eyes absent homolog 2 (Drosophila); ENSG00000064655 | EYA2 | 678 |
| Bone morphogenetic protein 7 precursor (BMP-7) (Osteogenic protein 1) (OP-1) (Eptotermin alfa), ENSG00000101144, BMP7 | BMP7 | 684 |
| NP_612444.1 (RefSeq peptide ID); ENSG00000133477 | NP_612444.1 (RefSeq peptide ID) | 689 |
| TBC1D10A; TBC1 domain family, member 10A; ENSG00000099992 | TBC1 D10A | 700 |
| RP4-695O20_B.9 (Vega gene ID); Putative Processed transcript; OTTHUMG00000030111 | RP4-695O20_B.9 (Vega gene ID) | 706 |
| glycoprotein Ib (platelet), beta polypeptide | GP1 BB | 720 |
| solute carrier family 35, member E4 | SLC35E4 | 751 |
| | No gene associated | 753 |
| | No gene associated | 773 |

**Table 2. Overview of SEQ ID NO of the genes or genomic regions and the SEQ ID NOs of corresponding bisulfite treated nucleic acids (DNA upmethylated sense strand, DNA upmethylated antisense strand, DNA downmethylated sense strand, DNA downmethylated antisense strand).**

| **SEQ ID NO:** | **SEQ ID NO** | **SEQ ID NO:** | **SEQ ID NO:** | **SEQ ID NO:** |
|---|---|---|---|---|
| **Genomic** | **Upmethylated Sense** | **Upmethylated Antisense** | **Downmethylated Sense** | **Downmethylated Antisense** |
| 415 | 1653 | 1654 | 3301 | 3302 |
| 416 | 1655 | 1656 | 3303 | 3304 |
| 419 | 1661 | 1662 | 3309 | 3310 |
| 420 | 1663 | 1664 | 3311 | 3312 |
| 424 | 1671 | 1672 | 3319 | 3320 |
| 426 | 1675 | 1676 | 3323 | 3324 |
| 430 | 1683 | 1684 | 3331 | 3332 |
| 436 | 1695 | 1696 | 3343 | 3344 |
| 470 | 1763 | 1764 | 3411 | 3412 |
| 474 | 1771 | 1772 | 3419 | 3420 |
| 476 | 1775 | 1776 | 3423 | 3424 |
| 498 | 1819 | 1820 | 3467 | 3468 |
| 500 | 1823 | 1824 | 3471 | 3472 |
| 505 | 1833 | 1834 | 3481 | 3482 |
| 508 | 1839 | 1840 | 3487 | 3488 |
| 509 | 1841 | 1842 | 3489 | 3490 |
| 529 | 1881 | 1882 | 3529 | 3530 |
| 538 | 1899 | 1900 | 3547 | 3548 |
| 540 | 1903 | 1904 | 3551 | 3552 |
| 558 | 1939 | 1940 | 3587 | 3588 |
| 570 | 1963 | 1964 | 3611 | 3612 |
| 574 | 1971 | 1972 | 3619 | 3620 |
| 579 | 1981 | 1982 | 3629 | 3630 |
| 591 | 2005 | 2006 | 3653 | 3654 |
| 615 | 2053 | 2054 | 3701 | 3702 |
| 634 | 2091 | 2092 | 3739 | 3740 |
| 637 | 2097 | 2098 | 3745 | 3746 |
| 640 | 2103 | 2104 | 3751 | 3752 |
| 644 | 2111 | 2112 | 3759 | 3760 |
| 648 | 2119 | 2120 | 3767 | 3768 |
| 649 | 2121 | 2122 | 3769 | 3770 |
| 652 | 2127 | 2128 | 3775 | 3776 |
| 678 | 2179 | 2180 | 3827 | 3828 |
| 684 | 2191 | 2192 | 3839 | 3840 |
| 689 | 2201 | 2202 | 3849 | 3850 |
| 700 | 2223 | 2224 | 3871 | 3872 |
| 706 | 2235 | 2236 | 3883 | 3884 |
| 720 | 2263 | 2264 | 3911 | 3912 |
| 751 | 2325 | 2326 | 3973 | 3974 |
| 753 | 2329 | 2330 | 3977 | 3978 |
| 773 | 2369 | 2370 | 4017 | 4018 |

**Table 3. Overview of SEQ ID NO of amplificates derived from the genes or genomic regions and the SEQ ID NOs of corresponding bisulfite treated nucleic acids (DNA upmethylated sense strand, DNA upmethylated antisense strand, DNA downmethylated sense strand, DNA downmethylated antisense strand).**

| **SEQ ID NO:** | **SEQ ID NO:** | **SEQ ID NO:** | **SEQ ID NO:** | **SEQ ID NO:** |
|---|---|---|---|---|
| **Genomic** | **Upmethylated Sense** | **Upmethylated Antisense** | **Downmethylated Sense** | **Downmethylated Antisense** |
| 3 | 829 | 830 | 2477 | 2478 |
| 4 | 831 | 832 | 2479 | 2480 |
| 7 | 837 | 838 | 2485 | 2486 |
| 8 | 839 | 840 | 2487 | 2488 |
| 12 | 847 | 848 | 2495 | 2496 |
| 14 | 851 | 852 | 2499 | 2500 |
| 18 | 859 | 860 | 2507 | 2508 |
| 24 | 871 | 872 | 2519 | 2520 |
| 58 | 939 | 940 | 2587 | 2588 |
| 62 | 947 | 948 | 2595 | 2596 |
| 64 | 951 | 952 | 2599 | 2600 |
| 86 | 995 | 996 | 2643 | 2644 |
| 88 | 999 | 1000 | 2647 | 2648 |
| 93 | 1009 | 1010 | 2657 | 2658 |
| 96 | 1015 | 1016 | 2663 | 2664 |
| 97 | 1017 | 1018 | 2665 | 2666 |
| 117 | 1057 | 1058 | 2705 | 2706 |
| 126 | 1075 | 1076 | 2723 | 2724 |
| 128 | 1079 | 1080 | 2727 | 2728 |
| 146 | 1115 | 1116 | 2763 | 2764 |
| 158 | 1139 | 1140 | 2787 | 2788 |
| 162 | 1147 | 1148 | 2795 | 2796 |
| 167 | 1157 | 1158 | 2805 | 2806 |
| 179 | 1181 | 1182 | 2829 | 2830 |
| 203 | 1229 | 1230 | 2877 | 2878 |
| 222 | 1267 | 1268 | 2915 | 2916 |
| 225 | 1273 | 1274 | 2921 | 2922 |
| 228 | 1279 | 1280 | 2927 | 2928 |
| 232 | 1287 | 1288 | 2935 | 2936 |
| 236 | 1295 | 1296 | 2943 | 2944 |
| 237 | 1297 | 1298 | 2945 | 2946 |
| 240 | 1303 | 1304 | 2951 | 2952 |
| 266 | 1355 | 1356 | 3003 | 3004 |
| 272 | 1367 | 1368 | 3015 | 3016 |
| 277 | 1377 | 1378 | 3025 | 3026 |
| 288 | 1399 | 1400 | 3047 | 3048 |
| 294 | 1411 | 1412 | 3059 | 3060 |
| 308 | 1439 | 1440 | 3087 | 3088 |
| 339 | 1501 | 1502 | 3149 | 3150 |
| 341 | 1505 | 1506 | 3153 | 3154 |
| 361 | 1545 | 1546 | 3193 | 3194 |

**Table 4. Overview of SEQ ID NO of the genes or genomic regions and of corresponding amplificates amplified by use of the corresponding Forward and Reverse Primer named by their SEQ ID NO.**

| **SEQ ID NO: Genomic Forward** | **Primer SEQ ID NO:** | **Reverse Primer SEQ ID NO:** | **SEQ ID NO: Genomic** |
|---|---|---|---|
| 415 | 4125 | 4126 | 3 |
| 416 | 4127 | 4128 | 4 |
| 419 | 4133 | 4134 | 7 |
| 420 | 4135 | 4136 | 8 |
| 424 | 4143 | 4144 | 12 |
| 426 | 4147 | 4148 | 14 |
| 430 | 4155 | 4156 | 18 |
| 436 | 4167 | 4168 | 24 |
| 470 | 4235 | 4236 | 58 |
| 474 | 4243 | 4244 | 62 |
| 476 | 4247 | 4248 | 64 |
| 498 | 4291 | 4292 | 86 |
| 500 | 4295 | 4296 | 88 |
| 505 | 4305 | 4306 | 93 |
| 508 | 4311 | 4312 | 96 |
| 509 | 4313 | 4314 | 97 |
| 529 | 4353 | 4354 | 117 |
| 538 | 4371 | 4372 | 126 |
| 540 | 4375 | 4376 | 128 |
| 558 | 4411 | 4412 | 146 |
| 570 | 4435 | 4436 | 158 |
| 574 | 4443 | 4444 | 162 |
| 579 | 4453 | 4454 | 167 |
| 591 | 4477 | 4478 | 179 |
| 615 | 4525 | 4526 | 203 |
| 634 | 4563 | 4564 | 222 |
| 637 | 4569 | 4570 | 225 |
| 640 | 4575 | 4576 | 228 |
| 644 | 4583 | 4584 | 232 |
| 648 | 4591 | 4592 | 236 |
| 649 | 4593 | 4594 | 237 |
| 652 | 4599 | 4600 | 240 |
| 678 | 4651 | 4652 | 266 |
| 684 | 4663 | 4664 | 272 |
| 689 | 4673 | 4674 | 277 |
| 700 | 4695 | 4696 | 288 |
| 706 | 4707 | 4708 | 294 |
| 720 | 4735 | 4736 | 308 |
| 751 | 4797 | 4798 | 339 |
| 753 | 4801 | 4802 | 341 |
| 773 | 4841 | 4842 | 361 |

**Table 5. Overview of genes or genomic regions and the corresponding SEQ ID NOs, HUGO IDs, Aliases, Reference Sequence IDs, Ensemble Gene IDs and Entrez IDs.**

| **SEQ NO: Genomic** | **IDGene** | **HUGO ID** | **Aliases** | **RefSeq ID** | **Ensembl Gene ID** | **Entrez Gene ID** |
|---|---|---|---|---|---|---|
| 415 | VARS, valyl-tRNA synthetase, ENSG00000096171 | VARS | G7A; VARS2 | NM_006295 | ENSG00000096171 | 7407 |
| 416 | major histocompatibility complex, class II, DP beta 1, OTTHUMG00000031076, HLA-DPB1 | HLA-DPB1 | DPB1; DP1B; MHC DPB1 | HLA-NM_002121 | OTTHUMG00000031 076 | 3115 |
| 419 | PRAME, Melanoma antigen preferentially expressed in tumors (Preferentially expressed antigen of melanoma) (OPA-interacting protein 4) (OIP4), ENSG00000185686 | PRAME | MAPE; OIP4 | NG_000002 | ENSG00000185686 | 23532 |
| 420 | ZNRF3 protein (Fragment), ENSG00000183579, ZNRF3 zinc and ring finger 3 (ZNRF3) | ZNRF3 | KIAA1133, BK747E2.3, FLJ22057, RNF203 | XM_290972, XP_290972 | ENSG00000183579 | 84133 |
| 424 | Myosin-18B (Myosin XVIIIb), ENSG00000133454 , MYO18B | MYO18B | BK125H2.1 | NM_014550 | ENSG00000133454 | 84700 |
| 426 | FBLN1; fibulin 1; ENSG00000077942 | FBLN1 | FBLN | NM_001996 | ENSG00000077942 | 2192 |
| 430 | no gene associated | | | | | |
| 436 | GPR24; G protein-coupled receptor 24; ENSG00000128285 | GPR24 | SLC1; MCHR1; MGC32129 | NM_005297 | ENSG00000128285 | 2847 |
| 470 | TCN2; transcobalamin II; macrocytic anemia; ENSG00000185339 | TCN2 | TC2; D22S676; D22S750 | NM_000355 | ENSG00000185339 | 6948 |
| 474 | APOBEC3B; apolipoprotein B mRNA editing enzyme, catalytic polypeptide-like 3B; ENSG00000179750 | APOBEC3B | ARP4; ARCD3; PHRBNL; APOBEC1L; FLJ21201; DJ742C19.2 | NM_004900 | ENSG00000179750 | 9582 |
| 476 | CRYBA4; crystallin, beta A4; ENSG00000196431 | CRYBA4 | | NM_001886 | ENSG00000196431 | 1413 |
| 498 | Caspase recruitment domain protein 10 (CARD-containing MAGUK protein 3) (Carma 3). ENSG00000100065, CARD10 | CARD10 | CARMA3, BIMP1 | NM_014550 | ENSG00000100065 | 29775 |
| 500 | HTF9C; Hpall tiny fragments HTF9C_HUM locus 9C; ENSG00000099899AN | (UniProt/Swiss -Prot ID) | HTF9C; MGC102728 | NM_022727 | ENSG00000099899 | 27037 |
| 505 | transmembrane protease, serine 6 | TMPRSS6 | | NM_153609 | ENSG00000187045 | 164656 |
| 508 | Platelet-derived growth factor B chain precursor (PDGF B-chain, ENSG00000100311 , PDGFB | PDGFB | SIS; SSV; PDGF2; c-sis | NM_002608 | ENSG00000100311 | 5155 |
| 509 | OTTHUMG00000030815, CTA-384D8.15 | CTA-384D8.15 (Vega gene ID) | Em:U62317.C 22.15 | NoAvailable | OTTHUMG00000030 815 | NoAvailable |
| 529 | RPL3; ribosomal protein L3; ENSG00000100316 | RPL3 | TARBP-B; MGC104284 | NM_000967 | ENSG00000100316 | 6122 |
| 538 | AIM1; absent in melanoma1; ENSG00000112297 | AlM1 | ST4 | NM_001624 | ENSG00000112297 | 202 |
| 540 | Disheveled associated activator of morphogenesis 2, ENSG00000146122, DAAM2 | DAAM2 | KIAA0381; MGC90515; dJ90A20A.1; RP1-278E11.1 | NM_015345 | ENSG00000146122 | 23500 |
| 558 | Nesprin-1 (Nuclear envelope spectrin repeat protein 1) (Synaptic nuclear envelope protein 1) (Syne-1) (Myocyte nuclear envelope protein 1) (Myne-1) (Enaptin), ENSG00000131018, SYNE1 | SYNE1 | SYNE-1 B, KIAA0796, 8B, nesprin-1, enaptin, MYNE1, CPG2 | N_015293 | ENSG00000131018 | 23345 |
| 570 | cytidine monophosphate-N-acetylneuraminic acid hydroxylase (CMP-N-acetylneuraminate monooxygenase); CMAH | CMAH | CSAH; NeuAc hydroxylase; CMP-Neu5Ac hydroxylase; CMP-sialic acid hydroxylase; CMP-N-acetylneurami nic acid hydroxylase; cytidine monophospho -N-acetylneurami nic acid hydroxylase | CMP-NR_002174 | OTTHUMG00000016 099 | 8418 |
| 574 | glutathione peroxidase 5, OTTHUMG00000016307, GPX5 | GPX5 | | NM_001509 | OTTHUMG00000016 307 | 2880 |
| 579 | forkhead box C1, OTTHUMG00000016182, FOXC1 | FOXC1 | FKHL7, FREAC3, ARA, IGDA, IHG1 | IRID1, NM_001453 | OTTHUMG00000016 182 | 2296 |
| 591 | KAAG1; kidney associated antigen 1; ENSG00000146049 | KAAG1 | RU2; MGC78738 | RU2AS; NM_181337 | ENSG00000146049 | 353219 |
| 615 | no gene associated | | | | | |
| 634 | no gene associated | | | | | |
| 637 | no gene associated | | | | | |
| 640 | no gene associated | | | | | |
| 644 | no gene associated | | | | | |
| 648 | no gene associated | | | | | |
| 649 | PTPNS1; protein tyrosine phosphatase, non-receptor type substrate 1; ENSG00000198053 | PTPNS1 | BIT; P84; SIRP; MYD-1; SHPS1; SHPS-1; SIRPalpha; SIRPalpha2; SIRP-ALPHA-1 | MFR; NM_080792 | ENSG00000198053 | 140885 |
| 652 | ENSG00000149346, NP_001009608.1, hypothetical protein LOC128710, chromosome 20 open reading frame 94 | C20orf94 | NP_00100960 8.1, bA204H22.1; bA254M13.1; dJ1099D15.3 | NM_00100960 8 | ENSG00000149346 | 128710 |
| 678 | EYA2; eyes absent homolog 2 (Drosophila); ENSG00000064655 | EYA2 | EAB1; MGC10614 | NM_005244 | ENSG00000064655 | 2139 |
| 684 | Bone morphogenetic protein precursor (BMP-7) (Osteogenic protein 1) (OP-1) (Eptotermin alfa), ENSG00000101144 , BMP7 | 7 BMP7 | OP-1 | NM_001719 | ENSG00000101144 | 655 |
| 689 | NP_612444.1 (RefSeq peptide ID); ENSG00000133477 | NP_612444.1 (RefSeq peptide ID) | | NoAvailable | ENSG00000133477 | NoAvailable |
| 700 | TBC1D10A; TBC1 domain family, member 10A; ENSG00000099992 | TBC1D10A | EP164; TBC1D10; dJ130H16.1; AC004997.C2 2.2 | NM_031937 | ENSG00000099992 | 83874 |
| 706 | RP4-695020_B.9 (Vega gene ID); Putative Processed transcript; OTTHUMG00000030111 | RP4-695020_B.9 (Vega gene ID) | dJ695O20B.C 22.9 | NoAvailable | OTTHUMG00000030 111 | NoAvailable |
| 720 | glycoprotein Ib (platelet), beta polypeptide | GP1BB | CD42c | NM_000407 | OTTHUMG00000030 191 | 2812 |
| 751 | solute carrier family 35, member E4 | SLC35E4 | | 9.1 | NM_00100147 ENSG00000100036 | 339665 |
| 753 | | No gene associated | | | | |
| 773 | | No gene associated | | | | |

**Table 6. Overview of the genomic regions and ranges of methylation within which the actual value of methylation of said genomic regions lies specific of the said cells, tissues and/or organs. The actual value of methylation is shown in Figures 1.1-1.403.**

| **SEQ ID Genomic** | **NO: lymphocyte** | **CD4 T-CD8 T-Emlymphocyte** | **bryonic Liver** | **Embryonic Skelet -al Muscl e** | **Fibroblast** | **Heart Muscl e** | **Keratinocyte** | **Liver** | **Melanocyte centa** | **Pla-** | **Skeletal Muscl e** | **Sperm** |
|---|---|---|---|---|---|---|---|---|---|---|---|---|
| 415 | 75-100% | 75-100% | 75-100% | 75-100% | 75-100% | 75-100% | 75-100% | 25-75% | 75-100% | 75-100% | 75-100% | 75-100% |
| 416 | 100% | 100% | 0-25% | 0-25% | 0-25% | 0-25% | 0-25% | 0-25% | 0-25% | 0-25% | 0-25% | 0-25% |
| 419 | 75-100% | 75-100% | 75-100% | 75-100% | 75-100% | 75-100% | 75-100% | 75-100% | 75-100% | 0-25% 75- | 75-100% | 0-25% |
| 420 | 75-100% | 75-100% | 75-100% | 0-25% | 100% | 75-100% | 75-100% | 75-100% | 75-100% | 75-100% | 0-25% 75- | 75-100% |
| 424 | 75-100% | 75-100% | 75-100% | 25-75% | 75-100% | 25-75% | 75-100% | 75-100% | 75-100% | 25-75% | 25-75% | 75-100% |
| 426 | 25-75% | 25-75% | 0-25% | 0-25% | 0-25% | 0-25% | 0-25% | 0-25% | 0-25% | 0-25% | 0-25% | 0-25% |
| 430 | 75-100% | 75-100% | 75-100% | 75-100% | 75-100% | 25-75% | 75-100% | 75-100% | 75-100% | 75-100% | 75-100% | 75-100% |
| 436 | 75-100% | 75-100% | 75-100% | 75-100% | 75-100% | 75-100% | 75-100% | 75-100% | 75-100% | 25-75% | 75-100% | 75-100% |
| 470 | 0-25% | 0-25% | 0-25% | 0-25% | 0-25% | 0-25% | 0-25% | 0-25% | 0-25% | 0-25% | 0-25% | 75 100% |
| 474 | 0-25% | 0-25% | 75-100% | 75-100% | 75-100% | 75-100% | 75-100% | 75-100% | 75-100% | 75-100% | 75-100% | 75-100% |
| 476 | 75-100% | 75-100% | 25-75% | 25-75% | 75-100% | 75-100% | 75-100% | 75-100% | 75-100% | 75-100% | 75-100% | 75-100% |
| 498 | 75-100% | 75-100% | 75-100% | 75-100% | 75-100% | 75-100% | 75-100% | 75-100% | 75-100% | 75-100% | 0-25% 75 | 100% |
| 500 | 75-100% | 75-100% | 75-100% | 75-100% | 75-100% | 75-100% | 75-100% | 75-100% | 75-100% | 75-100% | 25-75% | 75-100% |
| 505 | 0-25% | 0-25% | 75-100% | 0-25% | 0-25% | 0-25% | 0-25% | 75-100% | 0-25% | 0-25% | 0-25% | ND |
| 508 | 0-25% | 0-25% | 0-25% | 0-25% | 0-25% | 0-25% | 0-25% | 75-100% | 0-25% | 0-25% | 0-25% | ND |
| 509 | 75-100% | 75-100% | 25-75% | 75-100% | 75-100% | 75-100% | 75-100% | 75-100% | 75-100% | 75-100% | 75-100% | 0-25% |
| 529 | 75-100% | 75-100% | 75-100% | 75-100% | 75-100% | 75-100% | 75-100% | 25-75% | 75-100% | 75-100% | 75-100% | 75-100% |
| 538 | 0-25% | 0-25% | 0-25% | 0-25% | 0-25% | 0-25% | 0-25% | 0-25% | 0-25% | 25-75% | 0-25% | 0-25% |
| 540 | 75-100% | 75-100% | 75-100% | 75-100% | 75-100% | 75-100% | 75-100% | 0-25% 75- | 100% | 75-100% | 75-100% | 75-100% |
| 558 | 75- 100% | 75- 100% | 0-25% | 0-25% | 0-25% | 0-25% | 0-25% | 0-25% | 0-25% | 0-25% | 0-25% | 75- 100% |
| 570 | 75-100% | 75-100% | 75-100% | 75-100% | 75-100% | 75-100% | 0-25% 75- | 100% | 75-100% | 75-100% | 75-100% | 0-25% |
| 574 | 0-25% | 0-25% | 75-100% | 75-100% | 75-100% | 75-100% | 75-100% | 75-100% | 75-100% | 75-100% | 75-100% | 75-100% |
| 579 | 0-25% | 0-25% | 0-25% | 0-25% | 0-25% | 25 75% | 0-25% | 0-25% | 0-25% | 0-25% | 0-25% | 0-25% |
| 591 | 0-25% | 0-25% | 0-25% | 0-25% | 0-25% | 0-25% | 0-25% | 75 100% | 0-25% | 0-25% | 0-25% | 0-25% |
| 615 | 75-100% | 75-100% | 75-100% | 75-100% | 75-100% | 25-75% | 75-100% | 75-100% | 75-100% | 75-100% | 75-100% | 75-100% |
| 634 | 75-100% | 25-75% | 25-75% | 25-75% | 25-75% | 25-75% | 25-75% | 25-75% | 25-75% | 25-75% | 25-75% | 75-100% |
| 637 | 25-75% | 0-25% | 0-25% | 0-25% | 0-25% | 0-25% | 0-25% | 0-25% | 0-25% | 0-25% | 0-25% | 75-100% |
| 640 | 75-100% | 75-100% | 75-100% | 75-100% | 25-75% | 75-100% | 25-75% | 75-100% | 25-75% | 75-100% | 75-100% | 75-100% |
| 644 | 75-100% | 75-100% | 75-100% | 75-100% | 75-100% | 75-100% | 75- 100% | 75-100% 0-25% | 25- | 75% | 25-75% | 75-100% |
| 648 | 25-75% | 25-75% | 75-100% | 75-100% | 0-25% | 75-100% | 75-100% | 75-100% | 75-100% | 75-100% | 75-100% | 75-100% |
| 649 | 75-100% | 75-100% | 75-100% | 75-100% | 75-100% | 75-100% | 0-25% | 75-100% | 75-100% | 75-100% | 75-100% | |
| 652 | 25-75% | 75-100% | 75-100% | 75-100% | 75-100% | 75-100% | 75-100% | 75-100% | 75-100% | 75-100% | 75-100% | 75-100% |
| 678 | 75-100% | 75-100% | 75-100% | 25-75% | 75-100% | 75-100% | 75-100% | 75-100% | 75-100% | 75-100% | 25-75% | 75-100% |
| 684 | 0-25% | 0-25% | 0-25% | 0-25% | 0-25% | 0-25% | 0-25% | 25-75% | 0-25% | 0-25% | 0-25% | 0-25% |
| 689 | 75-100% | 75-100% | 75-100% | 75-100% | 75-100% | 75-100% | 0-25% | 75-100% | 75-100% | 75-100% | 75-100% | 0-25% |
| 700 | 0-25% | 0-25% | 0-25% | 0-25% | 0-25% | 75-100% | 0-25% | 25- 75% | 0-25% | 0-25% | 25-75% | 75-100% |
| 706 | 25-75% | 75-100% | 25-75% | 25-75% | 25-75% | 25-75% | 25-75% | 75-100% | 25-75% | 25-75% | 25-75% | 75-100% |
| ND = not determined | | | | | | | | | | | | |

**Table 7. Overview of the genomic regions and ranges of methylation within which the actual value of methylation of said genomic regions lies specific of the said cells, tissues and/or organs. The actual value of methylation is shown in Figures 1.1-1.403.**

| | | | | | | | | | | | | |
|---|---|---|---|---|---|---|---|---|---|---|---|---|
| **SEQ ID NO: lym-Genomic** | **phocyte** | **CD4 T-CD8 T-Emlymphocyte** | **bryonic Liver** | **Embryonic Skelet -al Muscl e** | **Fibroblast** | **Heart Muscl e** | **Keratinocyte** | **Liver** | **Melanocyte centa** | **Pla-** | **Skeletal Muscl e** | **Sperm** |
| 720 | 0-25% | 0-25% | 0-25% | 75-100% | 75-100% | 0-25% | 0-25% | 0-25% | 0-25% | 75-100% | 75-100% | 0-25% |
| 751 | 75-100% | 75-100% | 75-100% | 75-100% | 0-25% | 75-100% | 0-25% | 75-100% | 0-25% | 75-100% | 0-25% | 0-25% |
| 753 | 75-100% | 75-100% | 75-100% | ND | 0-25% | 75-100% | 75-100% | 75-100% | 75-100% | 0-25% | 0-25% | 75-100% |
| 773 | 75-100% | 75-100% | 75-100% | 75-100% | 0-25% | 75-100% | 0-25% | 75-100% | 0-25% | 75-100% | 75-100% | 75-100% |
| ND = not determined | | | | | | | | | | | | |

**Table 8A. Sequences for the differentiation and/or detection of T-lymphocytes.**

| **SEQ ID NO: Genomic** | **Tissue** |
|---|---|
| 634 | CD4 T-lymphocyte |
| 637 | CD4 T-lymphocyte |
| 652 | CD4 T-lymphocyte |
| 416 | CD4 T-lymphocyte, CD8 T-lymphocyte |
| 426 | CD4 T-lymphocyte, CD8 T-lymphocyte |
| 474 | CD4 T-lymphocyte, CD8 T-lymphocyte |
| 558 | CD4 T-lymphocyte, CD8 T-lymphocyte |
| 574 | CD4 T-lymphocyte, CD8 T-lymphocyte |
| 706 | CD8 T-lymphocyte |

**Table 8B. Sequences for the differentiation and/or detection of embryonic liver.**

| **SEQ ID NO: Genomic** | **Tissue** |
|---|---|
| 509 | embryonic liver |
| 476 | embryonic liver |
| 420 | embryonic liver |
| 678 | embryonic liver |
| 505 | embryonic liver |

**Table 8C. Sequences for the differentiation and/or detection of embryonic skeletal muscle.**

| **SEQ ID NO: Genomic** | **Tissue** |
|---|---|
| 476 | embryonic skeletal muscle |
| 420 | embryonic skeletal muscle |
| 678 | embryonic skeletal muscle |

**Table 8D. Sequences for the differentiation and/or detection of fibroblasts:**

| **SEQ ID NO: Genomic** | **Tissue** |
|---|---|
| 648 | fibroblast |
| 640 | fibroblast |

**Table 8E. Sequences for the differentiation and/or detection of heart muscle.**

| **SEQ ID NO: Genomic** | **Tissue** |
|---|---|
| 430 | heart muscle |
| 579 | heart muscle |
| 615 | heart muscle |
| 700 | heart muscle |
| 424 | heart muscle |

**Table 8G. Sequences for the differentiation and/or detection of keratinocytes.**

| **SEQ ID NO: Genomic** | **Tissue** |
|---|---|
| 570 | keratinocyte |
| 649 | keratinocyte |
| 689 | keratinocyte |
| 640 | keratinocyte |

**Table 8H. Sequences for the differentiation and/or detection of liver.**

| **SEQ ID NO: Genomic** | **Tissue** |
|---|---|
| 415 | liver |
| 508 | liver |
| 529 | liver |
| 540 | liver |
| 591 | liver |
| 684 | liver |
| 505 | liver |

**Table 81. Sequences for the differentiation and/or detection of melanocytes.**

| **SEQ ID NO: Genomic** | **Tissue** |
|---|---|
| 644 | melanocyte |
| 640 | melanocyte |

**Table 8J. Sequences for the differentiation and/or detection of placenta.**

| **SEQ ID NO: Genomic Tissue** | |
|---|---|
| 436 | placenta |
| 538 | placenta |
| 419 | placenta |

**Table 8K. Sequences for the differentiation and/or detection of skeletal muscle.**

| **SEQ ID NO: Genomic** | **Tissue** |
|---|---|
| 498 | skeletal muscle |
| 500 | skeletal muscle |
| 424 | skeletal muscle |
| 420 | skeletal muscle |
| 678 | skeletal muscle |

**Table 8L. Sequences for the differentiation and/or detection of sperm.**

| **SEQ ID NO: Genomic** | **Tissue** |
|---|---|
| 470 | sperm |

**Table 9D. Sequences for the differentiation and/or detection of fibroblasts.**

| **SEQ ID NO: Genomic** | **Tissue** |
|---|---|
| 720 | fibroblast |
| 753 | fibroblast |
| 751 | fibroblast |
| 773 | fibroblast |

**Table 9F. Sequences for the differentiation and/or detection of keratinocytes.**

| **SEQ ID NO: Genomic** | **Tissue** |
|---|---|
| 751 | keratinocyte |
| 773 | keratinocyte |

**Table 9H. Sequences for the differentiation and/or detection of melanocytes**

| **SEQ ID NO: Genomic** | **Tissue** |
|---|---|
| 751 | melanocyte |
| 773 | melanocyte |

### EXAMPLES

### Example 1

More than 300 specific regions of differential methylation have been identified in primary cells and tissues. Some of them are located in known promoter regions, while others are located in intra- or intergenic regions throughout the chromosomes 20, 22 and 6.

### a) Materials and Methods

Samples:
Studied primary cell cultures included lymphocytes (selected and sorted by CD4 and CD8 antigen expression), melanocytes, keratinocytes and fibroblasts. Cells were harvested and kept at -80 °C until RNA isolation. Isolated RNA samples from Heart, Liver and Skeletal Muscle were purchased from commercial suppliers (Ambion) and kept at-80°C until use in reverse transcription.

### Analyzed Genes

MYO18B gene was selected for the expression analysis considering the location of regions of differential methylation. In MYO18 the region of differential methylation located in the promoter region, see table below.

Total RNA Isolation and RT-PCR:
Total RNA was isolated from cells and tissues using RNeasy kits (Qiagen, Hilden, Germany).
Concentration and purity of the obtained RNA was determined spectrophotometrically, while the integrity was determined by electrophoresis on denaturing 1 M urea - 2 % agarose gel
cDNA was prepared using Omniscript RT kit (Qiagen, Hilden, Germany) with random hexamers in accordance to manufacturer's conditions. PCR was performed using 3 µl of the prepared cDNA, specific primers and HotStartTaq DNA polymerase kit in accordance with manufacturer's conditions. The PCR parameters used were: 15 min at 95 °C followed by 40 cycles of 1 min at 94°C, 1 min at the annealing temperature specific for each primer pair, and 1 min at 72 °C, ending with a final extension for 10 min at 72°C. Primers were designed to bind in successive exons in order to avoid amplification in case contaminating genomic DNA was present. Amplification conditions for each fragment were determined experimentally by amplifying cDNA produced from Universal Human RNA (BioCat, Heidelberg, Germany),
which is a pool total RNAs isolated from several tissues. The sequences of the specific primers for the analyzed gene is provided in the accompanying sequence listing according to the table below. It further shows the particular annealing temperature used in the amplification reaction. PCR products were separated through a 2.5 % Agarose gel.

### b) Results

The analyzed MYO18B gene showed a correlation between high methylation and gene silencing. In MYO18B the region of differential methylation was located in the promoter region. Table 10 shows the results for MYO18B gene. As an example the results for the gene MYO18B is shown in Figure 2. As a control the expression of SERPINB5 was also studied. Finally the expression of a housekeeping gene in the study sample demonstrated the feasibility of the assay and gave a baseline for the semiquantitative analysis of the expression.

**Table 10: Analyzed genes, primers and annealling temperatures and results.**

| Gene | Gene SEQ | Differential methylation | Forward Primer | Reverse Primer | Annealing | Correlation methylation/ |
|---|---|---|---|---|---|---|
| Name | ID NO: | region position | SEQ ID NO: | SEQ ID NO: | temperature | (°C)expression |
| MYO18B | 12 | Promoter | 4959 | 4960 | 60 | Inverse |
| SERPINB5 | | Promoter | 4965 | 4966 | 60 | Inverse (Positive control) |
| ACTB1 | | | 4967 | 4968 | 56 | No correlation (Negative control) |

### Example 2: Large-scale DNA methylation profiling of human chromosomes 6, 20 and 22.

Using bisulfite DNA sequencing, we report high-resolution methylation reference profiles of human chromosomes 6, 20 and 22, providing a resource of about 1.9 million CpG measurements in 43 samples derived from 12 different (healthy) tissues.

It was the aim of the study to establish DNA methylation reference profiles for three human chromosomes from a number of healthy (no known disease phenotype) human tissues and primary cells. The study was further controlled for age and sex and comprised the analysis of 43 different samples derived from sperm, various primary cell types (dermal fibroblasts, dermal keratinocytes, dermal melanocytes, CD4⁺ and CD8⁺ lymphocytes) and tissues (heart muscle, skeletal muscle, liver and placenta, table 9). Tissues were pooled from up to three age- and sex-matched individuals (see table 9 for details). Primary cells were cultured for no more than three passages to minimize the risk of introducing aberrant methylation. Additionally, the methylation of selected amplicons were compared before and after cultivation with no difference in average methylation being detected. As dermal fibroblasts, keratinocytes and melanocytes are the major cell types constituting the human epidermis, we compared the average methylation of selected amplicons in these cell types with the corresponding values derived from additional human skin samples. No significant deviation between the methylation of the primary cells and tissues were detected, indicating that cell culturing for a limited number of passages does not change DNA methylation.

In total, we analyzed 2,524 amplicons associated with 873 genes on chromosomes 6, 20 and 22. Based on Ensembl (NCBI34) annotation, the amplicons were assigned to 6 distinct categories. Taking the number of biological and technical replicates into account, we have determined the methylation status of 1.88 million CpG sites. The corresponding data have been deposited into the public HEP database and can be accessed at www.epigenome.org.

### Material & Methods

*Cell and Tissue samples:* Tissue samples were obtained from following sources: Asterand, (Detroit, US), Pathlore Plc. (Nottingham, UK), Tissue Transformation Technologies (T-cubed, Edison, US), Northwest Andrology (Missoula, US), NDRI (Philadelphia, US) and Biocat GmBH (Heidelberg, Germany). Only anonymized samples were used and ethical approval was obtained for the study. Contamination by blood cells is estimated to be low as blood specific methylation profiles were not detected in the tissues. Human primary cells were acquired from Cascade Biologics (Mansfield, United Kingdom), Cell Applications Inc. (San Diego, United States), Analytical Biological Services Inc. (Wilmington, US), Cambrex Bio Science (Verviers, Belgium) and from the DIGZ (Berlin, Germany). Dermal fibroblasts, keratinocytes and melanocytes were cultured according to the supplier's recommendations up to a maximum of 3 passages reducing the risk of aberrant methylation due to extended culturing. CD4⁺ T-lymphocytes were isolated from fresh whole blood by depletion of CD4⁺ monocytes followed by a negative selection. CD8⁺ cells were isolated from fresh whole blood by positive selection. Subsequent FACS analysis confirmed a purity of CD4⁺/CD8⁺ T-lymphocytes greater than 90%. In some cases, DNA samples were pooled according to the sex and age of the donors. All genders were confirmed by sex-specific PCR.

*Amplicon selection and classification:* Amplicons were selected and classified based on Ensembl (build NCBI 34) annotation, amplicons were designed in the following genomic regions:
5'-UTR: Overlapping by at least 200 bp with or within a core region of 2,000 bp upstream to 500 bp downstream of the TSS. Where multiple sites were annotated per gene, the first annotated TSS was used.
Exonic: Greater than 50% and at least 200 bp of amplicon overlapping with annotated exon. Intronic: Greater than 50% and at least 200 bp of amplicon overlapping with annotated intron. ECR evolutionary conserved regions): ≥70% DNA sequence similarity (including ≥4 CpGs) for at least 100 bp between human and mouse non-coding sequences. Out of 3,249 ECRs identified on chromosome 20, 290 intergenic and 206 intronic (496 in total) ECRs were selected.
Sp1: Overlapping with putative Sp1 sites identified by ChIP-chip analysis.
ncRNA: CD box snoRNAs as described by Lestrade, L. & Weber, M.J. snoRNA-LBME-db, a comprehensive database of human H/ACA and C/D box snoRNAs. Nucleic Acids Res. 34, 158-162 (2006) and miRNAs as reported by Griffiths-Jones, S. The microRNA Registry. Nucleic Acids Res. 32, 109-111 (2004) located on chromosome 22.
Other: amplicons that are not located within a gene or a 5'-UTR and additionally do not belong to any other category. CGI (CpG island) were classified based on the criteria by Gardiner-Garden and Frommer J. Mol. Biol. 196, 261-282 (1987).

*DNA extraction, PCR amplification and sequencing:* DNA was extracted using the Qiagen DNA Genomic-tip kit according the manufacturer's recommendation. After quantification, DNA was bisulfite converted as previously described in PCT/WO/2005/038051 (2005). Bisulfite-specific primers with a minimum length of 18bp were designed using a modified primer-3 program. The target sequence of the designed primers contained no CpGs allowing for an unbiased amplification of both hypo- and hypermethylated DNAs. All primers were tested for their ability to yield high quality sequences. Primers that gave rise to an amplicon of the expected size using non-bisulfite treated DNA as a template were discarded, thus ensuring the specificity for bisulphite-converted DNAs. Primers were also tested for specificity on bisulfite DNA by electronic PCR. DNA amplification was set up in 96-well plates using an automated pipeline. PCR amplicons were quality controlled by agarose gel electrophoresis, re-arrayed into 384-well plates for high-throughput processing, cleaned up using ExoSAP-IT (USB Corporation, Cleveland, Ohio) to remove any excess nucleotides and primers and sequenced directly in the forward and reverse directions. Sequencing was performed on ABI 3730 capillary sequencers using 1/32nd dilution of ABI Prism BigDye terminator V3.1 sequencing chemistry after hotstart (96°C for 30 seconds) thermocycling (92°C for 5 seconds, 50°C for 5 seconds, 60°C for 120 seconds x 44 cycles) and ethanol precipitation. PCR fragments were sequenced using the same PCR amplification primers. PCR primers for the differentially methylated amplificates are provided in Table 4. Trace files and methylation signals at a given CpG site were quantified using the software ESME as previously described in Bioinformatics 20, 3005-3012 (2004).

The bisulfite sequencing-based approach chosen here allows one to measure DNA methylation with high reproducibility and accuracy, as independent measurements are derived from both the sense and antisense strands of a PCR amplicon (R = 0.87; N = 557,837). In addition, about 4.1% of the amplicons were subjected to independent PCR amplification and sequencing. These technical replicates also displayed high correlation (R = 0.9; N = 15,655). Furthermore, the signal is independent of the position of the measured CpG within the amplicon, which is supported by high correlation between measurements of the same CpGs in overlapping amplicons (R = 0.85; N = 91,528).

*RNA extraction and RT-PCR:* Aliquots of the same samples of the human melanocytes, keratinocytes, fibroblasts, CD4⁺ and CD8⁺ cells that were used for methylation analysis were used for RNA analysis. Primary cell cultures (maximum of 3 passages) of human melanocytes, keratinocytes and dermal fibroblasts cells were harvested and kept at -80 °C until RNA isolation. Isolated RNA samples from heart, liver and skeletal muscle were purchased from Ambion (Austin, US) and kept at -80°C until used for reverse transcription. Total RNA was isolated using the RNeasy kit from Qiagen (Hilden, Germany) followed by cDNA synthesis using the Omniscript RT kit from the same supplier and random hexamers. PCR (92°C for 1 minute, 55-63°C (depending on assay) for 1 minute, 72°C for 1 minute for 30 to 40 cycles (depending on assay)) was performed using the HotStartTaq DNA polymerase kit (Qiagen) with 3 µl of the prepared cDNA and gene-specific primers. All kits were used according to the manufacturer's recommendations. PCR products were analyzed by electrophoresis on 2.5 % agarose gels. Universal RNA was obtained from Biocat (Heidelberg, Germany) and total RNA isolated from brain and sperm from Stratagene (La Jolla, California, US).

*Analysis* and Statistical methods: Methylation profiles were calculated as described previously in PLoS Biol. 2, 2170-2182 (2004) and are available from the HEP database/browser at www.epigenome.org. The methylation profile of each individual amplicon is provided in Figures 1.1-1.403.

### Results

Each individual matrix represents the sequencing data for an individual amplificate. Each of the discrete blocks of the matrix represent a single sample type and are labeled 'A' through 'L', said letters representing in each case the following tissue/cell types: A: Melanocytes; B: Heart Muscle; C: Skeletal muscle; D: Liver; E: Sperm; F: Embryonic skeletal muscle; G: Embryonic liver; H: Placental; I: Fibroblast; J: Keratinocytes; K: CD8; L: CD4.

The SEQ ID NO: of the genomic region of each amplificate is shown to the left of the matrices. This may be cross referenced in Table 4 to determine the amplificate and primer sequences. Each row of a matrix represents a single CpG site within the amplificate (according to the corresponding SEQ I DNO: from Table 4) and is numbered accordingly, each column represents a single pooled DNA sample.

The degree of methylation is represented by the shade of each position within the column from black representing 100% methylation to light gray representing 0% methylation. White positions represented a measurement for which no data was available.

### Differentially methylated regions (DMRs)

Kruskall-Wallis tests were used to determine differential methylation between tissues, measuring the proportion of uncorrected p-values that were smaller 0.001 for all CpGs. As this test is insensitive to samples that were only measured in a single sample such as sperm and placenta, the obtained number of DMRs is unlikely to be overstated due to putative aberrant methylation within these samples. Some DMRs were experimentally validated by sequencing independent DNA samples. Equality between two groups (age and sex) was performed using Wilcoxon tests.

Median CpG methylation values were used for the analysis of co-methylation. CpGs for which methylation values derived from both the forward and reverse strands displayed a difference of greater than 10% between the two values were excluded. Methylation changes were calculated based on the absolute methylation differences between CpG pairs of identical samples. To exclude a bias introduced by the amplicon selection, the analysis was performed using both, individual CpGs (window size 20,000bp) and CpGs of the same amplicons. Co-methylation of CpGs was described as a function of the distance (in bp) displaying the observed ratio of similar methylation degree.

For scatter plots, equal amounts of measurements were binned and ranked by numerical order of the X-axis values, representing means of X- and Y- data. For box plots and histograms, data were binned according to the intervals indicated on the X-axis containing different numbers of measurements.

As a measure for the probability of differential methylation, amplicons were sorted by their p-value and binned by rank into groups of 200 and scanned using 211 vertebrate position-weight matrices from the TRANSFAC library40 (version 3.2). For each motif, we picked a threshold such that it matched around 40% of amplicons and performed a 2 test to determine if the hit rate of the motif varied significantly between the highest and lowest 200 amplicons, ranked by P-value of differential methylation.

Tables 6 and 7 provide an overview of the genomic regions and ranges of methylation within which the actual value of methylation of said genomic regions lies specific of the said cells, tissues and/or organs. The actual value of methylation is shown in Figures 1.1-1.403.

Table 11 provides an overview of 43 different samples derived from sperm, various primary cell types (dermal fibroblasts, dermal keratinocytes, dermal melanocytes, CD4⁺ and CD8⁺ lymphocytes) and tissues (heart muscle, skeletal muscle, liver and placenta. The study was controlled for age and sex and comprised the analysis. Tissues were pooled from up to three age- and sex-matched individuals.

Table 12 provides summary statistics.

Table 13 provides an overview of the most preferred of the genes or genomic regions and ranges of methylation within which the actual value of methylation of said genomic regions lies specific of the said cells, tissues and/or organs. The actual value of methylation is shown in Figures 1.1-1.403. We found 17% of the 873 analyzed genes differentially methylated in their 5'-untranslated regions (5'-UTR), in at least one of the tissues examined. Differential methylation is observed more frequently in evolutionary conserved regions (ECRs) than within 5'-UTRs, suggesting that methylation has a functional role beyond a direct effect on transcription via promoter methylation. About one third of the differentially methylated 5'-UTRs are inversely correlated with transcription of the respective mRNAs. We did not find any significant sex or age-dependent differences in our study, indicating that methylation is ontogenetically more stable than previously anticipated.

**Table 11**

| **#** | **Tissue/cell type** | **Age (average)** | **Sex** | **Pool of** |
|---|---|---|---|---|
| 1 | heart muscle | 25 Y | female | 3 |
| 2 | heart muscle | 23 Y | male | 3 |
| 3 | heart muscle | 25 Y | male | 3 |
| 4 | heart muscle | 78 Y | male | 3 |
| 5 | heart muscle | 74 Y | male | 3 |
| 6 | heart muscle | 62 Y | male | 3 |
| 7 | skeletal muscle | 19 Y | male | 3 |
| 8 | skeletal muscle | 30 Y | male | 3 |
| 9 | skeletal muscle | 22 Y | male | 3 |
| 10 | skeletal muscle | 59 Y | male | 3 |
| 11 | skeletal muscle | 67 Y | male | 3 |
| 12 | skeletal muscle | 74 Y | male | 3 |
| 13 | skeletal muscle | 65 Y | female | 3 |
| 14 | skeletal muscle | 67 Y | female | 3 |
| 15 | skeletal muscle | 84 Y | female | 2 |
| 16 | liver | 23 Y | male | 3 |
| 17 | liver | 23 Y | male | 3 |
| 18 | liver | 30 Y | male | 3 |
| 19 | liver | 82 Y | male | 3 |
| 20 | liver | 65 Y | male | 3 |
| 21 | liver | 74 Y | male | 3 |
| 22 | liver | 70 Y | female | 3 |
| 23 | liver | 81 Y | female | 3 |
| 24 | sperm | 24 Y | male | 3 |
| 25 | fetal skeletal muscle | 23 W | male | 3 |
| 26 | fetal liver | 24 W | male | 3 |
| 27 | placenta | NB | female | 3 |
| 28 | melanocytes | 30 Y | female | 1 |
| 29 | melanocytes | 41 Y | female | 1 |
| 30 | melanocytes | 42 Y | female | 1 |
| 31 | dermal fibroblasts | 43 Y | female | 2 |
| 32 | dermal fibroblasts | 41 Y | female | 2 |
| 33 | dermal fibroblasts | 39 Y | female | 2 |
| 34 | dermal keratinocytes | 43 Y | female | 3 |
| 35 | dermal keratinocytes | 39 Y | female | 3 |
| 36 | dermal keratinocytes | 33 Y | female | 2 |
| 37 | CD4 lymphocytes | 20 Y | male | 1 |
| 38 | CD8 lymphocytes | 59 Y | male | 1 |
| 39 | CD8 lymphocytes | 60/61 Y | female | 1 |
| 40 | CD8 lymphocytes | 62 Y | male | 1 |
| 41 | CD4 lymphocytes | 59/60 Y | female | 1 |
| 42 | CD4 lymphocytes | 61 Y | female | 1 |
| 43 | CD4 lymphocytes | 62 Y | female | 1 |

Table 11 provides an overview of 43 different samples derived from sperm, various primary cell types (dermal fibroblasts, dermal keratinocytes, dermal melanocytes, CD4⁺ and CD8⁺ lymphocytes) and tissues (heart muscle, skeletal muscle, liver and placenta.

**Table 12 provides summary statistics.**

| Table 12 | | | | |
|---|---|---|---|---|
| | | | | |
| | Total | Chromosome 6 | Chromosome 20 | Chromosome 22 |
| CpG islands on chromosome | 2,279 | 1,07 | 662 | 547 |
| CpG islands covered | 511 | 256 | 29 | 226 |
| CpG islands percentage covered | 22% | 24% | 4% | 41% |
| Genes covered | 873 | 383 | 89 | 401 |
| Exons covered | 853 | 454 | 23 | 376 |
| Introns covered | 920 | 465 | 118 | 337 |
| | | | | |
| Number of tissues analyzed | 12 | | | |
| Number of samples analyzed | 43 | | | |
| Average length of amplicon +/-SD | 411 +/- 77bp | | | |
| Average number of CpGs per | | | | |
| amplicon | 16 +/- 10.8 | | | |
| Total number of different amplicons | 2,524 | | | |
| Number of CpGs analyzed | 1,885,003 | | | |

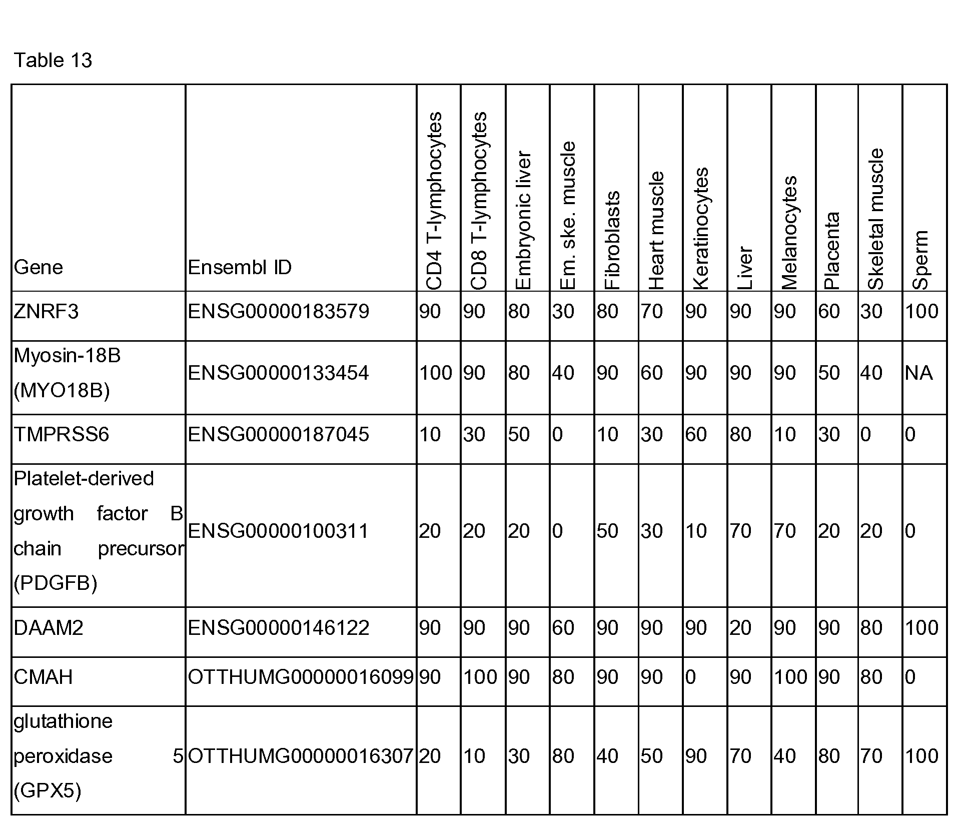

Table 13 provides an overview of the most preferred of the genes or genomic regions and ranges of methylation within which the actual value of methylation of said genomic regions lies specific of the said cells, tissues and/or organs. The actual value of methylation is shown in Figures 1.1-1.403.

## Claims

1. A method for classifying a biological sample comprising the following steps of:
a. obtaining a biological sample from a subject;
b. determining the expression status of gene KAAG1; and
c. classifying said biological sample according to said expression status.

2. A method according to claim 1 wherein the class is selected from the group consisting of organ type, tissue type, cell type and disease state.

3. A method according to claims 1 and 2 wherein said class consists of liver.

4. A method according to claim 1 wherein the expression is determined by measuring the level of at least one of mRNA, cDNA or polypeptide.

5. A method according to claim 6 wherein the expression is determined by use of at least one technique selected from the group of Northern blot analysis, reverse transcriptase PCR, real-time PCR, RNAse protection, and microarray.

6. A method according to any of claims 1 to 2 wherein said expression is determined by determining the level of methylation or methylation status of one or more CpG positions within said gene or genomic region.

7. A method according to claim 6, comprising contacting genomic DNA isolated from the biological sample with at least one reagent, or series of reagents that distinguishes between methylated and nonmethylated CpG dinucleotides within at least one target region of the genomic DNA, wherein the target region comprises, or hybridizes under stringent conditions to a sequence of at least 16 contiguous nucleotides of gene KAAG1 with SEQ ID NO: 591 wherein said contiguous nucleotides comprise at least one CpG dinucleotide sequence, and whereby classification of said sample is, at least in part, afforded.

8. A method according to claim 7, wherein, the reagent is a solution of a bisulfite, hydrogen sulfite or disulfite.

9. A method according to claim 7, further comprising the following steps of:
a. contacting the treated genomic DNA, or the treated fragment thereof, with an amplification enzyme and at least two primers comprising in each case a contiguous sequence at least 9 nucleotides in length that is complementary to, or hybridizes under moderately stringent or stringent conditions to a sequence selected from the group consisting of SEQ ID NO.: 2005, 2006, 3653, 3654 and complements thereof, wherein the treated DNA or a fragment thereof is either amplified to produce one or more amplificates, or is not amplified;
b. determining based on the presence or absence of, or on the quantity or on a property of said amplificate, the methylation state of at least one CpG dinucleotide sequence of gene KAAG1 with SEQ ID NO: 591, or an average, or a value reflecting an average methylation state of a plurality of CpG dinucleotide sequences of KAAG1 with SEQ ID NO: 591; and
c. classifying said sample according to said methylation state.

10. A method according to any of the preceding claims wherein the sample is selected from the group consisting of cells or cell lines, histological slides, biopsies, paraffin-embedded tissue, bodily fluids, sputum, stool, nipple aspirate, cerebrospinal fluid, ejaculate, urine, blood, and combinations thereof.

11. A nucleic acid comprising at least 16 contiguous nucleotides of a DNA sequence selected from the group consisting of SEQ ID Nos.: 2005, 2006, 3653, 3654, and sequences complementary thereto.

12. An oligonucleotide which is identical, is complementary, or hybridizes under stringent or highly stringent conditions to at least 9 base long segment of a sequence selected from SEQ ID NOs.: 2005, 2006,3653,3654.

13. A kit suitable for performing the methods according to any of the preceding claims comprising (a) one or more reagents to convert unmethylated cytosine bases to uracil or to anther base that is detectably dissimilar to cytosine in terms of hybridization properties (b) at least one oligonucleotide which is identical, is complementary, or hybridizes under stringent or highly stringent conditions to at least 9 base long segment of a sequence selected from SEQ ID Nos.: 2005, 2006, 3653, 3654.

14. A kit suitable for performing the methods according to any of the preceding claims comprising means for detecting polypeptides of gene KAAG1 with SEQ ID NO: 591 or means for measuring level of mRNA transcription of gene KAAG1 with SEQ ID NO.:591.

15. The use of a method according to claims 1 to 10, a nucleic acid according to claim 8, an oligomer according to claim 12, a kit according to claims 13-14 for classifying a biological sample.
